# EUROPÄISCHE PATENTSCHRIFT

(11) **EP 1 771 069 B1**
(45) Veröffentlichungstag und Bekanntmachung des Hinweises auf die Patenterteilung: **07.05.2014**
(21) Anmeldenummer: 05701132.2
(22) Anmeldetag: 22.01.2005
(51) Int. Cl.: A01N 43/56, C07D 231/16, C07D 231/14

(54) **N-(2-(HYDROXYMETHYL)PHENYL)-1H-PYRAZOL-4-CARBOXAMID DERIVATE UND VERWANDTE VERBINDUNGEN ALS MIKROBIZIDE WIRKSTOFFE ZUR ANWENDUNG IM PFLANZEN- UND MATERIALSCHUTZ**
N-(2-(HYDROXYMETHYL) PHENYL)-1H-PYRAZOLE-4-CARBOXAMIDE DERIVATIVES AND RELATED COMPOUNDS AS MICROBICIDAL ACTIVE INGREDIENTS FOR PHYTO-PROTECTION AND THE PROTECTION OF MATERIALS
DERIVES DE N-(2-(HYDROXYMETHYL)PHENYL)-1H-PYRAZOLO-4-CARBOXAMIDE ET COMPOSES APPARENTES UTILISES COMME SUBSTANCES ACTIVES MICROBICIDES DANS LA PROTECTION PHYTOSANITAIRE ET LA PROTECTION DES MATERIAUX

(30) Priorität: 06.02.2004 DE 102004005787
(43) Veröffentlichungstag der Anmeldung: 11.04.2007
(73) Patentinhaber: Bayer CropScience Aktiengesellschaft, 40789 Monheim (DE)
(72) Erfinder: DUNKEL, Ralf, 69001 Lyon (FR); ELBE, Hans-Ludwig, 42329 Wuppertal (DE); HARTMANN, Benoit, 40764 Langenfeld (DE); GREUL, Jörg, Nico, 42799 Leichlingen (DE); HERRMANN, Stefan, 40764 Langenfeld (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2005/000633
(87) Internationale Veröffentlichungsnummer: WO 2005/074686

(56) Entgegenhaltungen:
- WO-A-03/010149
- JP-A- 2004 189 738
- DIXON ET AL: "Kinetics and mechanism of the addition of water and ring-opening of 2-methyl- and 2-aryl-4H-3,1-benzoxazines to 2-aminobenzyl esters in the acidic pH range; change in rate-limiting step with buffer concentration and evidence for a tetrahedral carbonyl addition intermediate" J. CHEM. SOC. PERKIN TRANS. 2, Bd. 8, 1997, Seiten 1503-1509, XP002323920
- RAIFORD ET AL: "BEHAVIOR OF MIXED O-ACYL-N-ACYL DERIVATIVES IN WHICH THE REACTING GROUPS ARE NOT ON ADJACENT CARBON ATOMS" J. AM. CHEM. SOC., Bd. 48, 1926, Seiten 483-489, XP002323921
- DATABASE BEILSTEIN 29. Juni 1989 (1989-06-29), XP002323927 Database accession no. BRN: 1216128 & ASAKAWA ET AL: CHEM. PHARM. BULL., Bd. 27, 1979, Seiten 522-527,
- DATABASE BEILSTEIN 5. Februar 1990 (1990-02-05), XP002323928 Database accession no. BRN: 3325417 & CLOVER ET AL: J. CHEM. SOC. PERKIN TRANS. 2, Bd. 7, 1996, Seiten 1367-1376,
- DATABASE BEILSTEIN 25. Juli 2003 (2003-07-25), XP002323929 Database accession no. BRN: 9326393 & MISHIO ET AL: HETEROCYCLES, Bd. 58, 2002, Seiten 203-212,
- BELEKON ET AL: "Synthesis of -Amino Acids via Asymmetric Phase Transfer-Catalyzed Alkylation of Achiral Nickel(II) Complexes of Glycine-Derived Schiff Bases" J. AM. CHEM. SOC., Bd. 125, Nr. 42, 2003, Seiten 12860-12871, XP002323922
- CHAN SIK CHO ET AL: "Ruthenium-catalyzed oxidative coupling and cyclization between 2-aminobenzyl alcohol and secondary alcohols leading to quinolines" TETRAHEDRON, Bd. 59, Nr. 40, 2003, Seiten 7997-8002, XP002323923
- ROUSSEL ET AL: "Inhibition of the tissue factor/factor VIIa complex ? Lead optimisation using combinatorial chemistry" TETRAHEDRON, Bd. 55, Nr. 19, 1999, Seiten 6219-6230, XP002323924
- TSOUNGAS ET AL: "A convenient access to benzo-substituted phthalazines as potential precursors to DNA intercalators" TETRAHEDRON LETTERS, Bd. 42, Nr. 37, 2001, Seiten 6589-6592, XP002323925
- MULZER ET AL: "Chiral Acetals as Stereoinductors: Diastereoface Selective Alkylation of Dihydrobenzoxazine-Derived Amide Enolates" J. ORG. CHEM., Bd. 65, Nr. 20, 2000, Seiten 6540-6546, XP002323926

## Beschreibung

Die vorliegende Erfindung betrifft neue Carboxamide, mehrere Verfahren zu deren Herstellung und deren Verwendung zur Bekämpfung von unerwünschten Mikroorganismen.

Es ist bereits bekannt, dass zahlreiche Carboxamide fungizide Eigenschaften besitzen (vgl. z.B. WO 03/010149, WO 02/059086, EP-A 0 824 099, EP-A 0 737 682, EP-A 0 591 699, EP-A 0 589 301, EP-A 0 545 099, DE-A 24 09 011, DE-A 20 06 472, JP-A 2001-302605, JP-A 10-251240, JP-A 8-176112, JP-A 8-92223 und JP-A 53-72823). So sind bereits zahlreiche Alkylcarboxamide bekannt geworden, die im Alkylteil nicht substituiert sind, wie beispielsweise N-Allyl-N-[2-(1,3-dimethylbutyl)phenyl]-1-methyl-3-(trifluormethyl)-1H-pyrazol-4-carboxamid aus WO 02/059086, N-[2-(1,3-Dimethylbutyl)phenyl]-2,4-dimethyl-1,3-thiazol-5-carboxamid aus EP-A 0 824 099 und 5-Fluor-1,3-dimethyl-N-[2-(1,3,3-trimethylbutyl)phenyl]-1H-pyrazol-4-carboxamid aus WO 03/010149. Die Wirksamkeit dieser Stoffe ist gut, lässt aber in manchen Fällen, z.B. bei niedrigen Aufwandmengen zu wünschen übrig.

WO-A-2003/010149 offenbart Carboxamide deren Phenylring M mit einer Hydroxyalkylgruppe substituiert ist. WO-A-2003/010149 offenbart jedoch keine Carboxamide, bei denen der Phenylring M mit L1-Q-L2-R substituiert ist, wobei-R ungleich Wasserstoff ist, so dass die Substitution mit einer Hydroxyalkylgruppe ausgeschlossen ist.

Dixon et al. "Kinetics and mechanism ofthe addition of water and ring-opening of 2-methyl- and 2-aryl-4H-3, 1-benzoxazines to 2-aminobenzyl esters in the acidic pH range; change in rate limiting step with buffer concentration and evidence for a tetrahedral carbonyl addition intermediate", J. Chem. Soc. Perkin Trans. 2, Bd. 8, 1997, Seiten 1503 - 1510 offenbart Ergebnisse kinetischer Untersuchungen intramolekularer Reaktionen bei substituierten Benzylderivaten mit reaktiven Gruppen an Position 2 am Aromaten. Jedoch werden die erfindungsgemäßen Carboxamide oder deren Verwendung als Fungizide nicht offenbart.

Raiford et al., "Behavior of mixed O-acyl-N-acyl derivatives in which the reacting groups are not on adjacent carbon atoms", J. Am. Chem. Soc., Bd. 48, 1926, Seite 487, offenbart Ergebnisse zur Untersuchung der Migration von Acyl in Acetyl-Benzoyl-Derivaten. Allerdings werden die erfindungsgemäßen Carboxamide oder deren Verwendung als Fungizide nicht offenbart.

Database Beilstein 29. Juni 1989 (XP002323927), Database Beilstein 05. Februar 1990 (XP002323928) und Database Beilstein 25. Juli 2003 (XP002323929) offenbaren ebenfalls Carboxamide. Jedoch werden die erfindungsgemäßen Carboxamide, bei denen der Phenylring M mit L1-Q-L2-R substituiert ist, wobei R ungleich Wasserstoff ist, nicht offenbart.

Belekon et al., "Synthesis of amino acids via asymmetric phase transfer catalyzed alkylation of achiral nickel(II) complexes of glycine derived Schiff bases", J. Am. Chem. Soc.., Bd. 125, Nr. 42, 2003, Seiten 12860 - 12871, offenbart die Synthese von Carboxamiden als Zwischenprodukt für die Herstellung von Ni(II)-Komp!exen. Jedoch werden die erfindungsgemäßen Carboxamide nicht offenbart.

Es wurden nun neue Carboxamide der Formel (I) 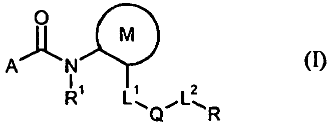 gefunden, in welcher
- R¹: für Wasserstoff, C₁-C₈-Alkyl, C₁₋C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-atkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₄-Alkoxy₋C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R², -CONR³R⁴ oder -CH₂NR⁵R⁶ steht,
- R²: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R³ und R⁴: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen stehen,
- R³ und R⁴: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
- R⁵ und R⁶: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 .bis 9 Fluor-, Chlor- und/oder Bromatomen stehen,
- R⁵ und R⁶: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
- R⁷: für Wasserstoff oder C₁-C₆-Alkyl steht,
- M: für einen jeweils einfach durch R⁸ substituierten Phenyl Ring steht,
- R⁸: für Wasserstoff, Fluor, Chlor, Methyl, iso-Propyl, Methylthio oder Trifluormethyl steht,
- R⁸: außerdem für Methoxy steht,
- R^{8-A}: für Wasserstoff, Methyl, Methylthio oder Trifluormethyl steht,
- L¹: für C₁-C₁₀-Alkylen (Alkandiyl) steht,
- Q: für O, S, SO, SO₂ oder NR⁹ steht,
- L²: für eine direkte Bindung, SiR¹⁰R¹¹ oder CO steht,
- R: für C₁-C₈-Alkyl C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl oder C₃-C₆-Cycloalkyl steht,
- R⁹: für Wasserstoff, C₁-C₈-Alkyl, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl oder C₃-C₆-Cycloalkyl steht,
- R¹⁰ und R¹¹: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- A: für den Rest der Formel (A1) 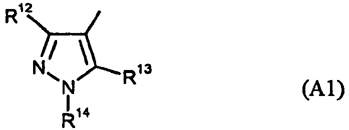 steht, in welcher
R¹² für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halo-genalkylthio mit jeweils 1 bis 5 Halogenatomen, Aminocarbonyl oder Aminocarbonyl-C₁-C₄-alkyl steht,
R¹³ für Wasserstoff Halogen, Cyano, C₁-C₄-Alkyl C₁-C₄-Alkoxy oder C₁-C₄-Alkylthio steht,
R¹⁴ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht, oder
- A: für den Rest der Formel (A2) 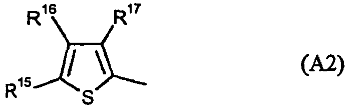 steht, in welcher
R¹⁵ und R¹⁶ unabhängig voneinander für Wasserstoff, Halogen, C₁-C₄-Alkyl oder C₁-C₄-Halogenalkyl mit 1 bis 5 Halogenatomen steht,
R¹⁷ für Halogen, Cyano oder C₁-C₄-Alkyl, oder C₁-C₄-Halogenalkyl oder C₁-C₄-Halogen-alkoxy mit jeweils 1 bis 5 Halogenatomen steht, oder
- A: für den Rest der Formel (A3) 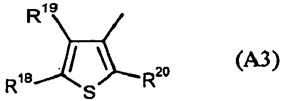 steht, in welcher
R¹⁸ und R¹⁹ unabhängig voneinander für Wasserstoff, Halogen, C₁-C₄-Alkyl oder C₁-C₄-Halogenalkyl mit 1 bis 5 Halogenatomen steht,
R²⁰ für Wasserstoff, Halogen, C₁-C₄-Alkyl oder C₁-C₄-Halogenalkyl mit 1 bis 5 Halogenatomen steht, oder
- A: für den Rest der Formel (A4) 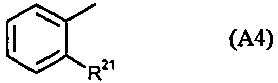 steht, in welcher
R²¹ für Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht, oder
- A: für den Rest der Formel (A5) 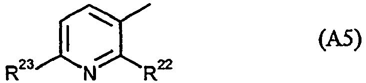 steht, in welcher
R²² für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R²³ für Wasserstoff, Halogen, Cyano, C₁-C₄-Alkyl, C₁-C₄-Akoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht, oder
- A: für den Rest der Formel (A6) 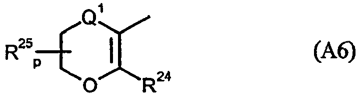 steht, in welcher
R²⁴ für C₁-C₄₋Alkyl oder C₁-C₄-Halogenalkyl mit 1 bis 5 Halogenatomen steht,
R²⁵ für C₁-C₄-Alkyl steht,
Q¹ für S (Schwefel), SO, SO₂ oder CH₂ steht,
p für 0, 1 oder 2, wobei R²⁵ für identische oder verschiedene Reste steht, wenn p für 2 steht, oder
- A: für den Rest der Formel (A7) 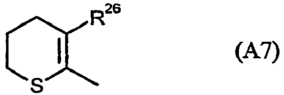 steht, in welcher
R²⁶ für C₁-C₄-Alkyl oder C₁-C₄-Halogenalkyl mit 1 bis 5 Halogenatomen steht, oder
- A: für den Rest der Formel (A8) 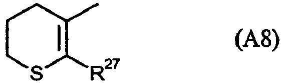 steht, in welcher
R²⁷ für C₁-C₄-Alkyl oder C₁-C₄-Halogenalkyl mit 1 bis 5 Halogenatomen steht, oder
- A: für den Rest der Formel (A9) 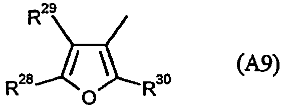 steht, in welcher
R²⁸ und R²⁹ unabhängig voneinander für Wasserstoff, Halogen, Amino, C₁-C₄-Alkyl oder C₁-C₄-Halogenalkyl mit 1 bis 5 Halogenatomen stehen,
R³⁰ für Wasserstoff, Halogen, C₁-C₄₋Alkyl oder C₁-C₄-Halogenalkyl mit 1 bis 5 Halogenatomen steht, oder
- A: für den Rest der Formel (A10) 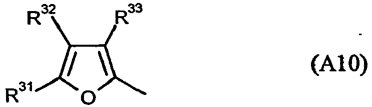 steht, in welcher
R³¹ und R³² unabhängig voneinander für Wasserstoff, Halogen, Amino, Nitro, C₁-C₄-Alkyl oder C₁-C₄-Halogenalkyl having 1 bis 5 Halogenatomen stehen,
R³³ für Wasserstoff, Halogen, C₁-C₄-Alkyl oder C₁-C₄-Halogenalkyl mit 1 bis 5 Halogenatomen steht, oder
- A: für den Rest der Formel (A11) 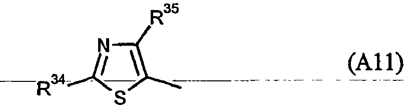 steht, in welcher
R³⁴ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R³⁵ für Halogen, C₁-C₄-Alkyl oder C₁-C₄-Halogenalkyl mit 1 bis 5 Halogenatomen steht, oder
- A: für den Rest der Formel (A12) 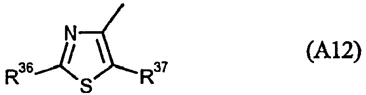 steht, in welcher
R³⁶ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R³⁷ für Halogen, C₁-C₄-Alkyl oder C₁-C₄-Halogenalkyl mit 1 bis 5 Halogenatomen steht, oder
- A: für den Rest der Formel (A13) 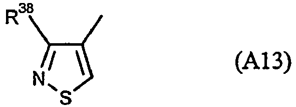 steht, in welcher
R³⁸ für Halogen, C₁-C₄₋Alkyl oder C₁-C₄-Halogenalkyl mit 1 bis 5 Halogenatomen steht, oder
- A: für den Rest der Formel (A14) 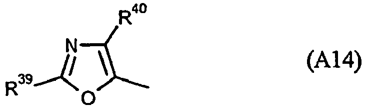 steht, in welcher
R³⁹ für Wasserstoff oder C₁-C₄-Alkyl steht,
R⁴⁰ für Halogen oder C₁-C₄-Alkyl steht, oder
- A: für den Rest der Formel (A15) 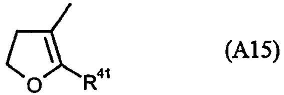 steht, in welcher
R⁴¹ für C₁-C₄-Alkyl oder C₁-C₄-Halogenalkyl mit 1 bis 5 Halogenatomen steht, oder
- A: für den Rest der Formel (A16) 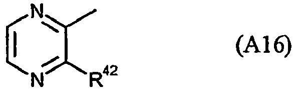 steht, in welcher
R⁴² für Wasserstoff, Halogen, C₁-C₄-Alkyl oder C₁-C₄-Halogenalkyl mit 1 bis 5 Halogenatomen steht, oder
- A: für den Rest der Formel (A17) 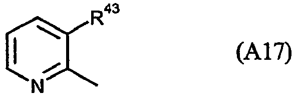 steht, in welcher
R⁴³ für Halogen, Hydroxy, C₁-C₄-Alkyl, C₁-C₄-Alkoxy, C₁-C₄-Alkylthio, C₁-C₄-Halogen-alkyle, C₁-C₄-Halogenalkylthio oder C₁-C₄-Halogenalkoxy mit jeweils 1 bis 5 Halogenatomen steht, oder
- A: für den Rest der Formel (A18) 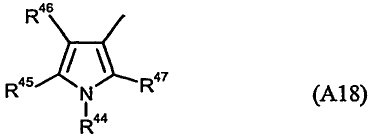 steht, in welcher
R⁴⁴ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆₋Alkylcarbonyl oder jeweils gegebenenfalls substituiertes Phenylsulfonyl oder Benzoyl steht,
R⁴⁵ für Wasserstoff, Halogen, C₁-C₄-Alkyl oder C₁-C₄-Halogenalkyl mit 1 bis 5 Halogenatomen steht,
R⁴⁶ für Wasserstoff, Halogen, Cyano, C₁-C₄-Alkyl oder C₁-C₄-Halogenalkyl mit 1 bis 5 Halogenatomen steht,
R⁴⁷ für Wasserstoff, Halogen, C₁-C₄-Alkyl oder C₁-C₄-Halogenalkyl mit 1 bis 5 Halogenatomen steht, oder
- A: für den Rest der Formel (A19) 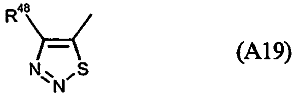 steht, in welcher
R⁴⁸ für C₁-C₄-Alkyl steht,
wobei R nicht für Alkoxy steht, wenn L² für eine direkte Bindung steht.

Weiterhin wurde gefunden, dass man Carboxamide der Formel (I) erhält, indem man
(a) Carbonsäure-Derivate der Formel (II) 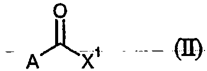 in welcher
   - A: die oben angegebenen Bedeutungen hat und
   - X¹: für Halogen oder Hydroxy, steht,
   mit Anilin-Derivaten der Formel (III) 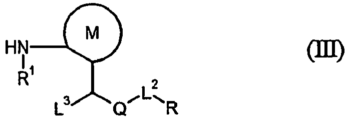 in welcher
   - R¹, M, Q, L² und R: die oben angegebenen Bedeutungen haben,
   - L³: für Wasserstoff oder C₁₋₄-Alkyl steht,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
(b) Carboxamide der Formel (IV) 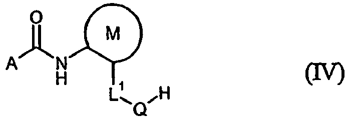 in welcher M, L¹, Q und A die oben angegebenen Bedeutungen haben, mit einer Verbindung der Formel (V) 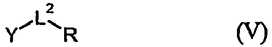 in welcher
   - L² und R: die oben angegebenen Bedeutungen haben,
   - Y: für Halogen, Triflat (Trifluormethylsulfonyl), Mesylat (Methylsulfonyl) oder Tosylat (4-Methylphenylsulfonyl) steht,
   in Gegenwart einer Base und in Gegenwart eines Verdünnungsmittels umsetzt, oder
(c) Carboxamide der Formel (I-a) 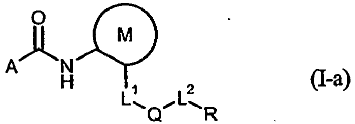 in welcher M, L¹, Q, L², R und A die oben angegebenen Bedeutungen haben, mit Halogeniden der Formel (VI)

   R^{1-A}-X² (VI)

   in welcher
   - X²: für Chlor, Brom oder Iod steht,
   - R^{1-A}: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halo-genalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen₋(C₁₋C₄-alkyl)carbonyl-C₁-C₃-alkyl, Halogen-C₁₋C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogen-alkoxy)carbonyl, (Halogen-C₁-C₄-alkoxy-C₁-C₄-alkyl)carbonyl, (C₃-C₈-Halogen-cycloalkyl)carbonyl mit jeweils 1 bis 9 Fluor-, Chlor- und/oder Bromatomen; oder -C(=O)C(=O)R², -CONR³R⁴ oder -CH₂NR⁵R⁶ steht, wobei R², R³, R⁴, R⁵ und R⁶, die oben angegebenen Bedeutungen haben,
in Gegenwart einer Base und in Gegenwart eines Verdünnungsmittels umsetzt.

Schließlich wurde gefunden, dass die neuen Carboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Carboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht bevorzugt für Wasserstoff, C₁-C₆₋Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C1-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆₋Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R², -CONR³R⁴ oder -CH₂NR⁵R⁶.
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluor-methylthio, Difluorchlormethylthio, Trifluormethylthio, Trifluormethylsulfinyl, Trifluormethyl-sulfonyl, Trifluormethoxymethyl; Formyl, -CH₂-CHO, -(CH₂)₂-CHO, -CH₂-CO-CH₃, -CH₂CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂₋CO₂CH₃, -CH₂-CO₂CH₂CH₃, -CH₂-CO₂CH(CH₃)₂, -(CH₂)₂CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃, Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluorme-thylcarbonyl, Trifluormethoxycarbonyl, oder -C(=O)C(=O)R², -CONR³R⁴ oder -CH₂NR⁵R⁶.
- R¹: steht ganz besonders bevorzugt für Wasserstoff Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- R²: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R²: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluor-methyl, Trifluormethoxy.
- R³ und R⁴: stehen unabhängig voneinander bevorzugt für Wasserstoff, C₁-C₆-Alkyl, C₁-C₃-Alkoxy-C₁-C₃-alkyl, C₁-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R³ und R⁴: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁷ enthalten kann.
- R³ und R⁴: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R³ und R⁴: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁷ substituiert sein kann.
- R⁵ und R⁶: stehen unabhängig voneinander bevorzugt für Wasserstoff, C₁-C₆-Alkyl C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R⁵ und R⁶: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁷ enthalten kann.
- R⁵ und R⁶: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁵ und R⁶: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁷ substituiert sein kann.
- R⁷: steht bevorzugt für Wasserstoff oder C₁-C₄-Alkyl.
- R⁷: steht besonders bevorzugt für Wasserstoff, Methyl, Ethyl, n- oder iso-Propyl, n-, iso-, sec- oder tert-Butyl.
- M: steht bevorzugt für 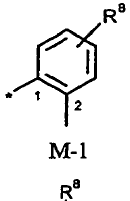
wobei die mit "*" markierte Bindung mit dem Amid verknüpft ist.
- R⁸: steht bevorzugt für Wasserstoff.
- R⁸: steht für den Fall, dass M für M-1 steht, außerdem bevorzugt für Fluor, wobei Fluor besonders bevorzugt in 4-, 5- oder 6-Position, ganz besonders bevorzugt in 4- oder 6-Position, ins-besondere in 4-Position steht
- R⁸: steht für den Fall, dass M für M-1 steht, außerdem bevorzugt für Chlor, wobei Chlor besonders bevorzugt in 4- oder 6-Position steht.
- R⁸: steht für den Fall, dass M für M-1 steht, außerdem bevorzugt für Methyl, wobei Methyl besonders bevorzugt in 3-Position und außerdem besonders bevorzugt in 4-Position steht.
- R⁸: steht für den Fall, dass M für M-1 steht, außerdem bevorzugt für Methoxy, wobei Methoxy besonders bevorzugt in 4-Position steht
- R⁸: steht für den Fall, dass M für M-1 steht, außerdem bevorzugt für Trifluormethyl, wobei Trifluormethyl besonders bevorzugt in 4- oder 6-Position steht
- R⁸: steht für den Fall, dass M für M-2, M-3, M-4 oder M-5 steht, außerdem bevorzugt für Fluor, wobei Fluor besonders bevorzugt in 6-Position (M-2, M-3) oder in 3-Position (M-4, M-5) steht
- R⁸: steht für den Fall, dass M für M-2, M-3, M-4 oder M-5 steht, außerdem bevorzugt für Chlor, wobei Chlor besonders bevorzugt in 6-Position (M-2, M-3) oder in 3-Position (M-4, M-5) steht
- R⁸: steht für den Fall, dass M für M-2, M-3, M-4 oder M-5 steht, außerdem bevorzugt für Methyl, wobei Methyl besonders bevorzugt in 4-Position (M-2) oder in 3-Position (M-3, M-4, M-5) steht
- R⁸: steht für den Fall, dass M für M-6 steht, außerdem bevorzugt für Methyl, wobei Methyl besonders bevorzugt in 3-Position steht
- R⁸: steht für den Fall, dass M für M-6 steht, außerdem bevorzugt für Trifluormethyl, wobei Tri-fluormethyl besonders bevorzugt in 3-Position steht
- R⁸: steht für den Fall, dass M für M-7, M-8 oder M-9 steht, außerdem bevorzugt für Chlor, wobei Chlor besonders bevorzugt in 5-Position (M-7, M-8) oder in 3-Position (M-9) steht
- R⁸: steht für den Fall, dass M für M-7, M-8 oder M-9 steht, außerdem bevorzugt für Methyl, wobei Methyl besonders bevorzugt in 5-Position (M-7, M-8) oder in 3-Position (M-9) steht.
- R⁸: steht für den Fall, dass M für M-12 steht, außerdem bevorzugt für Methyl, wobei Methyl besonders bevorzugt in 4-Position steht
- R⁸: steht für den Fall, dass M für M-12 steht, außerdem bevorizugt für Trifluormethyl, wobei Trifluormethyl besonders bevorzugt in 4-Position steht.
- R⁸: steht für den Fall, dass M für M-13 steht, außerdem bevorzugt für Methyl, wobei Methyl besonders bevorzugt in 3-Position steht
- R⁸: steht für den Fall, dass M für M-13 steht, außerdem bevorzugt für Trifluormethyl, wobei Trifluormethyl besonders bevorzugt in 3-Position steht
- R⁸: steht für den Fall, dass M für M-14 steht, außerdem bevorzugt für Methyl, wobei Methyl besonders bevorzugt in 3-Position steht.
- R⁸: steht für den Fall, dass M für M-14 steht, außerdem bevorzugt für Trifluormethyl, wobei Trifluormethyl besonders bevorzugt in 3-Position steht.

- R^{8-A}: steht bevorzugt für Wasserstoff.
- R^{8-A}: steht außerdem bevorzugt für Methyl.
- R^{8-A}: steht außerdem bevorzugt für Trifluormethyl.

- L¹: steht bevorzugt für C₁-C₆-Alkylen (Alkandiyl).
- L¹: steht besonders bevorzugt für -CH₂-, -CH(CH₃)- oder -(CH₂)₂C(CH₃)₂-.
- L¹: steht außerdem besonders bevorzugt für -(CH₂)₂-.

- Q: steht bevorzugt für O.
- Q: steht außerdem bevorzugt für S.
- Q: steht außerdem bevorzugt für SO.
- Q: steht außerdem bevorzugt für SO₂.
- Q: steht außerdem bevorzugt für NR⁹, besonders bevorzugt für NH.

- L²: steht bevorzugt für eine direkte Bindung.
- L²: steht außerdem bevorzugt für SiR¹⁰R¹¹.
- L²: steht außerdem bevorzugt für CO.

- R: steht bevorzugt für C₁-C₆-Alkyl, C₁-C₆-Alkoxy, C₁-C₃-Alkoxy-C₁-C₃-alkyl, C₁-C₃-Alkylthio-C₁-C₃-alkyl oder C₃-C₆-Cycloalkyl.
- R: steht außerdem bevorzugt für C₁-C₄-Halogenalkyl.

- R: steht besonders bevorzugt für Methyl, Ethyl, n- oder iso-Propyl, n-, sec-, iso-oder tert-Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthio-methyl, Methylthioethyl, Ethylthioethyl oder Cyclopropyl.
- R: steht außerdem besonders bevorzugt für 1-Methylbutyl, C₁-C₂-Häogenalkyl mit 1 bis 5 Fluor-, Chlor- oder Bromatomen, Cyclopentyl oder Cyclohexyl.
- R: steht ganz besonders bevorzugt für Methyl, Ethyl, n- oder iso-Propyl, iso- oder tert-Butyl, Methoxy, iso-Propoxy, iso- oder tert-Butoxy, Methoxymethyl oder Methylthiomethyl.
- R: steht außerdem ganz besonders bevorzugt für sec-Butyl, 1-Methylbutyl, Dichlormethyl, Cyclopropyl, Cyclopentyl oder Cyclohexyl.
- R: steht inbesondere bevorzugt für Methyl, Ethyl, n- oder iso-Propyl, iso- oder tert-Butyl, Methoxy, iso-Propoxy, iso- oder tert-Butoxy.
- R: steht außerdem insbesondere bevorzugt für sec-Butyl oder 1-Methylbutyl.
- R⁹: steht bevorzugt für Wasserstoff, C₁-C₆-Alkyl, C₁-C₃-Alkoxy-C₁-C₃-alkyl, C₁-C₃-Alkylthio-C₁-C₃-alkyl oder C₃-C₆-Cycloalkyl.
- R⁹: steht besonders bevorzugt für Wasserstoff; Methyl, Ethyl, n- oder iso-Propyl, n-, sec-, iso-oder tert-Butyl, Methoxymethyl, Ethoxymethyl, Methoxyethyl, Ethoxyethyl, Methylthio-methyl, Ethylthiomethyl, Methylthioethyl, Ethylthioethyl oder Cyclopropyl.
- R⁹: steht ganz besonders bevorzugt für Wasserstoff, Methyl, Ethyl, n- oder iso-Propyl, iso- oder tert-Butyl, Methoxymethyl oder Methylthiomethyl.
- R⁹: steht insbesondere bevorzugt, für Wasserstoff oder Methyl.
- R¹⁰ und R¹¹: stehen unabhängig voneinander bevorzugt für C₁-C₆-Alkyl, C₁-C₆-Alkoxy, C₁-C₃-Alkoxy-C₁-C₃-alkyl oder C₁-C₃-Alkylthio-C₁-C₃-alkyl.
- R¹⁰ und R¹¹: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R¹⁰ und R¹¹: stehen unabhängig voneinander ganz besonders bevorzugt für Methyl, Methoxy, Methoxymethyl oder Methylthiomethyl.
- R¹⁰ und R¹¹: stehen insbesondere bevorzugt jeweils für Methyl.
- A: steht bevorzugt für einen der Reste A1, A2, A3, A4, A5, A6, A9, A10, A11, A12, A17 oder A18.
- A: steht besonders bevorzugt für einen der Reste A1, A2, A4, A5, A6, A9, A11, A16, A17, A18.
- A: ganz besonders bevorzugt für den Rest A1.
- A: außerdem ganz besonders bevorzugt für den Rest A2.
- A: außerdem ganz besonders bevorzugt für den Rest A4.
- A: außerdem ganz besonders bevorzugt für den Rest A5.
- A: außerdem ganz besonders bevorzugt für den Rest A6.
- A: außerdem ganz besonders bevorzugt für den Rest A9.
- A: außerdem ganz besonders bevorzugt für den Rest A11.
- A: außerdem ganz besonders bevorzugt für den Rest A16.
- A: außerdem ganz besonders bevorzugt für den Rest A18.
- R¹²: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halo-genalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl.
- R¹²: steht besonders bevorzugt für Wasserstoff, Fluor, Chlor, Brom, Iod, Methyl, Ethyl, iso-Propyl, Monofluormethyl, Monofluorethyl, Difluormethyl, Trifluormethyl, Difluorchlormethyl, Trichlormethyl, Dichlormethyl, Cyclopropyl, Methoxy, Ethoxy, Trifluormethoxy, Trichlormethoxy, Methylthio, Ethylthio, Trifluormethylthio oder Difluormethylthio.
- R¹²: steht ganz besonders bevorzugt für Wasserstoff, Fluor, Chlor, Brom, Iod, Methyl, iso-Propyl, Monofluormethyl, Monofluorethyl, Difluormethyl, Trifluormethyl, Difluorchlormethyl oder Trichlormethyl.
- R¹²: steht insbesondere bevorzugt für Methyl, Difluormethyl, Trifluormethyl oder 1-Fluorethyl.
- R¹³: steht bevorzugt für Wasserstoff, Fluor, Chlor, Brom, Iod, Methyl, Ethyl, Methoxy, Ethoxy, Methylthio oder Ethylthio.
- R¹³: steht besonders bevorzugt für Wasserstoff, Fluor, Chlor, Brom, Iod oder Methyl.
- R¹³: steht ganz besonders bevorzugt für Wasserstoff, Fluor, Chlor oder Methyl.
- R¹⁴: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹⁴: steht besonders bevorzugt, für Wasserstoff, Methyl, Ethyl, iso-Propyl, Trifluormethyl, Difluormethyl, Hydroxymethyl, Hydroxyethyl oder Phenyl.
- R¹⁴: steht ganz besonders bevorzugt für Wasserstoff, Methyl, Trifluormethyl oder Phenyl.
- R¹⁴: steht insbesondere bevorzugt für Methyl.
- R¹⁵ und R¹⁶: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁵ und R¹⁶: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁵ und R¹⁶: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹⁵ und R¹⁶: stehen insbesondere bevorzugt jeweils für Wasserstoff.
- R¹⁷: steht bevorzugt für Fluor, Chlor, Brom, Cyano, Methyl, Ethyl, C₁-C₂-Halogenalkyl oder C₁-C₂-Halogenalkoxy mit jeweils 1 bis 5 Fluor, Chlor und/oder Bromatomen.
- R¹⁷: steht besonders bevorzugt für Fluor, Chlor, Brom, Cyano, Methyl, Trifluormethyl, Trifluormethoxy, Difluormethoxy, Difluorchlormethoxy oder Trichlormethoxy.
- R¹⁷: steht ganz besonders bevorzugt für Fluor, Chlor, Brom, Iod, Methyl, Trifluormethyl oder Trifluonnethoxy.
- R¹⁷: steht insbesondere bevorzugt für Methyl.
- R¹⁸ und R¹⁹: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder-Bromatomen.
- R¹⁸ und R¹⁹: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁸ und R¹⁹: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹⁸ und R¹⁹: stehen insbesondere bevorzugt jeweils für Wasserstoff.
- R²⁰: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R²⁰: steht besonders bevorzugt für Wasserstoff, Fluor, Chlor, Brom, Iod, Methyl oder Trifluormethyl.
- R²⁰: steht ganz besonders bevorzugt für Methyl.
- R²¹: steht bevorzugt für Fluor, Chlor, Brom, Iod, Hydroxy, Cyano, C₁-C₄-Alkyl, C₁-C₂-Halogenalkyl, C₁-C₂-Halogenalkoxy oder C₁-C₂-Halogenalkylthio mit jeweils 1 bis 5 Fluor, Chlor und/oder Bromatomen.
- R²¹: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Difluormethyl, Trifluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio.
- R²¹: steht ganz besonders bevorzugt für Fluor, Chlor, Brom, Iod, Methyl, Difluormethyl, Trifluormethyl oder Trichlormethyl.
- R²¹: steht insbesondere bevorzugt für Iod, Methyl, Difluormethyl oder Trifluormethyl.
- R²²: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R²²: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluormethylthio, Trifluormethylthio, Trifluormethoxy, Difluormethoxy, Difluorchlormethoxy oder Trichlormethoxy.
- R²²: steht ganz besonders bevorzugt für Fluor, Chlor, Brom, Iod, Methyl, Trifluormethyl, Difluormethyl oder Trichlormethyl.
- R²³: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulphinyl oder C₁-C₂-Alkylsulphonyl.
- R²³: steht besonders bevorzugt für Wasserstoff, Fluor, Chlor, Brom, Iod, Cyano, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy, Trichlormethoxy, Methylsulphinyl oder Methylsulphonyl.
- R²³: steht ganz besonders bevorzugt für Wasserstoff, Fluor, Chlor, Brom, Iod, n-Propyl, iso-Propyl, n-Butyl, iso-Butyl, sec-Butyl, tert-Butyl, Trifluormethyl, Difluormethyl, Trichlormethyl, Methylsulphinyl oder Methylsulphonyl.
- R²³: steht insbesondere bevorzugt für Wasserstoff.
- R²⁴: steht bevorzugt für Methyl, Ethyl oder C₁-C₂-Halogenalkyl mit 1 bis 5 Fluor, Chlor und/oder Bromatomen.
- R²⁴: steht besonders bevorzugt für Methyl, Ethyl, Trifluormethyl, Difluormethyl, Difluorchlormethyl oder Trichlormethyl.
- R²⁵: steht bevorzugt für Methyl oder Ethyl.
- R²⁵: steht besonders bevorzugt für Methyl.
- Q¹: steht bevorzugt für S (Schwefel), SO₂ oder CH₂.
- Q¹: steht besonders bevorzugt für S (Schwefel) oder CH₂.
- Q¹: steht ganz besonders bevorzugt für S (Schwefel).
- p: steht bevorzugt für 0 oder 1.
- p: steht besonders bevorzugt für 0.
- R²⁶: steht bevorzugt für Methyl, Ethyl oder C₁-C₂-Halogenalkyl mit 1 bis 5 Fluor, Chlor und/oder Bromatomen.
- R²⁶: steht besonders bevorzugt für Methyl, Ethyl, Trifluormethyl, Difluormethyl, Difluorchlormethyl oder Trichlormethyl.
- R²⁶: steht ganz besonders bevorzugt für Methyl, Trifluormethyl, Difluormethyl oder Trichlormethyl.
- R²⁷: steht bevorzugt für Methyl, Ethyl oder C₁-C₂-Halogenalkyl mit 1 bis 5 Fluor, Chlor und/oder Bromatomen.
- R²⁷: steht besonders bevorzugt für Methyl, Ethyl, Trifluormethyl, Difluormethyl, Difluorchlormethyl oder Trichlormethyl.
- R²⁷: steht ganz besonders bevorzugt für Methyl, Trifluormethyl, Difluormethyl oder Trichlormethyl.
- R²⁸ und R²⁹: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R²⁸ und R²⁹: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R²⁸ und R²⁹: stehen unabhängig voneinander ganz besonders bevorzugt für Wasserstoff, Fluor, Chlor, Brom, Methyl, Trifluormethyl, Difluormethyl oder Trichlormethyl.
- R²⁸ und R²⁹: stehen insbesondere bevorzugt jeweils für Wasserstoff.
- R³⁰: steht bevorzugt für Wasserstoff, Fluor, Chlor, Brom, Iod, Methyl, Ethyl oder C₁-C₂-Halogenalkyl mit 1 bis 5 Fluor, Chlor und/oder Bromatomen.
- R³⁰: steht besonders bevorzugt für Wasserstoff, Fluor, Chlor, Brom, Iod, Methyl, Ethyl, Trifluormethyl, Difluormethyl, Difluorchlormethyl oder Trichlormethyl.
- R³⁰: steht ganz besonders bevorzugt für Wasserstoff, Fluor, Chlor, Brom, Iod, Methyl, Trifluormethyl, Difluormethyl oder Trichlormethyl.
- R³⁰: steht insbesondere bevorzugt für Methyl.
- R³¹ und R³²: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Bromatomen.
- R³¹ und R³²: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl.
- R³¹ und R³²: stehen unabhängig voneinander ganz besonders bevorzugt für Wasserstoff, Fluor, Chlor, Brom, Methyl, Trifluormethyl, Difluormethyl oder Trichlormethyl.
- R³¹ und R³²: stehen insbesondere bevorzugt jeweils für Wasserstoff.
- R³³: steht bevorzugt für Wasserstoff, Fluor, Chlor, Brom, Methyl, Ethyl oder C₁-C₂-Halogenalkyl mit 1 bis 5 Fluor, Chlor und/oder Bromatomen,
- R³³: steht besonders bevorzugt für Wasserstoff, Fluor, Chlor, Brom, Methyl, Ethyl, Trifluormethyl, Difluormethyl, Difluorchlormethyl oder Trichlormethyl.
- R³³: steht ganz besonders bevorzugt für Wasserstoff, Fluor, Chlor, Brom, Methyl, Trifluormethyl, Difluormethyl oder Trichlormethyl.
- R³³: steht insbesondere bevorzugt für Methyl.
- R³⁴: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis. 5 Fluor, Chlor und/oder Bromatomen.
- R³⁴: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³⁴: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³⁴: steht insbesondere bevorzugt für Amino, Methylamino, Dimethylamino, Methyl oder Trifluormethyl.
- R³⁵: steht bevorzugt für Fluor, Chlor, Brom, Methyl, Ethyl oder C₁-C₂-Halogenalkyl mit 1 bis 5 Fluor, Chlor und/oder Bromatomen.
- R³⁵: steht besonders bevorzugt für Fluor, Chlor, Brom, Methyl, Ethyl, Trifluormethyl, Difluormethyl, Difluorchlormethyl oder Trichlormethyl.
- R³⁵: steht ganz besonders bevorzugt für Fluor, Chlor, Brom, Methyl, Trifluormethyl, Difluormethyl oder Trichlormethyl.
- R³⁵: steht insbesondere bevorzugt für Methyl, Trifluormethyl oder Difluormethyl.
- R³⁶: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R³⁶: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³⁶: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³⁶: steht insbesondere bevorzugt für Amino, Methylamino, Dimethylamino, Methyl oder Trifluormethyl.
- R³⁷: steht bevorzugt für Fluor, Chlor, Brom, Methyl, Ethyl oder C₁-C₂-Halogenalkyl mit 1 bis 5 Fluor, Chlor und/oder Bromatomen.
- R³⁷: steht besonders bevorzugt für Fluor, Chlor, Brom, Methyl, Ethyl, Trifluormethyl, Difluormethyl, Difluorchlormethyl oder Trichlormethyl.
- R³⁷: steht ganz besonders bevorzugt für Fluor, Chlor, Brom, Methyl, Trifluormethyl, Difluormethyl oder Trichlormethyl.
- R³⁷: steht insbesondere bevorzugt für Methyl, Trifluormethyl oder Difluormethyl.
- R³⁸: steht bevorzugt für Fluor, Chlor, Brom, Methyl, Ethyl oder C₁-C₂-Halogenalkyl mit 1 bis 5 Fluor, Chlor und/oder Bromatomen.
- R³⁸: steht besonders bevorzugt für Fluor, Chlor, Brom, Methyl, Ethyl, Trifluormethyl, Difluormethyl, Difluorchlormethyl oder Trichlorethyl.
- R³⁸: steht ganz besonders bevorzugt für Fluor, Chlor, Brom, Methyl, Trifluormethyl, Difluormethyl oder Trichlormethyl.
- R³⁹: steht bevorzugt für Wasserstoff, Methyl oder Ethyl.
- R³⁹: steht besonders bevorzugt für Methyl.
- R⁴⁰: steht bevorzugt für Fluor, Chlor, Brom, Methyl oder Ethyl,
- R⁴⁰: steht besonders bevorzugt für Fluor, Chlor oder Methyl.
- R⁴¹: steht bevorzugt für Methyl, Ethyl oder C₁-C₂-Halogenalkyl mit 1 bis 5 Fluor, Chlor und/oder Bromatomen.
- R⁴¹: steht besonders bevorzugt für Methyl, Ethyl, Trifluormethyl, Difluormethyl, Difluorchlormethyl oder Trichlormethyl.
- R⁴¹: steht ganz besonders bevorzugt, für Methyl, Trifluormethyl, Difluormethyl oder Trichlormethyl.
- R⁴¹: steht insbesondere bevorzugt für Methyl oder Trifluormethyl.
- R⁴²: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁴²: steht besonders bevorzugt für Wasserstoff, Fluor, Chlor, Brom, Methyl oder Trifluormethyl.
- R⁴³: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R⁴³: steht besonders bevorzugt, für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl oder Trichlormethyl.
- R⁴³: steht ganz besonders bevorzugt für Fluor, Chlor, Brom, Iod, Methyl, Trifluormethyl, Difluormethyl oder Trichlormethyl.
- R⁴⁴: steht bevorzugt für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl, Hydroxymethyl, Hydroxyethyl, Methylsulfonyl oder Dimethylaminosulfonyl.
- R⁴⁴: steht besonders bevorzugt für Wasserstoff, Methyl, Ethyl, Trifluormethyl, Methoxymethyl, Ethoxymethyl, Hydroxymethyl oder Hydroxyethyl.
- R⁴⁴: steht ganz besonders bevorzugt für Methyl oder Methoxymethyl.
- R⁴⁵: steht bevorzugt, für Wasserstoff, Fluor, Chlor, Brom, Methyl, Ethyl oder C₁-C₂-Halogenalkyl mit 1 bis 5 Fluor-, Chlor- und/oder Bromatomen.
- R⁴⁵: steht besonders bevorzugt für Wasserstoff, Fluor, Chlor, Brom, Methyl, Ethyl Trifluormethyl, Difluormethyl oder Trichlormethyl.
- R⁴⁵: steht ganz besonders bevorzugt für Wasserstoff oder Methyl.
- R⁴⁶: steht bevorzugt für Wasserstoff, Fluor, Chlor, Brom, Iod, Cyano, Methyl, Ethyl, iso-Propyl oder C₁-C₂-Halogenalkyl mit 1 bis 5 Fluor-, Chlor- und/oder Bromatomen.
- R⁴⁶: steht besonders bevorzugt für Wasserstoff, Fluor, Chlor, Brom, Cyano, Methyl, Ethyl, isoPropyl, Trifluormethyl, Difluormethyl, Difluorchlormethyl oder Trichlormethyl.
- R⁴⁶: steht ganz besonders bevorzugt für Wasserstoff, Methyl, Difluormethyl oder Trifluormethyl.
- R⁴⁷: steht bevorzugt für Wasserstoff, Fluor, Chlor, Brom, Methyl, Ethyl oder C₁-C₂Halogenalkyl mit 1 bis 5 Fluor, Chlor und/oder Bromatomen.
- R⁴⁷: steht besonders bevorzugt für Wasserstoff, Fluor, Chlor, Brom, Iod, Methyl oder Trifluormethyl.
- R⁴⁷: steht ganz besonders bevorzugt für Wasserstoff.
- R⁴⁸: steht bevorzugt für Methyl, Ethyl, n-Propyl oder iso-Propyl.
- R⁴⁸: steht besonders bevorzugt Methyl oder Ethyl.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Bevorzugt und jeweils als Teilmenge der oben genannten Verbindungen der Formel (I) zu verstehen sind folgende Gruppen von neuen Carboxamiden:
Gruppe 1: Carboxamide der Formel (I-a) 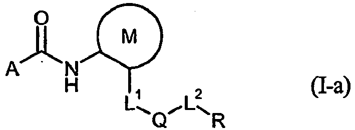 in welcher M, L¹, Q, L², R und A die oben angegebenen Bedeutungen haben.
Gruppe 2: Carboxamide der Formel (I-b) 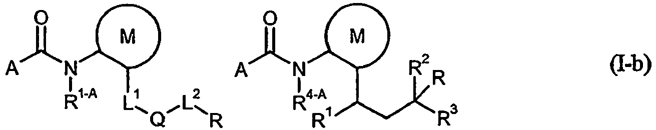 in welcher R^{1-A}, M, L¹, Q, L², R und A die oben angegebenen Bedeutungen haben.
   - R^{1-A}: steht bevorzugt für C₁-C₆-Alkyl, C₁-C₄-Alkylsulfinyl, C₁-C₄-Alkysulfonyl, C₁-C₃-Alkoxy-C₁-C₃-alkyl, C₃-C₆-Cycloakyl; C₁-C₄-Halogenalky, C₁-C₄-Halogenalkylthio, C₁-C₄-Halogenalkylsulfnyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
   (C₁-C₆-Alkyl)carbonyl (C₁-C₄-Alkoxy)carbonyl, (C₁-C₃ Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆Halogencycloalkyl)carbonyl mit jeweils 1 bis 9 Fluor-, Chlor- und/oder Bromatomen; oder -C(=O)C(=O)R², -CONR³R⁴ oder -CH₂NR⁵R⁶.
   - R^{1-A}: steht besonders bevorzugt für Methyl, Ethyl, n- oder iso-Propyl, n-, iso-, sec- oder tert Butyl, Pentyl oder Hexyl, Methylsulfinyl, Ethylsulfinyl, n- oder iso-Propy)sulfinyl, n-, iso-, sec- oder tert-Butylsulfinyl, Methylsulfonyl, Ethylsulfonyl, n- oder iso-Propylsulfonyl, n-, iso-, sec- oder tert Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Di-fluorchlormethylthio, Trifluormethylthio, Trifluormethylsulfinyl, Trifluormethylsulfonyl, Tri-fluormethoxmethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂,-CH₂-CO-CF₃, -CH₂-CO-CCl₃, -CH₂CO-CH₂CF₃, -CH₂-CO-CH₂CCl₃, -(CH₂)₂-CO-CH₂CF₃, -(CH₂)₂-CO-CH₂CO₃, -CH₂CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(-O)C(-O)R², -CONR³R⁴ oder -CH₂NR⁵R⁶.
   - R^{1-A}: steht ganz besonders bevorzugt für Methyl, Methoxymethyl, Formyl, -CH₂-CHO, -(CH₂)₂-CHO, -CH₂-CO-CH₃, -CH₂-CO-CH₂CH₃, -CH₂-O-CH(CH₃)₂, -C(=O)CHO, -C(=O)C(=O)CH₃, -C(=O)C(=O)CH₂OCH₃, -C(=O)CO₂CH₃, -C(O)CO₂CH₂CH₃.
Gruppe 3: Carboxamide der Formel (I-c) 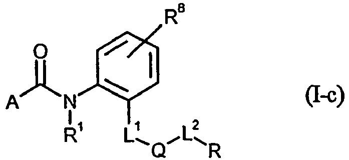 in welcher R¹, R⁸, L¹, Q, L², R und A die oben angegebenen Bedeutungen haben.
   Bevorzugt sind Carboxamide der Formel (I-c), in welcher R¹ für Wasserstoff steht.
   Bevorzugt sind Carboxamide der Formel (I-c), in welcher R⁸ für Wasserstoff steht
   Bevorzugt sind Carboxamide der Formel (I-c), in welcher R¹ und R⁸ jeweils für Wasserstoff stehen.
Gruppe 4: Carboxamide der Formel (I-d) 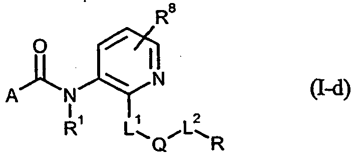 in welcher R¹, R⁸, L¹, Q, L², R und A die oben angegebenen Bedeutungen haben.
   Bevorzugt sind Carboxamide der Formel (I-d), in welcher R¹ für Wasserstoff steht.
   Bevorzugt sind Carboxamide der Formel (I-d), in welcher R⁸ für Wasserstoff steht
   Bevorzugt sind Carboxamide der Formel (I-d), in welcher R¹ und R⁸ jeweils für Wasserstoff stehen.
Gruppe 5: Carboxamide der Formel (I-e) 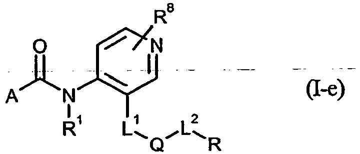 in welcher R¹, R⁸, L¹,Q, L², R und A die oben angegebenen Bedeutungen haben.
   Bevorzugt sind Carboxamide der Formel (I-e), in welcher R¹ für Wasserstoff steht
   Bevorzugt sind Carboxamide der Formel (I-e), in welcher R⁸ für Wasserstoff steht
   Bevorzugt sind Carboxamide der Formel (I-e), in welcher R¹ und R⁸ jeweils für Wasserstoff stehen.
Gruppe 6: Carboxamide der Formel (I-f) 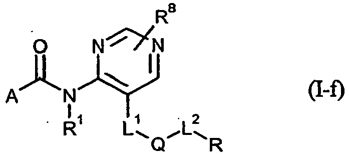 in welcher R¹, R⁸, L¹, Q, L², R und A die oben angegebenen Bedeutungen haben.
   Bevorzugt sind Carboxamide der Formel (I-f), in welcher R¹ für Wasserstoff steht.
   Bevorzugt sind Carboxamide der Formel (I-f), in welcher R⁸ für Wasserstoff steht
   Bevorzugt sind Carboxamide der Formel (I-f), in welcher R¹ und R⁸ jeweils für Wasserstoff stehen.
Gruppe 7: Carboxamide der Formel (I-g) 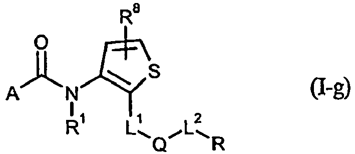 in welcher R¹, R⁸, L¹, Q, L², R und A die oben angegebenen Bedeutungen haben.
   Bevorzugt sind Carboxamide der Formel (I-g), in welcher R¹ für Wasserstoff steht
   Bevorzugt sind Carboxamide der Formel (I-g), in welcher R⁸ für Wasserstoff steht
   Bevorzugt sind Carboxamide der Formel (I-g), in welcher R¹ und R⁸ jeweils für Wasserstoff stehen.

Hervorgehoben sind Verbindungen der Formel (I) (und ebenso der Gruppen 1 bis 7), in welcher R¹ für Wasserstoff steht.
Hervorgehoben sind Verbindungen der Formel (I) (und ebenso der Gruppen 1 bis 7), in welcher R¹ für Formyl steht
Hervorgehoben sind außerdem Verbindungen der Formel (I) (und ebenso der Gruppen 1 bis ⁷), in welcher R¹ für -C(=O)C(=O)R² steht, wobei R² die oben angegebenen Bedeutungen hat

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein. Ebenfalls können doppelt gebundene Kohlenwasserstoffreste wie Alkylen (Alkandiyl) soweit möglich jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. So schließt die Definition Dialkylamino auch eine unsymmetrisch durch Alkyl substituierte Aminogruppe wie z.B. Methyl-ethylamino ein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend. Insbesondere können die in den Gruppen 1 bis 6 genannten Verbindungen sowohl mit den allgemeinen wie auch mit bevorzugten, besonders bevorzugten usw. Bedeutungen kombiniert werden, wobei auch hier jeweils alle Kombinationen zwischen den Vorzugsbereichen möglich sind.

### Beschreibung der erfindungssemäßen Verfahren zum Herstellen der Hexylearboxanilide der Formel (I) sowie der Zwischenprodukte

### Verfahren (a)

Verwendet man 2-Trifluormethylbenzoesäurechlorid und {2-[1-(Isopropylsofonyl)ethyl]phenyl)-amin als Ausgangsstoffe, so kann das erfindungsgemäße Verfahren (a) durch das folgende Formelschema veranschaulicht werden:

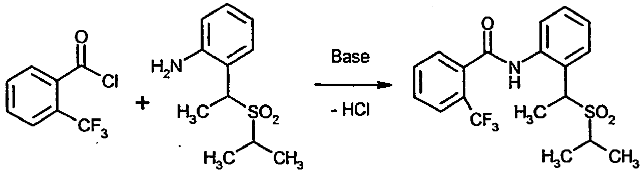

Die zur Durchführung des erfindungsgemäßen Verfahrens (a) als Ausgangsstoffe benötigten Carbonsäure-Derivate sind durch die Formel (II) allgemein definiert. In dieser Formel (II) hat A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden. X¹ steht bevorzugt für Chlor, Brom oder Hydroxy.

Die Carbonsäure-Derivate der Formel (II sind größtenteils bekannt und/oder lassen sich nach bekannten Verfahren herstellen (vgl. WO 93/11117, EP-A 0 545 099, EP-A 0 589 301 und EP-A 0 589 313).

Die zur Durchführung des erfindungsgemäßen Verfahrens (a) als Ausgangsstoffe weiterhin benötigten Anilin-Derivate sind durch die Formel (III) allgemein definiert. In dieser Formel (III) haben R¹, M, Q, L² und R bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden. L³ steht bevorzugt für Wasserstoff oder C₁-C₅-Alkyl, besonders bevorzugt für Wasserstoff oder Methyl.

Die Anilin-Derivate der Formel (III) sind neu.

Anilin-Derivate der Formel (III-a) 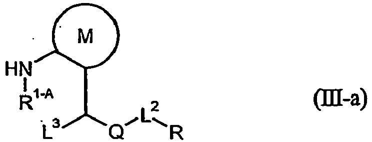 in welcher R^{1-A}, M, Q, L², R und L³ die oben angegebenen Bedeutungen haben, werden erhalten, indem man
(d) Anilin-Derivate der Formel (III-b) 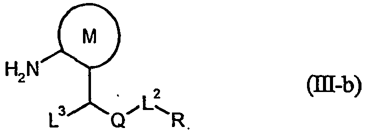 in welcher M, Q, L², R und L³ die oben angegebenen Bedeutungen haben,
   mit Halogeniden der Formel (VI)

   R^{1-A}- X² (VI)

   in welcher R^{1-A} und X² die oben angegebenen Bedeutungen haben,
   in Gegenwart einer Base und in Gegenwart eines Verdünnungsmittels umsetzt.

Anilin-Derivate der Formel (III-b) werden erhalten, indem man
(e) eine Nitro-Verbindung der Formel (VII) 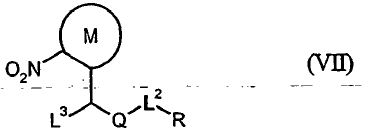 in welcher M, Q, L², R und L³ die oben angegebenen Bedeutungen haben,
   in Gegenwart eines Metalls und eines Reduktionsmittels sowie gegebenenfalls in Gegenwart eines Verdünnungsmittels und gegebenenfalls in Gegenwart eines weiteren Reaktionshilfsmittels umsetzt

Die zur Durchführung des erfindungsgemäßen Verfahrens (e) als Ausgangsstoffe benötigten NitroVerbindungen sind durch die Formel (VII) allgemein definiert. In dieser Formel (VII) haben M, Q, L², R und L³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) bzw. der Formel (III) als bevorzugt, besonders bevorzugt bzw. ganz besonders bevorzugt für diese Reste angegeben wurden.

Nitro-Verbindungen der Formel (VII) werden erhalten, indem man
(f) eine Nitro-Verbindung der Formel (VIII) 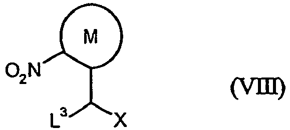 in welcher
   - M und L³: die oben angegebenen Bedeutungen haben,
   - X: für Chlor, Brom oder Iod steht,
   mit einer Verbindung der Formel (IX) 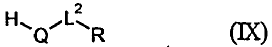 in welcher Q, L² und R die oben angegebenen Bedeutungen haben,
   in Gegenwart einer Base und gegebenenfalls in Gegenwart eines Verdünnungsmittels umsetzt.

Die zur Durchführung des erfindungsgemäßen Verfahrens (f) als Ausgangsstoffe benötigten Nitro-Verbindungen sind durch die Formel (VID) allgemein definiert. In dieser Formel (VIII) haben M und L³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) bzw. der Formel (III) als bevorzugt, besonders bevorzugt bzw. ganz besonders bevorzugt für diese Reste angegeben wurden. X steht bevorzugt für Chlor.

Die zur Durchführung des erfindungsgemäßen Verfahrens (f) weiterhin als Ausgangsstoffe benötigten Verbindungen sind durch die Formel (IX) allgemein definiert. In dieser Formel (IX) haben Q, L² und R bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) bzw. der Formel (III) als bevorzugt, besonders bevorzugt bzw. ganz besonders bevorzugt für diese Reste angegeben wurden.

Verbindungen der Formel (IX) sind bekannt oder können nach bekannten Verfahren erhalten werden.

Nitro-Verbindungen der Formel (VII-a) 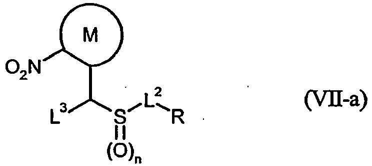 in welcher
- M, L², R und L³: die oben angegebenen Bedeutungen haben,
- n: für 1 oder 2 steht,
werden erhalten, indem man
(g) eine Nitro-Verbindung der Formel (VII-b) 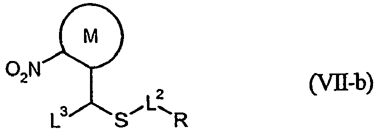 in welcher M, L², R und L³ die oben angegebenen Bedeutungen haben,
   in Gegenwart eines Oxidationsmittels sowie gegebenenfalls in Gegenwart eines Verdünnungsmittels und gegebenenfalls in Gegenwart eines weiteren Reaktionshilfsmittels umsetzt.

Nitro-Verbindungen der Formel (VII-c) 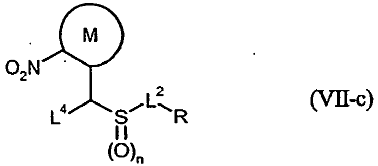 in welcher
- M, L², R und n: die oben angegebenen Bedeutungen haben,
- L⁴: für C₁-C₉-Alkyl, bevorzugt für C₁-C₅-Alkyl, besonders bevorzugt für Methyl steht,
werden erhalten, indem man
(h) eine Nitro-Verbindung der Formel (VII-d) 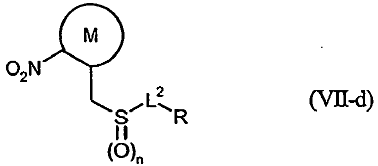 in welcher M, L², R und n die oben angegebenen Bedeutungen haben,
   mit Halogeniden der Formel (X)

   L⁴-X² (X)

   in welcher L⁴ und X² die oben angegebenen Bedeutungen haben,
   in Gegenwart einer Base und in Gegenwart eines Verdünnungsmittels umsetzt..

Halogenide der Formel (X) sind bekannt.

Die Verbindungen der Formeln (VII-a), (VII-b), (VII-c) und (VII-d) sind Untergruppen der NitroVerbindungen der Formel (VII) und werden von der allgemeinen Beschreibung dieser Verbindungen umfasst. Die bevorzugten, besonders bevorzugten usw. Definitionen gelten hier entsprechend.

Nitro-Verbindungen der Formel (VIII) werden erhalten, indem man
(j) Hydroxy-Derivate der Formel (XI) 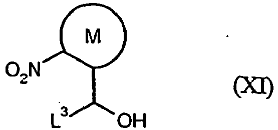 in welcher M und L³ die oben angegebenen Bedeutungen haben,
   gegebenenfalls in Gegenwart eines Verdünnungsmittels, gegebenenfalls in Gegenwart eines Säureakzeptors und gegebenenfalls in Gegenwart eines Katalysators halogeniert.

Die zur Durchführung des erfindungsgemäßen Verfahrens (j) als Ausgangsstoffe benötigten Hydroxy-Derivate sind durch die Formel (XI) allgemein definiert. In dieser Formel (XI) haben M und L³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) bzw. der Formel (III) als bevorzugt, besonders bevorzugt bzw. ganz besonders bevorzugt für diese Reste angegeben wurden.

Hydroxy-Derivate der Formel (XI werden erhalten, indem man
(k) acrylierte Aromaten der Formel (XII) 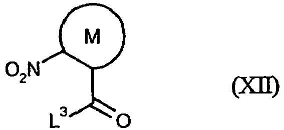
in welcher M und L³ die oben angegebenen Bedeutungen haben,
in Gegenwart eines Reduktionsmittels sowie gegebenenfalls in Gegenwart eines Verdünnungsmittels, gegebenenfalls in Gegenwart einer Säure und gegebenenfalls in Gegenwart eines Katalysators umsetzt.

Anilin-Derivate der Formel (III) können auch in Analogie zu bekannten Verfahren erhalten werden (vgL EP-A 0 737 682).

### Verfahren (b)

Verwendet man N-[2-(Hydroxymethyl)phenyl]-1-methyl-3-(trifluormethyl)-1H-pyrazol-4-carboxamid und 2-Iodpropan als Ausgangsstoffe, so kann das erfindungsgemäße Verfahren (b) durch das folgende Formelschema veranschaulicht werden:

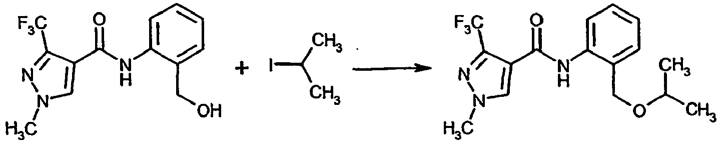

Die zur Durchführung des erfindungsgemäßen Verfahrens (b) als Ausgangsstoffe benötigten Carboxamide sind durch die Formel (IV) allgemein definiert. In dieser Formel (IV) haben M, L¹, Q und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden.

Die zur Durchführung des erfindungsgemäßen Verfahrens (b) weiterhin als Ausgangsstoffe benötigten Verbindungen sind durch die Formel (V) allgemein definiert, In dieser Formel (V) haben L² und R bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden. Y steht bevorzugt für Chlor, Brom, Iod, Triflat (Trifluormethylsulfonyl), Mesylat (Methylsulfonyl) oder Tosylat (4-Methylphenylsulfonyl), besonders bevorzugt für Brom, Iod oder Triflat (Trifluormethylsulfonyl).

Verbindungen der Formel (V) sind bekannt oder können nach bekannten Verfahren erhalten werden. Carboxamide der Formel (IV) sind neu. Sie werden erhalten, indem man
(m) Carbonsäure-Derivate der Formel (II) 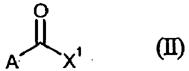 in welcher
   - A: die oben angegebenen Bedeutungen hat und
   - X¹: für Halogen oder Hydroxy steht,
   mit Anilin-Derivaten der Formel (XIII) 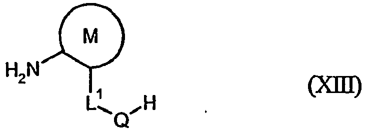 in welcher M, L¹ und Q die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt.

Die zur Durchführung des erfindungsgemäßen Verfahrens (m) als Ausgangsstoffe benötigten Carbonsäure-Derivate der Formel (II) sind bereits in Zusammenhang mit dem erfindungsgemäßen Verfahren (a) beschrieben worden.

Die zur Durchführung des erfindungsgemäßen Verfahrens (m) als Ausgangsstoffe weiterhin benötigten Anilin-Derivate sind durch die Formel (XIII) allgemein definiert. In dieser Formel (XIII) haben M, L¹ und Q bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Anilin-Derivate der Formel (XIII) sind bekannt oder können nach bekannten Verfahren erhalten werden.

### Verfahren (c)

Verwendet man 3-(Difluormethyl)-N-{2-[(isopropylthio)methyl]phenyl}-1-methyl-1H-pyrazol-4-carboxamid und Ethyl-chlor(oxo)acetat als Ausgangsstoffe, so kann der Verlauf des erfindungsgemäßen Verfahrens (c) durch das folgende Formelschema veranschaulicht werden:

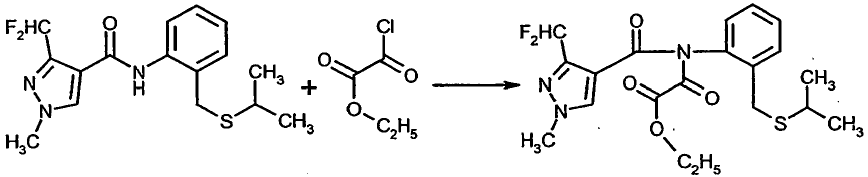

Die zur Durchführung des erfindungsgemäßen Verfahrens (c) als Ausgangsstoffe benötigten Hexylcarboxanilide sind durch die Formel (I-a) allgemein definiert. In dieser Formel (I-a) haben M, L¹, Q, L², R und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Hexylcarboxanilide der Formel (I-a) sind ebenfalls erfindungsgemäße Verbindungen und Gegenstand dieser Anmeldung. Sie können nach dem erfindungsgemäßen Verfahren (a) erhalten werden (mit R¹ = Wasserstoff).

Die zur Durchführung des erfindungsgemäßen Verfahrens (c) als Ausgangsstoffe weiterhin benötigten Halogenide sind durch die Formel (VI) allgemein definiert. In dieser Formel (VI) hat R^{1-A} bevorzugt, besonders bevorzugt bzw. ganz besonders bevorzugt diejenigen Bedeutungen, die bereits im Zusammenhang mit der Beschreibung der erfindungsgemäßen Verbindungen der Formel (I-b) als bevorzugt, besonders bevorzugt bzw. ganz besonders bevorzugt für diesen Rest angegeben wurden. X² steht bevorzugt für Chlor oder Brom.

Halogenide der Formel (VI) sind bekannt.

### Reaktionsbedingungen

Als Verdünnungsmittel zur Durchführung der erfindungsgemäßen Verfahren (a) und (m) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. chlorbenzoyl, Dichlorbenzol, Dichlormethan, Chloroform, Tetrachlormethan, Dichlorethan oder Tri-chlorethan; Ether, wie Diethylether, Diisopropylether, Methyl-tert-butylether, Methyl-tert-Amylether, Dioxan, Tetrahydrofuran, 1,2 Dimethoxyethan, 1,2-Diethoxyetban oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren (a) und (m) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert. butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren (a) und (m) werden gegebenenfalls in Gegenwart eines geeigneten Kondensationsmittels durchgeführt. Als solche kommen alle üblicherweise für derartige Amidie-rungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid, Oxalylchlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2--dihydrochinolin (EEDQ), Triphenylphosphin/Tetrachlorkohlenstoff oder Brom-tripyrrolidinophosphonium-hexafluorophosphat.

Die erfindungsgemäßen Verfahren (a) und (m) werden gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung der erfindungsgemäßen Verfahren (a) und (m) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro mol des Carbonsäure-Derivates der Formel (II) im Allgemeinen 0,2 bis 5 mol, vorzugsweise 0,5 bis 2 mol an Anilin-Derivat der Formel (III) ein.

Zur Durchführung des erfindungsgemäßen Verfahrens (m) zur Herstellung der Verbindungen der Formel (IV) setzt man pro mol des Carbonsäure-Derivates der Formel (TI) im Allgemeinen 0,2 bis 5 mol, vorzugsweise 0,5 bis 2 mol an Anilin-Derivat der Formel (XIII) ein.

Als Verdünnungsmittel zur Durchführung der erfindungsgemäßen Verfahren (b), (c), (d) und (h) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert butylether, Methyltert-Amylether, Dioxan, Tetrahydrofuran, 1,2-Dimethoxyethan 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren (b), (c), (d) und (h) werden in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholat, -acetate, -carbonate oder -hydrogencarbonate, wie z.B. Natriumhydrid, Natriumamid, Natrium-methylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (b), (c), (d) und (h) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des Carboxamids der Formel (IV) im Allgemeinen 0,2 bis 5 Mol, vorzugsweise 0,5 bis 2 Mol an einer Verbindung der Formel (V) ein.

Zur Durchführung des erfindungsgemäßen Verfahrens (c) zur Herstellung der Verbindungen der Formel (I) setzt man pro Mol des Hexylcarboxanilids der Formel (I-a) im Allgemeinen 0,2 bis 5 Mol, vorzugsweise 0,5 bis 2 Mol an Halogenid der Formel (VI) ein.

Zur Durchführung des erfindungsgemäßen Verfahrens (d) zur Herstellung der Verbindungen der Formel (III-a) setzt man pro Mol des Anilin-Derivates der Formel (III b) im Allgemeinen 0,2 bis 5 Mol, vorzugsweise 0,5 bis 2 Mol an Halogenid der Formel (VI) ein.

Zur Durchführung des erfindungsgemäßen Verfahrens (h) zur Herstellung der Verbindungen der Formel (VII-c) setzt man pro Mol einer Nitro-Verbindung der Formel (VII-d) im Allgemeinen 0,2 bis 5 Mol, vorzugsweise 0,5 bis 2 Mol an Halogenid der Formel (X) ein.

Als Verdünnungsmittel zur Durchführung des erfindungsgemäßen Verfahrens (e) kommen alle inerten organischen Lösungsmittel in Betracht Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Düsopropylether, Methyl-t-butylether, Methyl-tert-Amylether, Dioxan, Tetrahydrofuran, 1,2 Dimethoxyethan, 1,2 Diethoxyethan oder Anisol; Amide, wie N,N-Dimethylformamid, N,N-Dimethylacetamid, N-Methylformanilid, N-Methylpyrrolidon oder Hexamethylphosphorsäuretriamid, Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethy lenglykolmonoethylether, Triethylenglykol, deren Gemische mit Wasser oder reines Wasser.

Das erfindungsgemäße Verfahren (e) wird in Gegenwart eines Metalls durchgeführt. Als solche kommen vorzugsweise Übergangsmetalle, wie beispielsweise Palladium, Platin, Rhodium, Nickel (Raney-Nickel), Eisen, Cobalt, Ruthenium, Iridium, Zink, oder Osmium infrage. Die Metalle können gegebenenfalls an Trägermaterialien, wie z. B. Kohle, Harze, Zeolithe, Alkali- oder Erdalkalisulfate gebunden sein.

Das erfindungsgemäße Verfahren (e) wird in Gegenwart eines Reduktionsmittels durchgeführt. Als solche kommen vorzugsweise elementarer Wasserstoff, Formiatsalze, vorzugsweise Alkaliformiatsalze, wie z. B: Natriumformiat, aber auch Ammoniuniformiat oder auch Metallhydride bzw. komplexe Metallhydride, wie z.B. Lithiumaluminiumhydrid, Natriumborhydrid infrage.

Das erfindungsgemäße Verfahren (e) kann in Gegenwart von Säuren durchgeführt werden. Als solche kommen vorzugsweise organische Säuren, wie z. B. Ameisensäure, Essigsäure, Ascorbinsäure, aber auch Mineralsäuren, wie z.B. Salzsäure oder Schwefelsäure infrage.

Das erfindungsgemäße Verfahren (e) kann in Gegenwart von Basen durchgeführt werden. Als solche kommen vorzugsweise organische Basen, wie z. B. Pyridin, aber auch wässrige Lösungen von Alkali- oder Erdalkalimetallhydroxiden, wie z.B. Natriumhydroxid oder Bariumhydroxid infrage.

Die Reaktionstemperaturen können bei der Durchführung des erfindungsgemäßen Verfahrens (e) in einem größeren Bereich variiert werden. Im allgemeinen arbeitet man bei Temperaturen von -80°C bis 300°C, vorzugsweise bei Temperaturen von 0°C bis 200°C.

Bei der Verwendung von elementarem Wasserstoff wird das erfindungsgemäße Verfahren (e) unter einem Wasserstoffdruck zwischen 0,5 and 200 bar, bevorzugt zwischen 1 und 100 bar durchgeführt.

Zur Durchführung des erfindungsgemäßen Verfahrens (e) zur Herstellung der Verbindungen der Formel (III-b) setzt man pro Mol einer Nitro-Verbindung der Formel (VII) im Allgemeinen 0,8 bis 1000 Mol, vorzugsweise 1 bis 500 Mol an Reduktionsmittel (Ammoniumformiat, Hydrid etc.) ein.

Als Verdünnungsmittel zur Durchführung des erfindungsgemäßen Verfahren (f) kommen alle inerten organischen Lösungsmittel in Betracht Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (f) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide -alkoholate, -acetate, -carbonate oder hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahren (f) in einem größeren Bereich variiert werden. Im allgemeinen arbeitet man bei Temperaturen von 0°C bis 200°C, vorzugsweise bei Temperaturen von 20°C bis 150°C.

Zur Durchführung des erfindungsgemäßen Verfahrens (f) zur Herstellung der Verbindungen der Formel (VII) setzt man pro Mol einer Nitro-Verbindung der Formel (VIII) im Allgemeinen 0,2 bis 5 Mol, vorzugsweise 0,5 bis 2 Mol einer Verbindung der Formel (IX) ein.

Als Verdünnungsmittel zur Durchführung des erfindungsgemäßen Verfahrens (g) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid, Sulfone, wie Sulfolan.

Das erfindungsgemäße Verfahren (g) wird in Gegenwart eines Oxidationsmittels durchgeführt. Als solche kommen alle organischen und anorganischen Oxidationsmittel infrage, vorzugsweise elementarer Sauerstoff, Ozon, Peroxide, wie z.B. Wasserstoffperoxid, m-Chlorperbenzoesäure, Benzoylperoxid, tert-Butylperoxid; Chlorlauge (Natriumhypochlorid); Chromsalze wie z.B. Chrom(VI)-oxid, Chromsäure, Natriumdichromat, Pyridiniumchlorochromat; Mangansalze, wie z.B. Kaliumpermanganat, Braunstein; Selendioxid; Iodate und Periodate; Kaliumperoxodisulfat.

Das erfindungsgemäße Verfahren (g) kann in Gegenwart von Säuren durchgeführt werden. Als solche kommen vorzugsweise organische Säuren, wie z. B. Ameisensäure, Essigsäure, Ascorbinsäure, aber auch Mineralsäuren, wie z.B. Salzsäure oder Schwefelsäure infrage.

Das erfindungsgemäße Verfahren (g) kann in Gegenwart von Basen durchgeführt werden. Als solche kommen vorzugsweise organische Basen, wie z. B. Pyridin, aber auch wässrige Lösungen von Alkali- oder Erdalkalimetallhydroxiden, wie z.B. Natriumhydroxid oder Bariumhydroxid infrage.

Die Reaktionstemperaturen können bei der Durchführung des erfindungsgemäßen Verfahrens (g) in einem größeren Bereich variiert werden. Im Allgemeinen arbeitet man bei Temperaturen von -80°C bis 300°C, vorzugsweise bei Temperaturen von -20°C bis 100°C.

Zur Durchführung des erfindungsgemäßen Verfahrens (g) zur Herstellung der Verbindungen der Formel (VII-a) setzt man pro Mol einer Nitro-Verbindung der Formel (VII-b) im Allgemeinen 0,6 bis 10 Mol, vorzugsweise 0,8 bis 5 Mol an Oxidationsmittel ein.

Als Verdünnungsmittel zur Durchführung des erfindungsgemäßen Verfahrens (j) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (j) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tnbutylamin, N,N-Dimethylanilin, N,N-Dimethyl-benzylamin, Pyridin, N-Methylpipezidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (j) wird in Gegenwart eines geeigneten Halogenierungsmittels durchgeführt. Als solche kommen alle üblicherweise für derartige Halogenierungsreaktionen verwendbaren Halogenierungsmittel infrage. Beispielhaft genannt seien Halogenidbildner wie Phosgen, Phosphortribromid, Phosphortrichlorid, Phosphorpentachlorid, Phosphoroxychlorid, Oxalylchlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Das erfindungsgemäße Verfahren (j) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens (j) in einem größeren Bereich variiert werden. Im allgemeinen arbeitet man bei Temperaturen von 0°C bis 200°C, vorzugsweise bei Temperaturen von 0°C bis 150°C.

Zur Durchführung des erfindungsgemäßen Verfahrens (j) zur Herstellung der Verbindungen der Formel (VIII) setzt man pro mol eines Hydroxy-Derivates der Formel (XI) im Allgemeinen 0,2 bis 10 mol, vorzugsweise 0,5 bis 5 mol an Halogenierungsmittel ein.

Als Verdünnungsmittel zur Durchführung des erfindungsgemäßen Verfahrens (k) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, iso-Propanol.

Das erfindungsgemäße Verfahren (k) wird in Gegenwart eines geeigneten Reduktionsmittels durchgeführt. Als solche kommen alle üblichen anorganischen oder organischen Reduktionsmittel infrage. Hierzu gehören vorzugsweise Erdalkalimetall- oder Alkalimetallhydride, wie z.B. Natriumhydrid, oder komplexe Hydride, wie z.B. Lithiumaluminiumhydrid, Natriumborhydrid, Natriumcyanoborhydrid, Diisobutylaluminiumhydrid, Boran, Diboran oder Borankomplexe, wie z.B. Boran-Pyridin, Silane, wie z.B. Triethylsilan, Metalle, wie z.B. Natrium, Lithium, Zink, Eisen, oder Wasserstoff.

Das erfindungsgemäße Verfahren (k) wird gegebenenfalls in Gegenwart einer geeigneten Säure oder Lewissäure durchgeführt. Als solche kommen alle üblicherweise für derartige Säure/Lewissäure vermittelten Reduktionen verwendbaren Säuren/Lewissäuren infrage. Beispielhaft genannt seien Salzsäure, Essigsäure, Trifluoressigsäure, Bortrifluorid oder komplexe Bortrifluoride, wie z.B. Bortrifluoridetherat, Aluminiumtrichlorid, Certrichlorid, anorganische oder organische Titanverbindungen, wie z.B. Titantetrachlorid, Titantetraisopropylat.

Das erfindungsgemäße Verfahren (k) wird gegebenenfalls in Gegenwart eines Katalysators durchgeführt. Beispielsweise genannt seien Metalle oder Metallsalze, insbesondere Übergangsmetalle oder deren Salze, wie z.B. Platin, Palladium, Nickel (Raney-Nickel), Iridium, Rhodium, Osmium, Eisen, Ruthenium, Cobalt. Diese Metalle bzw. Metallsalze können gegebenenfalls auch an Harze oder Oberflächen bzw. Trägermaterialien (z. B. Kohle) gebunden oder aufgetragen sein.

Die Reaktionstemperaturen können bei der Durchführung des erfindungsgemäßen Verfahrens (k) in einem größeren Bereich variiert werden. Im allgemeinen arbeitet man bei Temperaturen von 0°C bis 200°C, vorzugsweise bei Temperaturen von 0°C bis 150C.

Bei der Verwendung von Wasserstoff als Reduktionsmittel kann das erfindungsgemäße Verfahren (k) in einem größeren Druckbereich variiert werden. Im Allgemeinen arbeitet man bei Drücken von 1 bar bis 300 bar, vorzugsweise bei 1 bar bis 100 bar.

Zur Durchführung des erfindungsgemäßen Verfahrens (k) zur Herstellung der Verbindungen der Formel (XI) setzt man pro mol eines acylierten Aromaten der Formel (XII) im Allgemeinen 0,2 bis 10 mol, vorzugsweise 0,5 bis 5 mol an Reduktionsmittel ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Atten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Alternaria brassicae;
Pseudocercosporella-Arten, wie beispielsweise Pseudocercosporella heipotrichoides,
Rhizoctonia-Arten, wie beispielsweise Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünscht Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanzund Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Altemaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor microbieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimoiganismen genannt Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Potyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychmolinsulfat; Acibenzolar-S-methyl; Aldimorph; Amidoflumet, Ampropylfos; Ampropylfos-potassium; Andoprim; Anilazine; Azaconazole; Azoxystrobin; Benalaxyl; Benalaxyl-M; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazol; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenaripimorph; Ferbam; Fluazinam; Flubenzimine; Fludioxonil; Flumetover; Flumorph; Fluoromide; Fluoxastrobin; Fluquinconazole; Fluiprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesiate)1; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozlin; Natamycin; Nicobifen; Nitromal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Sphoxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofosmethyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-arboxylat; Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper, Oxine-copper.

### Bakterizide:

Bronopol, Dichlorophen, Nitrapyrin, Nickel-dimethyldithiocarbamat, Kasugamycin, Octhilinou, Furancarbonsäure, Oxytetracyclin, Probenazol, Streptomycin, Tecloftalam, Kupfersulfat und andere Kupfer-Zubereitungen.

### Insektizid / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonisten*/*-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avermectin, Emamectin, Emamectin-benzoate, Ivermectin, Milbemectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazaron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Seite-I-Elektronentransportinhibitoren*
   12.1 METTs (z.B. Fenazaquin, Fenpyroximate, Pyrimidifen, Pyridaben, Tebufenpyrad, Tolfenpyrad)
   12.2 Hydramethylnone
   12.3 Dicofol
*13. Seite-II-Elektronentransportinhibitoren*
   13.1 Rotenone
*14. Seite-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsäuren [z.B. 3-(2,5-Dimethylphenyl)-8-methoxy-2-oxo-1-azaspiro[4.5]dec-3-en-4yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Phthalamide*
   (z.B. N²-[1,1-Dimethyl-2-(methylsulfonyl)ethyl]-3-iod-N¹-[2-methyl-4-[1,2,2,2-tetrafluor-1-(trifluor-methyl)ethyl]phenyl]-1,2-benzenediearboxamide (CAS-Reg.-No.: 272451-65-7), Flubendiamide)
*21. Nereistoxin Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin,
   ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.
Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermätophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasserbzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Wiezen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihende Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Herstellung von Verbindung Nr. 40

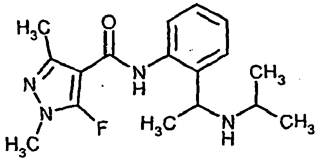

Zu einer Lösung bestehend aus 0,27 g (1,5 mmol) 2-[1-(Isopropylamino)ethyl]anilin (III-4) und 0,42 ml (3,0 mmol) Triethylamin in 5 ml Dichlormethan wird eine Lösung bestehend aus 0,27 g (1,5 mmol) 5-Fluor-1,3-dimethyl-1H-pyrazol-4-carbonsäurechlorid in 10 ml Dichlormethan zugetropft. Die Reaktionsmischung wird 2 Stunden bei 50°C und anschließend 16 h bei Raumtemperatur gerührt. Zur Aufarbeitung wird die Reaktionsmischung auf Wasser gegeben, die organische Phase über Magnesiumsulfat getrocknet und im Vakuum aufkonzentriert. Säulenchromatographie (Hexan/Aceton 4 : 1) liefert 0,27 g (56 % der Theorie) 5-Fluor-N-{2-[1-(isopropylamino)ethyl]phenyl}-1,3-dimethyl-1H-pyrazol-4-carboxamid [log P (pH 2,3) = 0,58].

### Herstellung von Verbindung Nr. 60

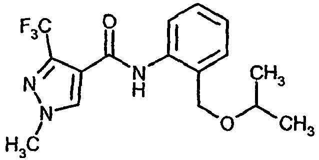

Bei Raumtemperatur werden 156,0 mg (3,9 mmol) 60%iges Natriumhyrid in Öl zu einer Lösung bestehend aus 897,8 mg (3,0 mmol) N-[2-(Hydroxymethyl)phenyl]-1-methyl-3-(trifluormethyl)-1H-pyrazol-4-carboxamid (IV-1) in 2 ml Dimethylformamid gegeben. Nach 30 Minuten werden 0,6 ml (6,0 mmol) 2-Iodpropan zugegeben. Die Reaktionsmischung wird 6 Stunden bei 100°C und 16 Stunden bei Raumtemperatur gerührt. Anschließend wird mit 1 ml Methanol versetzt, auf Wasser gegeben, mit Essigsäureethylester extrahiert, die organische Phase über Magnesiumsulfat getrocknet, vom Trockenmittel abfiltriert und im Vakuum aufltonzentriert. Säulenchromatographie (Cyclohexan/Essigsäureethylester 3 : 1) liefert 100,0 mg (9,7 % der Theorie) an N-[2-(Isopropoxymethyl)phenyl]-1-methyl-3-(trifluormethyl)-1H pyrazol-4-carboxamid [logP (pH 2,3) = 2,85].

Analog Beispiel 1 und 2, sowie entsprechend den Angaben in der allgemeinen Beschreibung der erfindungsgemäßen Herstellverfahren (a) bis (m) wurden auch die in der nachstehenden Tabelle 1 genannten Verbindungen der Formel (I) erhalten:

**Tabelle 1**

| | | | | | |
|---|---|---|---|---|---|
| 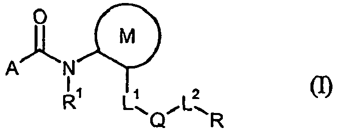 | | | | | |

| **Nr.** | **R¹** | **M** | **-L¹QL²R** | **A** | **logP (pH 2,3) Fp. (°C)** |
|---|---|---|---|---|---|
| 1 | H | 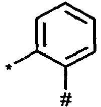 | -CH₂-O₂H₃ | 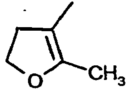 | 2,18 |
| 2 | H | 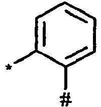 | -CH(CH₃)-SO₂-CH(CH₃)₂ | 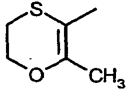 | 2,07 |
| 3 | H | 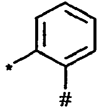 | -CH(CH₃)-SO₂-CH(CH₃)₂ | 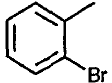 | 2,30 |
| 4 | H | 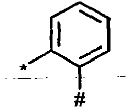 | -CH(CH₃)-SO₂-CH(CH₃)₂ | 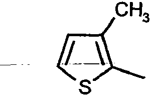 | 2,38 |
| 5 | H | 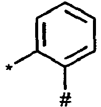 | -CH(CH₃)-SO₂-CH(CH₃)₂ | 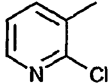 | 1,68 |
| 6 | H | 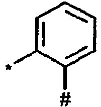 | -CH(CH₃)-SO₂-CH(CH₃)₂ | 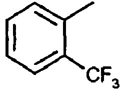 | 2,53 |
| 7 | H | 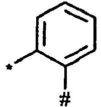 | -CH(CH₃)-SO₂-CH(CH₃)₂ | 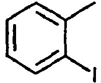 | 2,43 |
| 8 | H | 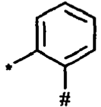 | -CH(CH₃)-SO₂-CH(CH₃)₂ | 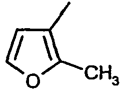 | 2,21 |
| 9 | H | 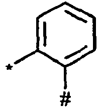 | -CH(CH₃)-SO₂-CH(CH₃)₂ | 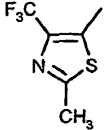 | 2,35 |
| 10 | H | 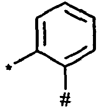 | -CH(CH₃)-SO₂-CH(CH₃)₂ | 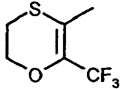 | 2,39 |
| 11 | H | 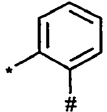 | -CH(CH₃)-SO₂-CH(CH₃)₂ | 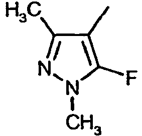 | 1,66 |
| 12 | H | 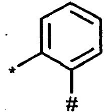 | -CH(CH₃)-SO₂-CH(CH₃)₂ | 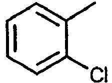 | 2,23 |
| 13 | H | 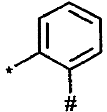 | -CH(CH₃)-SO₂-CH(CH₃)₂ | 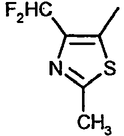 | 2,16 |
| 14 | H | 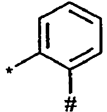 | -CH(CH₃)-SO₂-CH(CH₃)₂ | 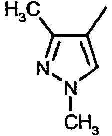 | 1,46 |
| 15 | H | 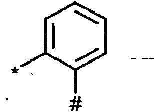 | -CH(CH₃)-SO₂-CH(CH₃)₂ | 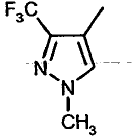 | 2,09 |
| 16 | H | 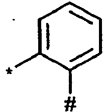 | -CH(CH₃)-SO₂-CH(CH₃)₂ | 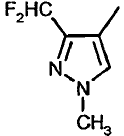 | 1,86 |
| 17 | H | 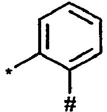 | -CH(CH₃)-S-CH(CH₃)₂ | 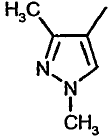 | 2,53 |
| 18 | H | 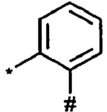 | -CH(CH₃)-S-CH(CH₃)₂ | 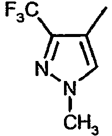 | 3,27 |
| 19 | H | 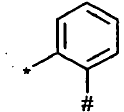 | -CH(CH₃)-S-CH(CH₃)₂ | 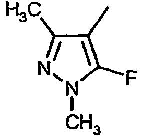 | 2,98 |
| 20 | H | 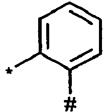 | -CH(CH₃)-S-CH(CH₃)₂ | 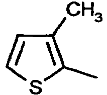 | 4,03 |
| 21 | H | 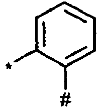 | -CH(CH₃)-S-CH(CH₃)₂ | 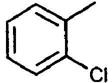 | 3,74 |
| 22 | H | 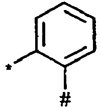 | -CH(CH₃)-S-CH(CH₃)₂ | 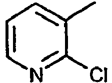 | 2,95 |
| 23 | H | 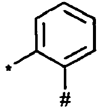 | -CH(CH₃)-S-CH(CH₃)₂ | 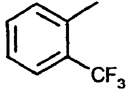 | 3,90 |
| 24 | H | 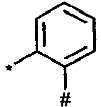 | -CH(CH₃)-S-CH(CH₃)₂ | 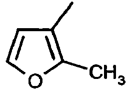 | 3,71 |
| 25 | H | 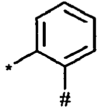 | -CH(CH₃)-S-CH(CH₃)₂ | 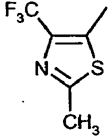 | 3,73 |
| 26 | H | 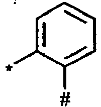 | -CH(CH₃)-S-CH(CH₃)₂ | 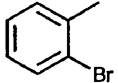 | 3,79 |
| 27 | H | 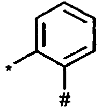 | -CH(CH₃)-S-CH(CH₃)₂ | 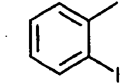 | 3,91 |
| 28 | H | 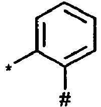 | -CH(CH₃)-S-CH(CH₃)₂ | 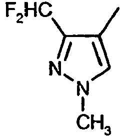 | 3,01 |
| 29 | H | 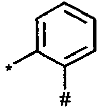 | -CH(CH₃)-S-CH(CH₃)₂ | 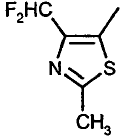 | 3,55 |
| 30 | H | 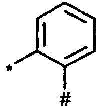 | -CH(CH₃)-S-CH(CH₃)₂ | 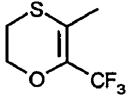 | 3,76 |
| 31 | H | 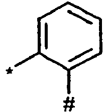 | -CH(CH₃)-O- CH₃ | 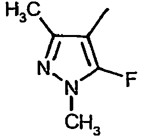 | 2,17 |
| 32 | H | 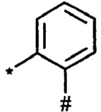 | -CH(CH₃)-O-CH₃ | 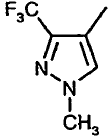 | 2,46 |
| 33 | H | 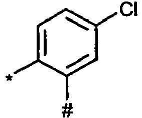 | -CH(CH₃)-O-CH₃ | 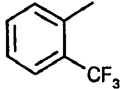 | 3,56 |
| 34 | H | 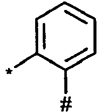 | -CH(CH₃)-O-CH₃ | 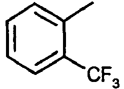 | 2,99 |
| 35 | H | 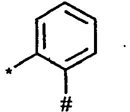 | -CH₂-S-CH(CH₃)₂ | 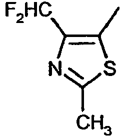 | 3,27 |
| 36 | H | 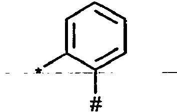 | CH₂-S-CH(CH₃)₂ | 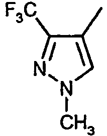 | 3,05 |
| 37 | H | 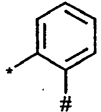 | -CH₂-S-CH(CH₃)₂ | 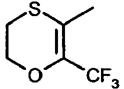 | 3,48 |
| 38 | H | 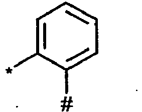 | -CH₂-S-CH(CH₃)₂ | 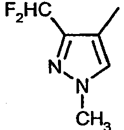 | 2,76 |
| 39 | H | 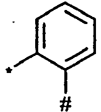 | -CH₂-S-CH(CH₃)₂ | 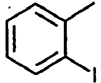 | 3,68 |
| 40 | H | 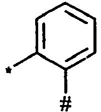 | -CH(CH₃)-NH-CH(CH₃)₂ | 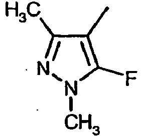 | 0,58 |
| 41 | H | 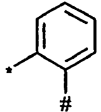 | -CH(CH₃)-NH-CH(CH₃)₂ | 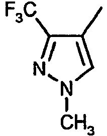 | 1,06 |
| 42 | H | 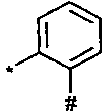 | -CH(CH₃)-S-CH(CH₃)(CH₂)₂CH₃ | 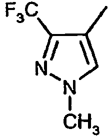 | 4,02 |
| 43 | H | 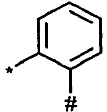 | -CH(CH₃)-S-CH(CH₃)(CH₂)₂CH₃ | 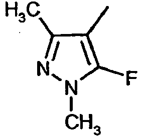 | 3,82 |
| 44 | H | 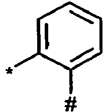 | -CH(CH₃)-S-CH(CH₃)(CH₂)₂CH₃ | 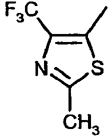 | 4,52 |
| 45 | H | 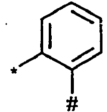 | -CH(CH₃)-S-CH(CH₃)CH₂CH₃ | 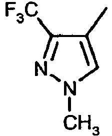 | 4,14 |
| 46 | H | 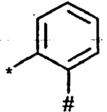 | -CH(CH₃)-S-CH(CH₃)CH₂CH₃ | 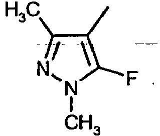 | 3,38 |
| 47 | H | 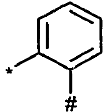 | -CH(CH₃)-S-CH(CH₃)(CH₂)₂CH₃ | 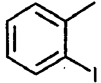 | 4,75 |
| 48 | H | 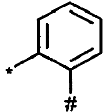 | -CH(CH₃)-S-CH(CH₃)CH₂CH₃ | 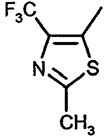 | 4,12 |
| 49 | H | 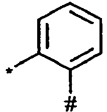 | -CH(CH₃)-S-CH(CH₃)CH₂CH₃ | 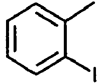 | 4,33 |
| 50 | H | 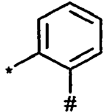 | -CH₂-O-C(O)-CHCl₂ | 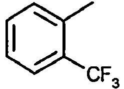 | 3,15 |
| 51 | H | 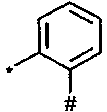 | -CH₂-O-C(O)-CH(CH₃)₂ | 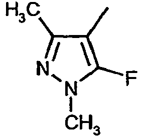 | 2,39 |
| 52 | H | 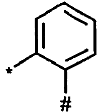 | -CH₂-O-C(O)-CHCl₂ | 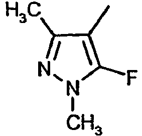 | 2,36 |
| 53 | H | 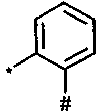 | -CH₂-O-C(O)-CH(CH₃)₂ | 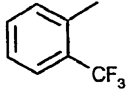 | 3,24 |
| 54 | H | 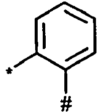 | -CH₂-O-CH(CH₃)₂ | 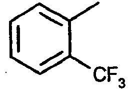 | 2,51 |
| 55 | H | 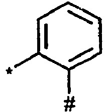 | -CH₂-O-C(O)-CH(CH₃)₂ | 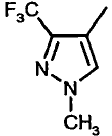 | 2,74 |
| 56 | H | 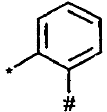 | -CH₂-O-C(O)-CHCl₂ | 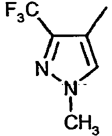 | 2,69 |
| 57 | H | 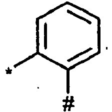 | -CH₂-O-C(O)-CH(CH₃)₂ | 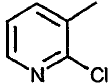 | 2,38 |
| 58 | H | 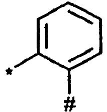 | -CH₂-O-C(O)-CHCl₂ | 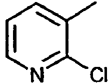 | 2,38 |
| 59 | C(O)i-Pr | 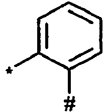 | -CH₂-O-C(O)-CH(CH₃)₂ | 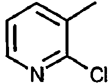 | 3,49 |
| 60 | H | 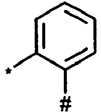 | -CH₂-O-CH(CH₃)₂ | 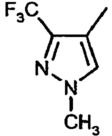 | 2,85 |
| 61 | H | 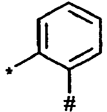 | -(CH₂)₂C(CH₃)₂-O-C(O)-CH₃ | 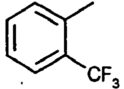 | 3,30 |
| | | | | | |
| | | | | | |
| 64 | H | 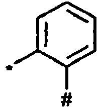 | -(CH₂)₂-O-C(O)-CH(CH₃)₂ | 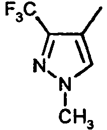 | 2,79 |
| 65 | H | 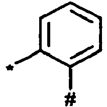 | -(CH₂)₂-O-Si(CH₃)₂-C(CH₃)₃ | 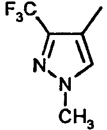 | 4,59 |
| 66 | H | 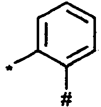 | -(CH₂)₂-O-C(O)-CHCl₂ | 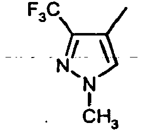 | 2,76 |
| 67 | H | 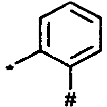 | -(CH₂)₂O-C(O)-CH(CH₃)₂ | 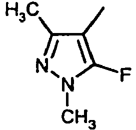 | 2,40 |
| 68 | H | 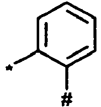 | -(CH₂)₂-O-C(O)-CHCl₂ | 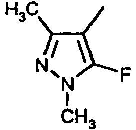 | 2,43 |
| 69 | H | 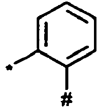 | -(CH₂)₂-O-Si(CH₃)₂-C(CH₃)₃ | 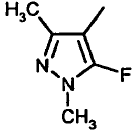 | 4,49 |
| 70 | H | 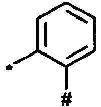 | -CH(CH₃)-S-CH(CH₃)(CH₂)₂CH₃ | 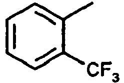 | 4,71 |
| 71 | H | 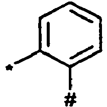 | -CH(CH₃)-S-CH(CH₃)(CH₂)₂CH₃ | 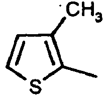 | 4,93 |
| 72 | H | 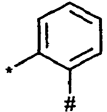 | -CH(CH₃)-S-CH(CH₃)(CH₂)₂CH₃ | 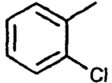 | 4,60 |
| 73 | H | 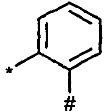 | -CH(CH₃)-S-CH(CH₃)CH₂CH₃ | 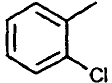 | 4,17 |
| 74 | H | 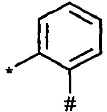 | -CH(CH₃)-S-CH(CH₃)CH₂CH₃ | 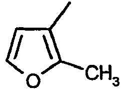 | 4,17 |
| 75 | H | 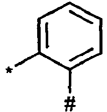 | -CH(CH₃)-S-CH(CH₃)CH₂CH₃ | 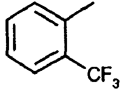 | 4,31 |
| 76 | H | 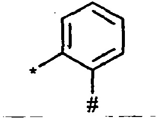 | -CH(CH₃)-S-CH(CH₃)CH₂CH₃ | 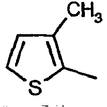 | 4,47 |
| 77 | H | 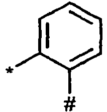 | -CH(CH₃)-S-CH(CH₃)(CH_{zh}CH₃ | 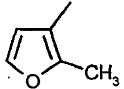 | 4,61 |
| 78 | H | 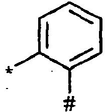 | -CH(CH₃)-S-CH(CH₃)CH₂CH₃ | 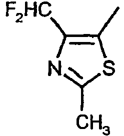 | 3,95 |
| 79 | H | 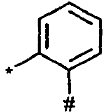 | -CH(CH₃)-S-CH(CH₃)(CH₂)₂CH₃ | 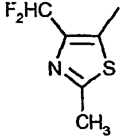 | 4,36 |
| 80 | H | 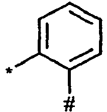 | -CH(CH₃)-S-CH(CH₃)CH₂CH₃ | 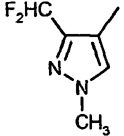 | 3,38 |
| 81 | H | 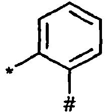 | -CH(CH₃)-S-CH(CH₃)(CH₂)₂CH₃ | 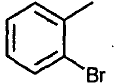 | 4,63 |
| 82 | H | 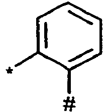 | -CH(CH₃)-S-CH(CH₃)(CH₂)₂CH₃ | 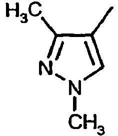 | 3,27 |
| 83 | H | 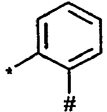 | -CH(CH₃)-S-CH(CH₃)CH₂CH₃ | 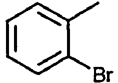 | 4,21 |
| 84 | H | 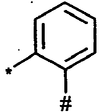 | -CH(CH₃)-S-CH(CH₃)CH₂CH₃ | 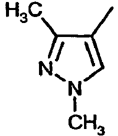 | 2,90 |
| 85 | H | 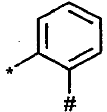 | -CH(CH₃)-S-CH(CH₃)(CH₂)₂CH₃ | 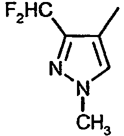 | 3,76 |
| 86 | H | 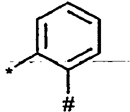 | CH(CH₃)-S-CH(CH₃)CH₂CH₃ | 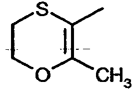 | 4,07 |
| 87 | H | 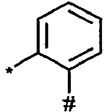 | -CH(CH₃)-S-CH(CH₃)(CH₂)₂CH₃ | 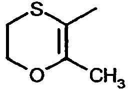 | 4,53 |
| 88 | H | 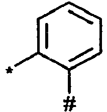 | -CH(CH₃)-S-CH(CH₃)CH₂CH₃ | 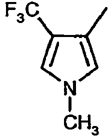 | 3,92 |
| 89 | H | 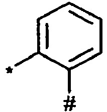 | -CH(CH₃)-S-CH(CH₃)CH₂CH₃ | 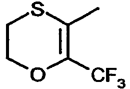 | 4,10 |
| 90 | H | 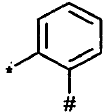 | 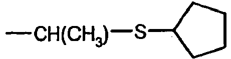 | 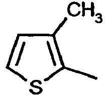 | 3,16 |
| 91 | H | 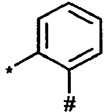 | 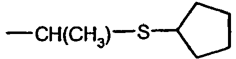 | 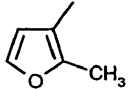 | 4,31 |
| 92 | H | 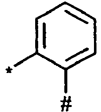 | 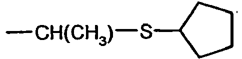 | 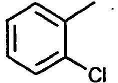 | 4,29 |
| 93 | H | 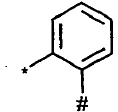 | 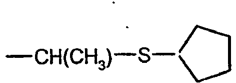 | 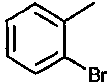 | 4,34 |
| 94 | H | 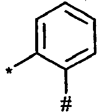 | 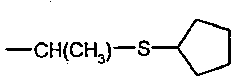 | 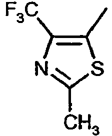 | 4,24 |
| 95 | H | 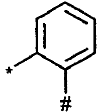 | 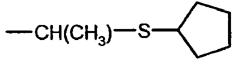 | 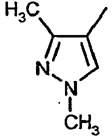 | 3,04 |
| 96 | H | 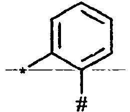 | 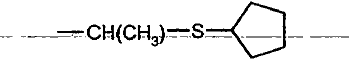 | 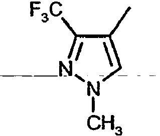 | 3,76 |
| 97 | H | 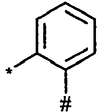 | 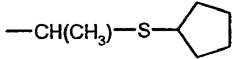 | 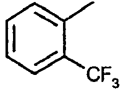 | 4,41 |
| 98 | H | 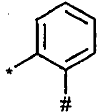 | -CH(CH₃)-S-CH(CH₃)₂ | 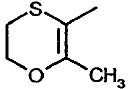 | 3,65 |
| 99 | H | 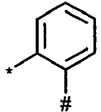 | 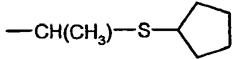 | 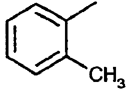 | 4,44 |
| 100 | H | 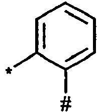 | 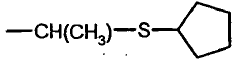 | 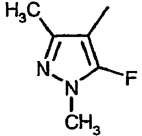 | 4,03 |
| 101 | H | 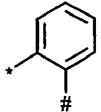 | 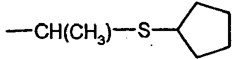 | 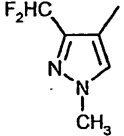 | 3,48 |
| 102 | H | 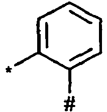 | 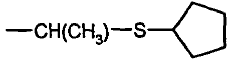 | 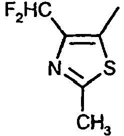 | 4,07 |
| 103 | H | 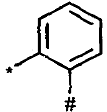 | -CH(CH₃)-S-CH(CH₃)₂ | 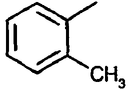 | 3,88 |
| 104 | H | 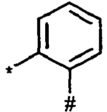 | CH(CH₃)-S-CH(CH₃)₂ | 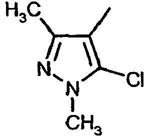 | 3,20 |
| 105 | H | 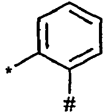 | -CH(CH₃)-S-CH(CH₃)₂ | 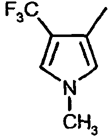 | 3,57 |
| 106 | H | 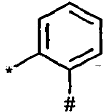 | -CH(CH₃)-S-CH(CH₃)₂ | 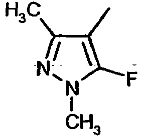 | 3,63 |
| 107 | H | 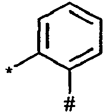 | 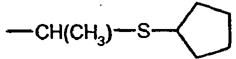 | 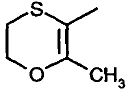 | 4,24 |
| 108 | H | 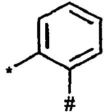 | 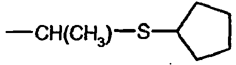 | 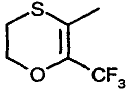 | 4,21 |
| 109 | H | 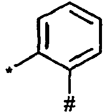 | -CH(CH₃)-S-CH(CH3)(CH₂)₂CH₃ | 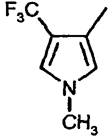 | 4,29 |
| 110 | H | 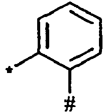 | -CH(CH₃)-S-CH(CH₃)(CH₂)₂CH₃ | 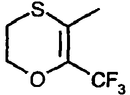 | 4,49 |
| 111 | H | 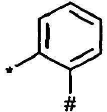 | -CH(CH₃)-S-CH(CH₃)(CH₂)₂CH₃ | 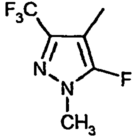 | 4,38 |
| 112 | H | 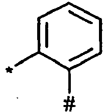 | -CH(CH₃)-S-CH(CH₃)(CH₂)₂CH₃ | 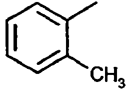 | 4,76 |
| 113 | H | 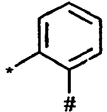 | -CH(CH₃)-S-CH(CH₃)(CH₂)₂CH₃ | 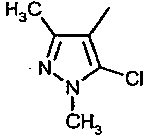 | 4,07 |
| 114 | H | 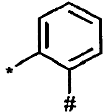 | -CH(CH₃)-S-CH(CH₃)(CH₂)₂CH₃ | 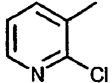 | 3,79 |
| 115 | H | 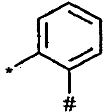 | 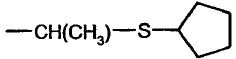 | 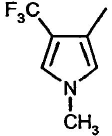 | 4,02 |
| 116 | H | 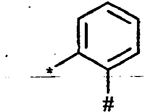 | 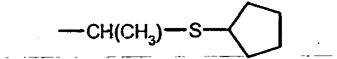 | 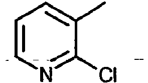 | 3,46 |
| 117 | H | 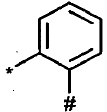 | 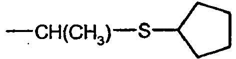 | 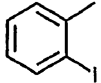 | 4,45 |
| 118 | H | 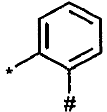 | 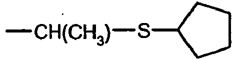 | 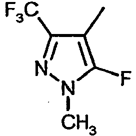 | 4,12 |
| 119 | H | 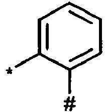 | 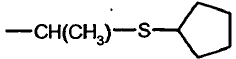 | 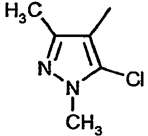 | 3,76 |
| 120 | H | 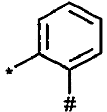 | -CH(CH₃)-S-CH(CH₃)CH₂CH₃ | 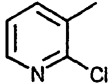 | 3,35 |
| 121 | H | 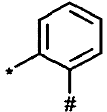 | -CH(CH₃)-S-CH(CH₃)CH₂CH₃ | 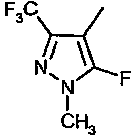 | 3,99 |
| 122 | H | 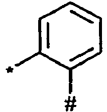 | -CH(CH₃)-S-CH(CH₃)CH₂CH₃ | 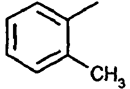 | 4,31 |
| 123 | H | 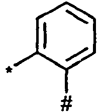 | -CH(CH₃)-S-CH(CH₃)CH₂CH₃ | 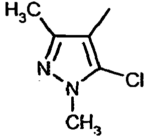 | 3,60 |
| 124 | H | 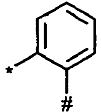 | -CH(CH₃)-O-CH(CH₃)₂ | 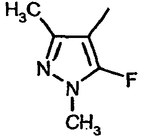 | 2,90 |
| 125 | H | 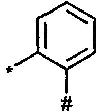 | -CH(CH₃)-O-CH(CH₃)₂ | 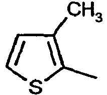 | 4,50 |
| 126 | H | 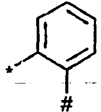 | -CH(CH₃)-O-CH(CH₃)₂ | 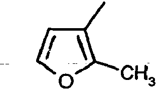 | 3,80 |
| 127 | H | 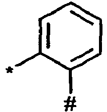 | -CH(CH₃)-O-CH(CH₃)₂ | 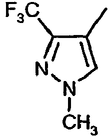 | 3,14 |
| 128 | H | 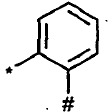 | -CH(CH₃)-O-CH(CH₃)₂ | 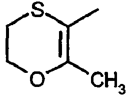 | 3,47 |
| 129 | H | 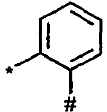 | -CH(CH₃)-O-CH(CH₃)₂ | 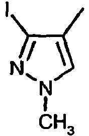 | 2,75 |
| 130 | H | 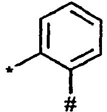 | -CH(CH₃)-O-CH(CH₃)₂ | 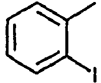 | 3,83 |
| 131 | H | 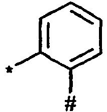 | -CH(CH₃)-O-CH(CH₃)₂ | 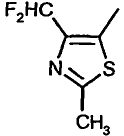 | 3,58 |
| 132 | H | 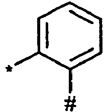 | -CH(CH₃)-O-CH(CH₃)₂ | 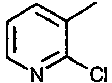 | 2,72 |
| 133 | H | 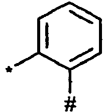 | -CH(CH₃)-O-CH(CH₃)₂ | 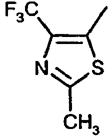 | 3,65 |
| 134 | H | 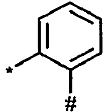 | -CH(CH₃)-O-CH(CH₃)₂ | 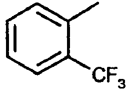 | 3,81 |
| 135 | H | 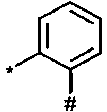 | -CH(CH₃)-O-CH(CH₃)₂ | 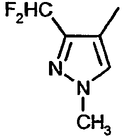 | 2,89 |
| 136 | H | 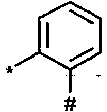 | -CH(CH₃)-O-CH(CH₃)₂ | 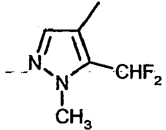 | 3,67 |
| 137 | H | 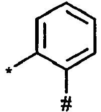 | -CH(CH₃)-O-CH(CH₃)₂ | 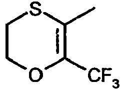 | 3,64 |
| 138 | H | 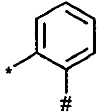 | -CH(CH₃)-O-CH(CH₃)₂ | 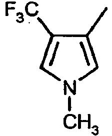 | 5,60 |
| 139 | H | 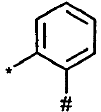 | -CH₂-N(CH₃)₂ | 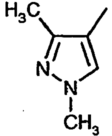 | 98°C |
| 140 | H | 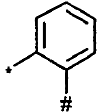 | -CH₂-N(CH₃)₂ | 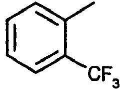 | |
| 141 | H | 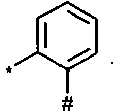 | -CH₂-S-CH₂CH₃ | 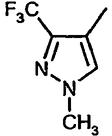 | |
| 142 | H | 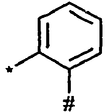 | -CH₂-S-CH₂CH₃) | 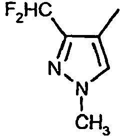 | 103°C |
| 143 | H | 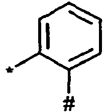 | -CH₂-S-CH₂CH₃ | 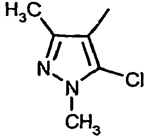 | 96°C |
| 144 | H | 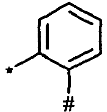 | -CH₂-S-CH₂CH₃ | 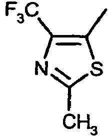 | |
| 145 | H | 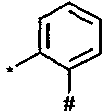 | -CH₂-SO-CH₂CH₃ | 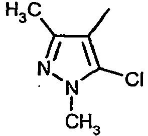 | |
| 146 | H | 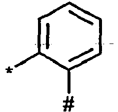 | -CH₂-S-CH₂CH₃ | 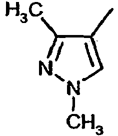 | 105°C |
| 147 | H | 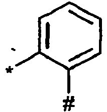 | -CH₂-SO-CH₂CH₃ | 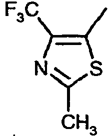 | |
| 148 | H | 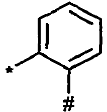 | -CH₂-SO-CH₂CH₃ | 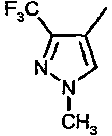 | |
| 149 | H | 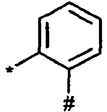 | -CH₂-SO-CH₂CH₃ | 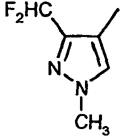 | |
| 150 | H | 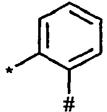 | -CH_{z}-SO₂-CH₂CH₃ | 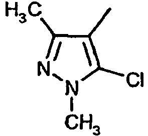 | 148°C |
| 151 | H | 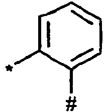 | -CH₂-SO₂-CH₂CH₃ | 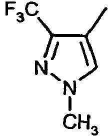 | 135°C |
| 152 | H | 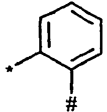 | -CH₂-SO₂-CH₂CH₃ | 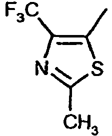 | 142°C |
| 153 | H | 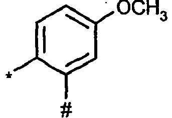 | -CH₂-S-CH₂CH₃ | 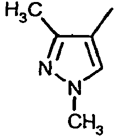 | 142°C |
| 154 | H | 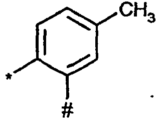 | -CH₂-S-CH₂CH₃ | 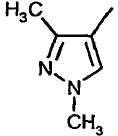 | 116°C |
| 155 | H | 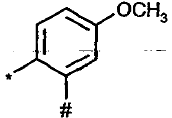 | -CH₂-S-CH₂CH₃ | 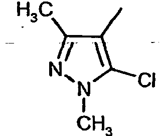 | 125°C |
| 156 | H | 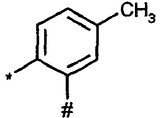 | -CH₂S-CH₂CH₃ | 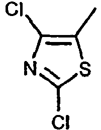 | 72°C |
| 157 | H | 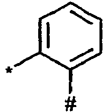 | -CH₂-SO₂-CH₂CH₃ | 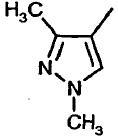 | |
| 158 | H | 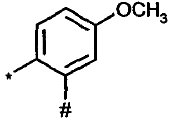 | -CH₂-S-CH₂CH₃ | 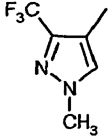 | 135°C |
| 159 | H | 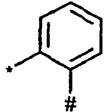 | -CH₂-SO₂-CH₂CH₃ | 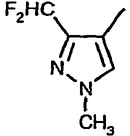 | 143°C |
| 160 | H | 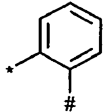 | -CH₂-SO-CH₂CH₃ | 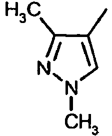 | |
| 161 | H | 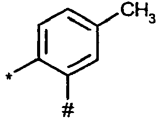 | -CH₂-S-CH₂CH₃ | 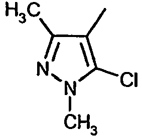 | 127°C |
| 162 | H | 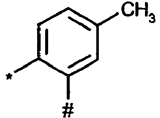 | -CH₂-S-CH₂CH₃ | 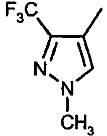 | |
| 163 | H | 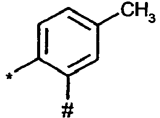 | -CH₂-S-CH₂CH₃ | 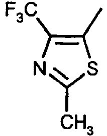 | |
| 164 | H | 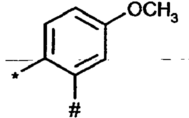 | -CH₂-S-CH₂CH₃ | 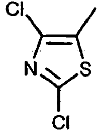 | 116°C |
| 165 | H | 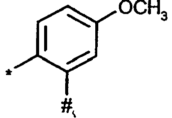 | -CH₂-S-CH₂CH₃ | 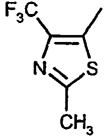 | 98°C |
| 166 | H | 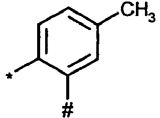 | -CH₂-S-CH₂CH₃ | 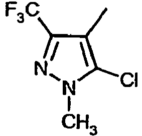 | 132°C |
| 167 | H | 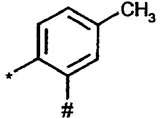 | -CH₂-S-CH₂CH₃ | 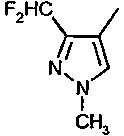 | 86°C |
| 168 | H | 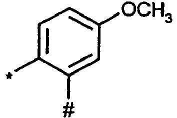 | -CH₂-S-CH₂CH₃ | 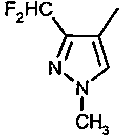 | 110°C |
| 169 | H | 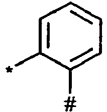 | -CH₂-S-CH₂CH₃ | 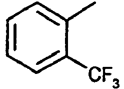 | 79°C |
| 170 | H | 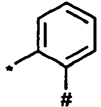 | -CH₂-S-CH₂CH₃ | 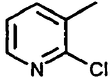 | 75°C |
| 171 | H | 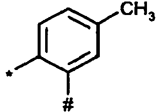 | -CH₂-S-CH₂CH₃ | 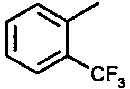 | 105°C |
| 172 | H | 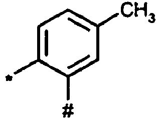 | -CH₂-S-CH₂CH₃ | 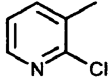 | 112°C |
| 173 | H | 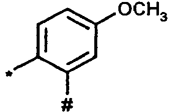 | -CH₂-S-CH₂CH₃ | 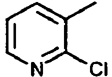 | 138°C |
| 174 | H | 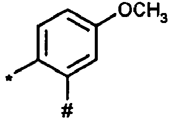 | -CH₂-S-CH₂CH₃ | 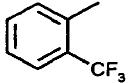 | 120°C |
| 175 | H | 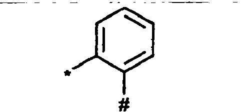 | -CH₂-S-CH₂CH₃ | 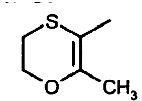 | 71°C |
| 176 | H | 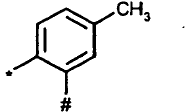 | -CH₂-S-CH₂CH₃ | 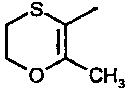 | |
| 177 | H | 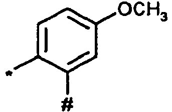 | -CH₂S-CH₂CH₃ | 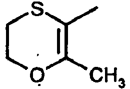 | 68°C |
| | | | | | |
| 179 | H | 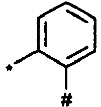 | -CH₂-S-CH₂CH(CH₃)₂ | 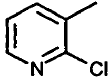 | 92°C |
| 180 | H | 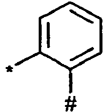 | -CH₂-S-CH₂CH(CH₃)₂ | 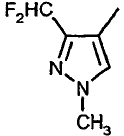 | |
| 181 | H | 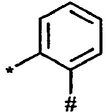 | -CH₂-S-CH₂CH(CH₃)₂ | 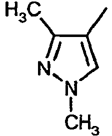 | 86°C |
| 182 | H | 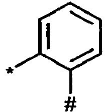 | -CH₂-S-CH₂CH(CH₃)₂ | 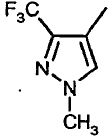 | 82°C |
| 183 | H | 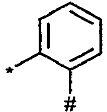 | -CH₂-S-CH₂CH(CH₃)₂ | 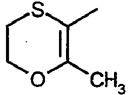 | 45°C |
| 184 | H | 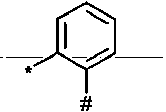 | -CH₂-S-CH₂CH(CH₃)₂ | 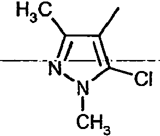 | |
| 185 | H | 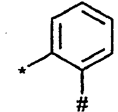 | -CH₂-S-CH₂CH(CH₃)₂ | 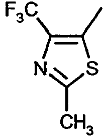 | |
| 186 | H | 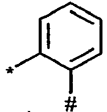 | -CH₂-S-CH₂CH(CH₃)₂ | 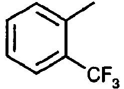 | 94°C |
| 187 | H | 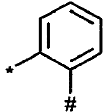 | -CH₂-S-CH₂CH(CH₃)₂ | 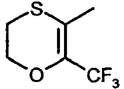 | 77°C |
| 188 | H | 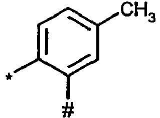 | -CH₂-S-CH₂CH₃ | 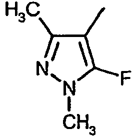 | 96°C |
| 189 | H | 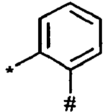 | -CH₂-S-CH₂CH(CH₃)₂ | 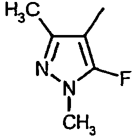 | |
| 190 | H | 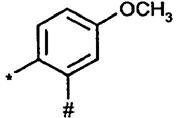 | -CH₂-S-CH₂CH₃ | 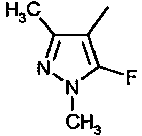 | 147°C |
| 191 | H | 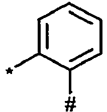 | -CH₂-S-CH₃ | 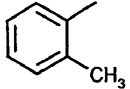 | |
| 192 | H | 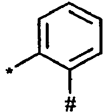 | -CH(CH₃)-S-CH₂CH₃ | 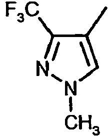 | |
| 193 | H | 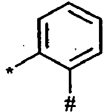 | -CH(CH₃)-S-CH₂CH₃ | 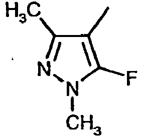 | |
| 194 | H | 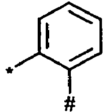 | -CH(CH₃)-S-CH₂CH₃ | 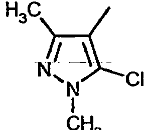 | |
| 195 | H | 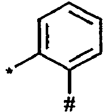 | -CH(CH₃)-S-CH₂CH₃ | 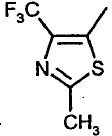 | |
| 196 | H | 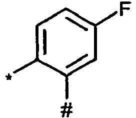 | -CH(CH₃)-S-CH₂CH₃ | 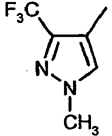 | |
| 197 | H | 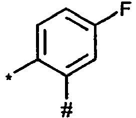 | -CH(CH₃)-S-CH₂CH₃ | 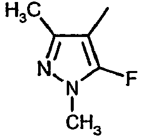 | |
| 198 | H | 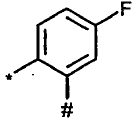 | -CH(CH₃)-S-CH₂CH₃ | 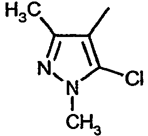 | |
| ^{a}) Die mit "*" markierte Bindung ist mit dem Amid verknüpft. | | | | | |

### Herstellung der Ausgangsstoffe der Formel (III)

### Beispiel (III-1)

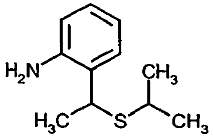

44 g (0,188:mol) 1-[1-(Isopropylthio)ethyl]-2-nitrobenzol (VII-1) werden in 250 ml Ethanol gelöst, mit 3 g Raney-Nickel versetzt und im Autoklav bei Raumtemperatur 6 Stunden mit 3 bar Wasserstoff hydriert. Nach 6 Stunden werden erneut 3 g Raney-Nickel hinzugegeben und weitere 16 Stunden bei Raumtemperatur mit 3 bar Wasserstoff hydriert. Zur Aufarbeitung wird vom Katalysator abfiltriert und das Lösungsmittel in Vakuum entfernt. Das Rohprodukt wird säulenchromatographisch gereinigt (Kieselgel, Hexan/Methyl-tert-butylether 3:1). Man erhält 32 g (Gehalt 97,3 %, HPLC, 84,4 % der Theorie) an 2-[1-(Isopropylthio)ethyl]anilin in Form eines gelben Öls [logP (pH 2,3) = 2,45].

### Beispiel (III-2)

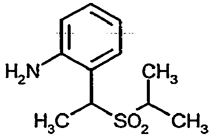

In einem 500 ml Dreihalskolben mit Rührer und Thermometer werden 16,2 g (60,5 mmol) 1-[1-(Iso-propylsulfonyl)ethyl]-2-nitrobenzol (VII-4) in 160 ml Methanol vorgelegt, unter Rühren mit 160 ml konzentrierter Salzsäure versetzt und bei 20-40°C portionsweise 31,5 g Zinnpulver (265,2 mmol) zugegeben. Die Mischung wird bei 40°C ca. 1 Stunde nachgerührt. Zur Aufarbeitung wird abgekühlt, filtriert und mit 1575 ml einer eisgekühlten 10%igen Natronlauge verrührt. Man extrahiert zweimal mit Dichlormethan, trocknet über Natriumsulfat und zieht das Lösungsmittel im Vakuum ab. Man erhält 13,3 g (Gehalt 95,8 %, HPLC, 92,6 % der Theorie) an 2-[1-(Isopropylsulfonyl)ethyl]anilin [logP (pH 2,3) = 1,28].

### Beispiel (III-3)

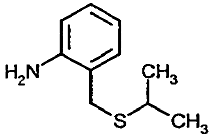

In einem 500 ml Dreihalskolben mit Rührer und Thermometer werden 11,8 g (55,6 mmol) 1-[1-(Iso-propylthio)methyl]-2-nitrobenzol (VII-2) in 150 ml Methanol vorgelegt, unter Rühren mit 150 ml konzentrierter Salzsäure versetzt und bei 20-40°C portionsweise 17,6 g Zinnpulver (148,5 mmol) zugegeben. Die Mischung wird bei 40°C für ca. 1 Stunde nachgerührt Zur Aufarbeitung wird abgekühlt, filtriert und mit 1300 ml einer eisgekühlten 10%igen Natronlauge verrührt. Man extrahiert zweimal mit Dichlormethan, trocknet über Natriumsulfat und verdampft das Lösungsmittel im Vakuum. Das Rohprodukt wird mit Hexan/Methyl-tert-butylether 3:1 an Kieselgel gereinigt Man erhält 4,6 g (Gehalt 94,6 %, HPLC, 43,2 % der Theorie) an 2-[(Isopropylthio)methyl]anilin in Form eines gelben Öls [logP (pH 2,3) =1,94].

### Beispiel (III-4)

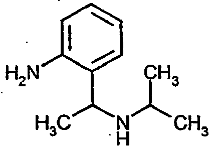

In einem Autoklav wird eine Lösung von 5 g N-[1-(2-Nitrophenyl)ethyl]propan-2-amin (VII-5) (24 mmol) in 30 ml Methanol mit 0,5 g Raney-Nickel versetzt und 5 Stunden bei 50°C und 50 bar Wasserstoff hydriert Zur Aufarbeitung wird abgekühlt, der Katalysator abfiltriert und das Lösungsmittel im Vakuum entfernt. Man erhält 2,8 g (Gehalt 98,1 %, 64,2 % der Theorie) an 2-[1-(Isopropyl-amino)ethyl]anilin [logP (pH 2,3) = 0,05].

### Herstellung der Ausgangsstoffe der Formel (IV)

### Beispiel (IV-1)

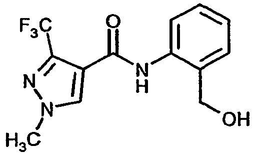

Bei Raumtemperatur wird eine Lösung bestehend aus 34,5 g (0,16 Mol) 1-Methyl-3-trifluoromethyl-1H-pyrazol-4-carbonsäurechlorid in 50 ml Tetrahydrofuran zu einer Lösung bestehend aus 20,0 g (0,16 Mol) (2-Amino-phenyl)-methanol und 36 ml (0,26 Mol) Triethylamin in 250 ml Tetrahydrofuran getropft. Nach Abklingen der exothermen Wärmetönung wird für 6 Stunden rückflussiert und weitere 48 Stunden bei Raumtemperatur gerührt. Die Reaktionsmischung wird auf ca. 250 ml Wasser gegeben, mit Essigsäureethylester extrahiert, mit 2 N Salzsäure gewaschen, über Magnesiumsulfat getrocknet und im Vakuum aufkonzentriert. Säulenchromatographie (Gradient Cyclohexan/Essigsäureethylester) liefert 33,8 g (69 % der Theorie) an N-[2-(Hydroxymethyl)phenyl]-1-methyl-3-(trifluormethyl)-1H-pyrazol-4-carboxamid [logP (pH 2,3) =1,55].

### Herstellung der Ausgangsstoffe der Formel (VII)

### Beispiel (VII-1)

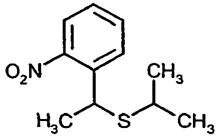

In einem 1 Liter-Dreihalskolben mit Rührer, Tropftrichter und Thermometer werden 34,8 g (0,354 mol) Natrium-2-propanthiolat in 450 ml Acetonitril vorgelegt und bei einer Temperatur von 30-40°C 63 g 1-(1-Chlorethyl)-2-nitrobenzol (VIII-1) (Gehalt 99,4 %, 0,337 mol) in 20 ml Acetonitril gelöst zugetropft. Die Suspension wird noch 16 Stunden bei 40°C nachgerührt, abgekühlt und das Lösungsmittel im Vakuum abgezogen. Der verbleibende Rückstand wird in Dichlormethan aufgenommen, gewaschen, über Natriumsulfat getrocknet und das Lösungsmittel im Vakuum entfernt. Das Rohprodukt wird säulenchromatographisch gereinigt (Kieselgel, Hexan/Aceton 29:1). Man erhält 48 g (Gehalt 98,8 %, HPLC, 62,4 % der Theorie) an 1-[1-(Isopropylthio)ethyl]-2-nitrobenzol in Form eines gelben Öls [logP (pH 2,3) = 3,89].

### Beispiel (VII-2)

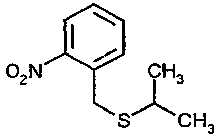

In einem 250 ml Dreihalskolben mit Rührer, Tropftrichter und Thermometer werden 10,3 g (0,105 mol) Natrium-2-propanthiolat in 75 ml Acetonitril vorgelegt und unter Kühlung bei 30-40°C eine Lösung von 17,2 g (0,1 mol) 2-Nitrobenzylchlorid in 20 ml Acetonitril zugetropft. Die Suspension wird 16 Stunden bei 40-50 °C nachgerührt. Zur Vervollständigung der Umsetzung werden noch 6 g Natrium-2-propanthiolat (0,061 mol) zugesetzt und weitere 24 Stunden bei 40-50°C gerührt. Zur Aufarbeitung wird abgekühlt und das Lösungsmittel im Vakuum entfernt Der Rückstand wird in Methyl-tert-butylether aufgenommen, gewaschen und über Natriumsulfat getrocknet Das Natriumsulfat wird abfiltriert und die Lösung im Vakuum eingeengt. Das Rohprodukt wird säulenchromatographisch gereinigt (Kieselgel, Cyclohexan/Essigsäureethylester 50:1). Man erhält 11,8 g (Gehalt 92 %, HPLC, 51,2 % der Theorie) an 1-[1-(Isopropylthio)methyl]-2-nitrobenzol in Form eines braunen Öls [logP (pH 2,3) = 3,28].

### Beispiel (VII-3)

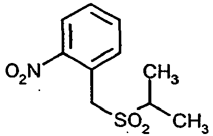

In einem 1 Liter-Dreihalskolben mit Rührer, Tropftrichter und Thermometer werden 32,8 g 1-[1-(Iso-propylthio)methyl]-2-nitrobenzol (VII-2) (0,155 mol) in 465 ml Dichlormethan vorgelegt und unter Rühren nacheinander 14,3 g Ameisensäure (0,31 mol) und 1,6 g Ammoniummolybdat zugegeben. Unter schnellem Rühren werden bei Raumtemperatur 45,3 g (0,466 mol) 35%ige wässrige Wasserstoffperoxidlösung zugetropft. Die Mischung wird 16 Stunden nachgerührt. Zur Aufarbeitung wird die organische Phase abgetrennt, jeweils einmal mit verdünnter Natriumhydrogensulfit-Lösung und mit Wasser gewaschen und die organische Lösung über Natriumsulfat getrocknet. Das Lösungsmittel wird im Vakuum abdestilliert und der Rückstand mit Diethylether verrührt, das ausgefallene Produkt abgesaugt und getrocknet Man erhält 30,5 g (Gehalt 99 %, HPLC, 80 % der Theorie) an 1-[(Isopro-pylsulfonyl)methyl]-2-nitrobenzol in Form eines gelben Feststoffes [logP (pH 2,3) =1,63].

### Beispiel (VII-4)

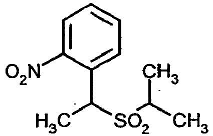

In einem 1 Liter-Dreihalskolben mit Rührer und Thermometer werden 18,9 g (77,7 mmol) 1-[(Isopro-pylsulfonyl)methyl]-2-nitrobenzol (VII-3) in 390 ml Acetonitril vorgelegt und nacheinander 90,1 g (652 mmol) Kaliumcarbonat, 0,26 g 18-Krone-6 und 12,1 g (85,5 mmol) Iodmethan zugegeben. Man rührt 4 Stunden unter Rückfluss, setzt dann noch 2,5 g Iodmethan (17,6 mmol) nach und rührt weitere 4 Stunden unter Rückfluss. Zur Aufarbeitung wird abgekühlt und im Vakuum eingeengt. Der Rückstand wird in Essigsäureethylester aufgenommen, mit Wasser gewaschen und über Natriumsulfat getrocknet Nach dem Abziehen des Lösungsmittels wird das Rohprodukt säulenchromatographisch gereinigt (Kieselgel, Hexan/Aceton 7:3 ). Man erhält 16,2 g (Gehalt 96,1 %, HPLC, 77,9 % der Theorie) an 1-[1-(Isopropylsulfonyl)ethyl]-2-nitrobenzol [logP (pH 2,3) = 1,99].

### Beispiel (VII-5)

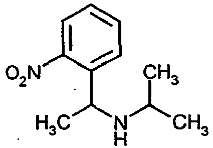

In einem Autoklav werden 13,8 g 1-(1-Chlorethyl)-2-nitrobenzol (VIII-1) (98,3%ig, 73,1 mmol) und 43,2 g Isopropylamin (731 mmol) 24 Stunden bei 60°C und Eigendruck gerührt. Nach dem Abkühlen des Reaktionsgemisches wird das überschüssige Isopropylamin im Vakuum entfernt und das Rohprodukt mit Cyclohexan/Essigsäureethylester 4:1 an Kieselgel gereinigt. Man erhält 5 g (Gehalt 94,4 %, 31 % der Theorie) an N-[1-(2-Nitrophenyl)ethyl]propan-2-amin in Form eines Öls [logP (pH 2,3) = 0,55].

### Herstellung der Ausgangsstoffe der Formel (VIII)

### Beispiel (VIII-1)

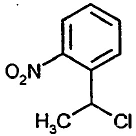

In einem 6 Liter-Dreihalskolben mit Rührer, Tropftrichter und Thermometer werden 311 g 1-(2-Ni-trophenyl)ethanol (XI-1) (Gehalt 95,8 %, 1,78 mol) in 3000 ml Dimethylformamid gelöst. Unter Rühren werden in einer Portion 921,4 g (7,13 mol) Diisopropylethylamin zugegeben, 5 Minuten gerührt und bei guter Kühlung in einem Temperaturbereich zwischen 20 und 35°C 612,5 g (5,35 mol) Methansulfonsäurechlorid zugetropft. Nach Abklingen der Reaktion wird noch 90 Stunden bei Raumtemperatur gerührt, unter Vakuum das Lösungsmittel entfernt, in Essigsäureethylester aufgenommen, 3 mal mit Wasser gewaschen, über Natriumsulfat getrocknet und das Lösungsmittel im Vakuum entfernt. Das Rohprodukt wird säulenchromatographisch gereinigt (Kieselgel, Hexan/Aceton 9:1). Man erhält 219 g (Gehalt 100 %, HPLC, 66,2 % der Theorie) an 1-(1-Chlorethyl)-2-nitrobenzol in Form eines bräunlichen Öls [logP (pH 2,3) = 2,87].

### Herstellung der Ausgangsstoffe der Formel (XI)

### Beispiel (XI-1)

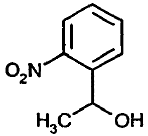

In einem 6-Liter Dreihalskolben mit Rührer, Tropftrichter, Thermometer und Blasenzähler werden 320 g (1,938 mol) 2 Nitroacetophenon in 3200 ml Methanol vorgelegt und unter schwacher Kühlung bei 30-40°C eine Lösung von 73,3 g (1,938 mol) Natriumborhydrid in 288 ml Wasser zugetropft. Nach Abklingen der Reaktion wird noch 16 Stunden bei Raumtemperatur nachgerührt. Zur Aufarbeitung wird mit verdünnter Salzsäure neutralisiert und das Lösungsmittel am Rotationsverdampfer abgezogen. Der verbliebene Rückstand wird mit Dichlormethan aufgenommen, mit Wasser gewaschen, über Natriumsulfat getrocknet und das Lösungsmittel im Vakuum entfernt. Man erhält 311 g (Gehalt 95,1 %, HPLC, 91,3 % der Theorie) von 1-(2-Nitrophenyl)ethanol [logP (pH 2,3) = 1,49] in Form eines hellen Öls.

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
Eluenten für die Bestimmung im sauren Bereich (pH 2,3): 0,1 % wässrige Phosphorsäure, Acetonitril; linearer Gradient von 10 % Acetonitril bis 90 % Acetonitril.
Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).
Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator. | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht Nach Antrocknen des Spritzbelages werden die Pflanzen mit einer wässrigen Sporensuspension des Apfelmehltauerregers Podosphaera leucotricha inokuliert Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Podosphaera-Test (Apfel)** / **protektiv** | | |
|---|---|---|
| Wirkstoff Erfindungsgemäß | Aufwandmenge an Wirkstoff in g/ha | Wirkungsgrad in % |
| 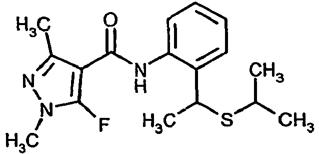 | 100 | 91 |
| 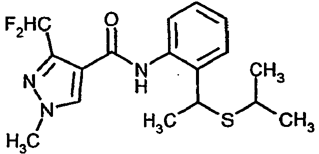 | 100 | 84 |
| 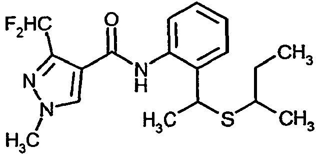 | 100 | 94 |

### Beispiel B

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung aufprotektive Wirksamkeit werden junge Pflanzen mit der Wirkstoffzubereitung in der angegebenen Aufwandmenge besprüht Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel)** / **protektiv** | | |
|---|---|---|
| Wirkstoff Erfindungsgemäß | Aufwandmenge an Wirkstoff in g/ha | Wirkungsgrad in % |
| 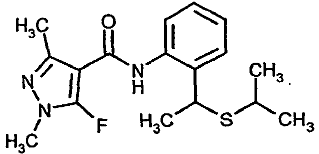 | 100 | 95 |
| 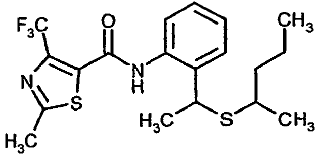 | 100 | 89 |
| 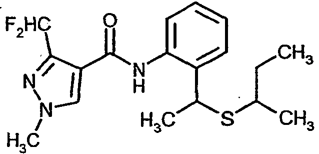 | 100 | 99 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne)** / **protektiv** | | |
|---|---|---|
| Wirkstoff Erfindungsgemäß | Aufwandmenge an Wirkstoff in g/ha | Wirkungsgrad in % |
| 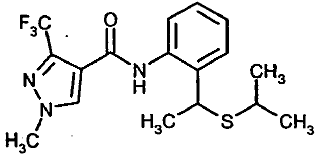 | 500 | 84 |
| 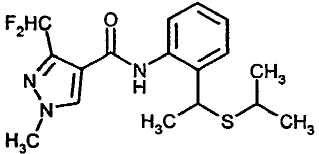 | 500 | 100 |
| 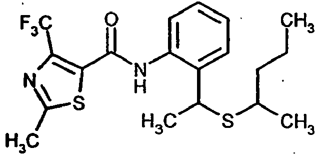 | 500 | 99 |
| 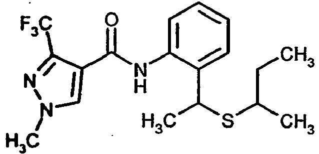 | 500 | 100 |
| 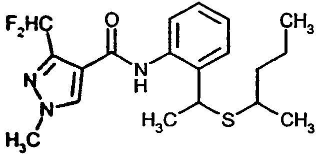 | 500 | 97 |
| 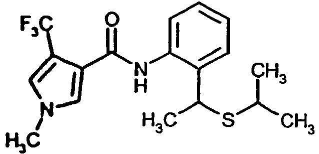 | 500 | 97 |

### Beispiel D

### Puccinia-Test (Weizen) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen)** / **protektiv** | | |
|---|---|---|
| Wirkstoff Erfindungsgemäß | Aufwandmenge an Wirkstoff in g/ha | Wirkungsgrad in % |
| 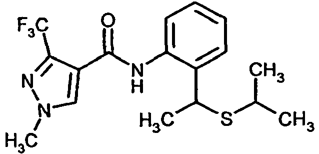 | 500 | 100 |
| 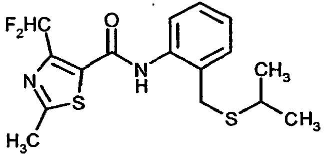 | 500 | 93 |
| 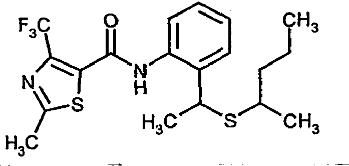 | 500 | 100 |
| 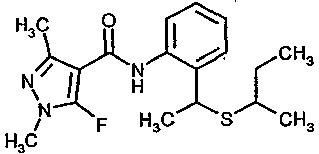 | 500 | 100 |
| 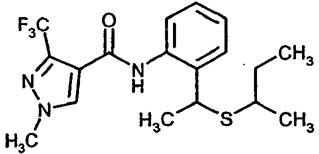 | 500 | 100 |
| 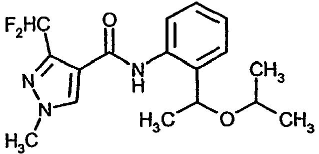 | 500 | 100 |
| 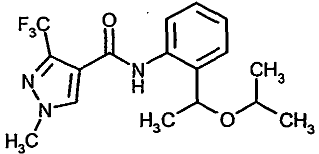 | 500 | 100 |
| 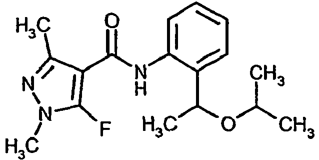 | 500 | 100 |
| 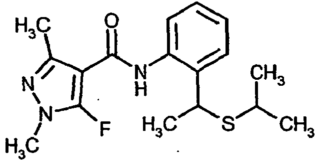 | 500 | 100 |
| 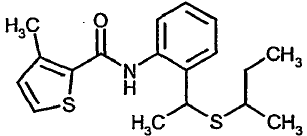 | 500 | 100 |
| 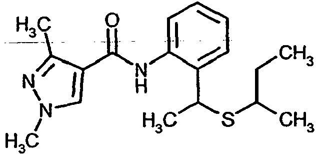 | 500 | 100 |
| 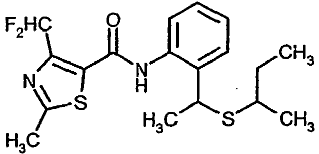 | 500 | 100 |
| 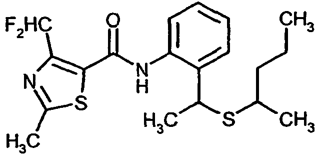 | 500 | 100 |
| 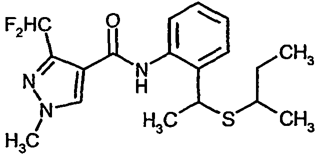 | 500 | 100 |
| 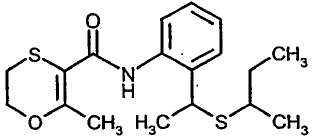 | 500 | 100 |
| 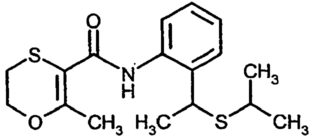 | 500 | 100 |
| 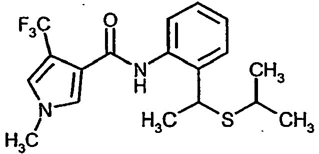 | 500 | 100 |
| 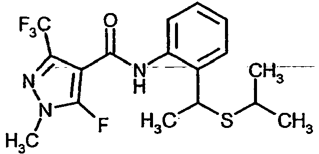 | 500 | 96 |
| 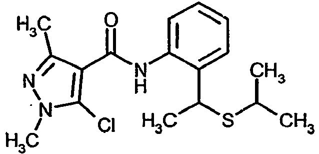 | 500 | 100 |
| 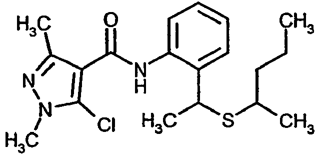 | 500 | 100 |
| 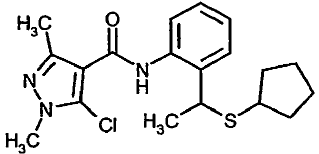 | 500 | 100 |

### Beispiel E

### Alternaria-Test (Tomate) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24 h bei 100 % relativer Feuchte und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Alternaria-Test (Tomate)** / **protektiv** | | |
|---|---|---|
| Wirkstoff Erfindungsgemäß | Aufwandmenge an Wirkstoff in g/ha | Wirkungsgrad in % |
| 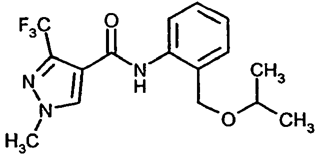 | 750 | 95 |
| 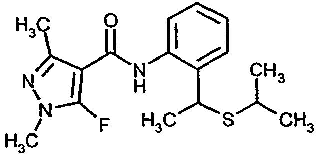 | 750 | 90 |
| 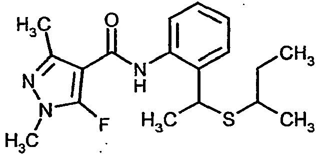 | 750 | 95 |

## Patentansprüche

1. Carboxamide der Formel (I) 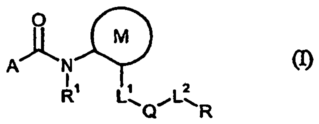 in welcher
R¹ für Wasserstoff C₁-C₈-Alkyl, C₁-C₆-Alkylsulfonyl, C₁-C₆-Alkylsulfonyl, C₁-C₄-Alkoxy-C₁-C₄-alkyl C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Color- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R², -CONR³R⁴ oder -CH₂NR⁵R⁶ steht,
R² für Wasserstoff C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R³ und R⁴ unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R³ und R⁴ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
R⁵ und R⁶ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R⁵ und R⁶ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
R⁷ für Wasserstoff oder C₁-C₆-Alkyl steht,
M für einen jeweils einfach durch R⁸ substituierten Phenyl- Ring steht,
R⁸ für Wasserstoff, Fluor, Chlor, Methyl, iso-Propyl, Methylthio oder Trifluormethyl steht,
R⁸ außerdem für Methoxy steht
R^{8-A} für Wasserstoff, Methyl, Methylthio oder Trifluormethyl steht,
L¹ für C₁-C₁₀-Alkylen (Alkandiyl) steht,
Q für O, S, SO, SO₂ oder NR⁹ steht,
L² für eine direkte Bindung, SiR¹⁰R¹¹ oder CO steht,
R für C₁-C₈-Alkyl, C₁-C₈-Alkoxy, C₁-C₄-Alkoxy-C₁-C₄-alkyl, C₁-C₄-Alkyl-thio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl oder C₃-C₅-Cycloalkyl steht,
R⁹ für Wasserstoff, C₁-C₈-Alkyl, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl oder C₃-C₆-Cycloalkyl steht,
R¹⁰ und R¹¹ unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
A für den Rest der Formel (A1) 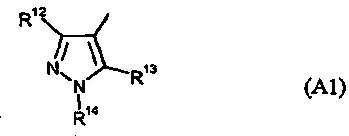 steht, in welcher
R¹² für Wasserstoff, Cyano, Halogen, Nitro, C₁-C₄-Alkyl, C₁-C₄-Alkoxy, C₁-C₄-Alkylthio, C₃-C₆-Cyclolalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-aLkyl steht,
R¹³ für Wasserstoff, Halogen, Cyano, C₁-C₄-Alkyl, C₁-C₄-Alkoxy oder C₁-C₄-Alkylthio steht,
R¹⁴ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,
oder
A für den Rest der Formel (A2) 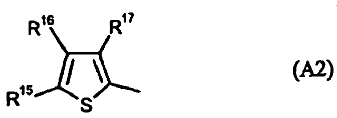 steht, in welcher
R¹⁵ und R¹⁶ unabhängig voneinander für Wasserstoff, Halogen, C₁-C₄-Alkyl oder C₁-C₄-Halogenalkyl mit 1 bis 5 Halogenatomen steht,
R¹⁷ für Halogen, Cyano oder C₁-C₄-Alkyl, oder C₁-C₄-Halogenalkyl oder C₁-C₄-Halogenalkoxy mit jeweils 1 bis 5 Halogenatomen steht,
oder
A für den Rest der Formel (A3) 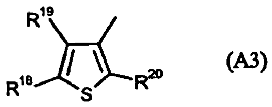 steht, in welcher
R¹⁸ und R¹⁹ unabhängig voneinander für Wasserstoff, Halogen, C₁-C₄-Alkyl oder C₁-C₄-Halogenalkyl mit 1 bis 5 Halogenatomen steht,
R²⁰ für Wasserstoff, Halogen, C₁-C₄-Alkyl oder C₁-C₄-Halogenalkyl mit 1 bis 5 Halogenatomen steht,
oder
A für den Rest der Formel (A4) 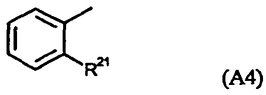 steht, in welcher
R²¹ für Halogen, Hydroxy, Cyano, C₁-C₆-Alkyl, C₁-C₄-Halogen-alkyl, C₁-C₄-Halogenalkoxy oder C₁-C₄-Halogenalkylthio mit jeweils 1 bis 5 Halogenatomen steht,
oder
A für den Rest der Formel (A5) 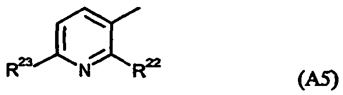 steht, in welcher
R²² für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R²³ für Wasserstoff Halogen, Cyano, C₁-C₄-Alkyl, C₁-C₄-Alkoxy, C₁-C₄-Alkylthio, C₁-C₄₋Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder
A für den Rest der Formel (A6) 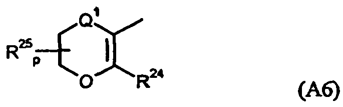 steht, in welcher
R²⁴ für C₁-C₄-Alkyl oder C₁-C₄-Halogenalkyl mit 1 bis 5 Halogenatomen steht,
R²⁵ für C₁-C₄-Alkyl steht,
Q¹ für S (Schwefel), SO, SO₂ oder CH₂ steht,
p für 0, 1 oder 2, wobei R²⁵ für identische oder verschiedene Reste steht, wenn p für 2 steht,
oder
A für den Rest der Formel (A7) 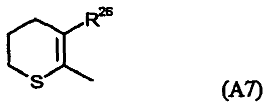 steht, in welcher
R²⁶ für C₁-C₄-Alkyl oder C₁-C₄-Halogenalkyl mit 1 bis 5 Halogenatomen steht,
oder
A für den Rest der Formel (A8) 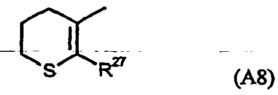 steht, in welcher
R²⁷ für C₁-C₄-Alkyl oder C₁-C₄-Halogenalkyl mit 1 bis 5 Halogenatomen steht,
oder
A für den Rest der Formel (A9) 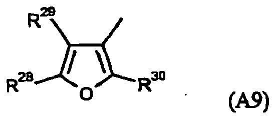 steht, in welcher
R²⁸ und R²⁹ unabhängig voneinander für Wasserstoff, Halogen, Amino, C₁-C₄-Alkyl oder C₁-C₄-Halogenalkyl mit 1 bis 5 Halogenatomen stehen,
R³⁰ für Wasserstoff, Halogen, C₁-C₄-Alkyl oder C₁-C₄-Halogenalkyl mit 1 bis 5 Halogenatomen steht,
oder
A für den Rest der Formel (A10) 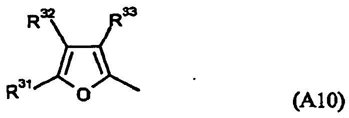 steht, in welcher
R³¹ und R³² unabhängig voneinander für Wasserstoff, Halogen, Amino, Nitro, C₁-C₄-Alkyl oder C₁-C₄-Halogenalkyl having 1 bis 5 Halogenatomen stehen.
R³³ für Wasserstoff, Halogen, C₁-C₄-Alkyl oder C₁-C₄-Halogenalkyl mit 1 bis 5 Halogenatomen steht,
oder
A für den Rest der Formel (A11) 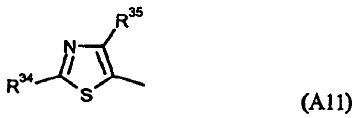 steht, in welcher
R³⁴ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R³⁵ für Halogen, C₁-C₄-Alkyl oder C₁-C₄-Halogenalkyl mit 1 bis 5 Halogenatomen steht,
oder
A für den Rest der Formel (A12) 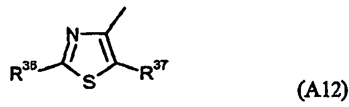 steht, in welcher
R³⁶⁻ für Wasserstoff Halogen, Amino, -C₁-C₄-Alkylamino, Di-(C₁-C₄-alkyl)amino, Cyano, C₁-C₄Alkyl oder C₁-C₄-Halogenalkyl mit 1 bis 5 Halogenatomen steht,
R³⁷ für Halogen, C₁-C₄-Alkyl oder C₁-C₄-Halogenalkyl mit 1 bis 5 Halogenatomen steht,
oder
A für den Rest der Formel (A13) 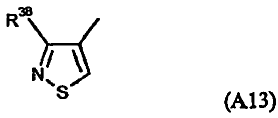 steht, in welcher
R³⁸ für Halogen, C₁-C₄-Alkyl oder C₁-C₄-Halogenalkyl mit 1 bis 5 Halogenatomen steht,
oder
A für den Rest der Formel (A14) 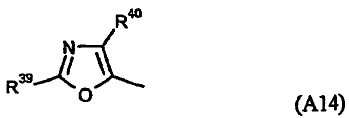 steht, in welcher
R³⁹ für Wasserstoff oder C₁-C₄-Alkyl steht,
R⁴⁰ für Halogen oder C₁-C₄-Alkyl steht,
oder
A für den Rest der Formel (A15) 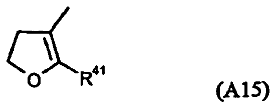 steht, in welcher
R⁴¹ für C₁-C₄-Alkyl oder C₁-C₄-Halogenalkyl mit 1 bis 5 Halogenatomen steht,
oder
A für den Rest der Formel (A16) 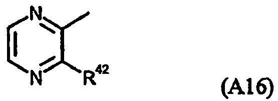 steht, in welcher
R⁴² für Wasserstoff Halogen, C₁-C₄-Alkyl oder C₁-C₄-Halogenalkyl mit 1 bis 5 Halogenatomen steht,
oder
A für den Rest der Formel (A17) 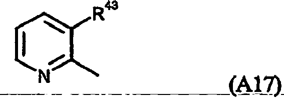 steht, in welcher
R⁴³ für Halogen, Hydroxy, C₁-C₄-Alkyl, C₁-C₄-Alkoxy, C₁-C₄-Alkylthio C₁-C₄-Halogenalkyl, C₁-C₄-Halogenalkylthio oder C₁-C₄-Halogenalkoxy mit jeweils 1 bis 5 Halogenatomen steht,
oder
A für den Rest der Formel (A18) 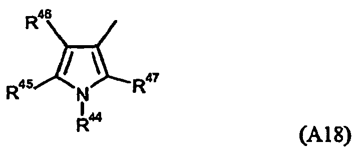 steht, in welcher
R⁴⁴ für Wasserstoff Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkyl-sulfonyl, Di(C₁-C₄-alkyl)aminosulfonyl, C₁-C₄-Alkylcarbonyl oder jeweils gegebenenfalls substituiertes Phenylsulfonyl oder Benzoyl steht,
R⁴⁵ für Wasserstoff Halogen, C₁-C₄-Alkyl oder C₁-C₄-Halogenalkyl mit 1 bis 5 Halogenatomen steht,
R⁴⁶ für Wasserstoff, Halogen, Cyano, C₁-C₄-Alkyl oder C₁-C₄-Halogenalkyl mit 1 bis 5 Halogenatomen steht,
R⁴⁷ für Wasserstoff Halogen, C₁-C₄-Alkyl oder C₁-C₄-Halogenalkyl mit 1 bis 5 Halogenatomen steht,
oder
A für den Rest der Formel (A19) 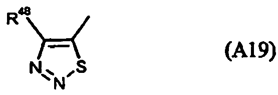 steht, in welcher
R⁴⁸ für C₁-C₄-Alkyl steht.

2. Carboxamide der Formel (I) gemäß Anspruch 1, in welcher R nicht für Alkoxy steht, wenn L² für eine direkte Bindung steht.

3. Carboxamide der Formel (I) gemäß Anspruch 1 oder 2, in welcher
R¹ für Wasserstoff C₁-C₆-Alkyl, C₁-C₄-Alkylsulfinyl, C₁-C₄-Alkylsulfonyl, C₁-C₄-Alkoxy-C₁-C₃-alkyl, C₄-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₄-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₄-alkyl, Halogen-(C₁-C₃-alk-oxy)carbonyl-C₁-C₃-alkyl-mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R², -CONR³R⁴ oder -CH₂NR⁵R⁶ steht,
R² für Wasserstoff C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₄-Halogencycloalkyl mit jeweils 1 bis 9 Fluor-, Chlor- und/oder Bromatomen steht,
R³ und R⁴ unabhängig voneinander jeweils für Wasserstoff C₁-C₄-Alkyl, C₁-C₃Alkoxy-C₁-C₃-alkyl, C₃-C₆-Cycloalkyl; C₁-C₄-Halogenalkyl, Halogen-C₁-C₃-alkoxy-C₁-C₃-Alkyl, C₃-C₆-Halogencycloalkyl mit jeweils 1 bis 9 Fluor-, Chlor- und/oder Bromatomen stehen,
R³ und R⁴ außerdem gemeinsam mit dem Stickstoffatom, an das sie gebunden sind, einen gegebenenfalls einfach bis vierfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR⁷ enthalten kann,
R⁵ und R⁶ unabhängig voneinander für Wasserstoff, C₁-C₆-Alkyl, C₃-C₆-Cycloalky- C₁-C₄-Halogenalkyl, C₃-C₆-Halogencycloalkyl mit jeweils 1 bis 9 Fluor-, Chlor- und/oder Bromatomen stehen,
R⁵ und R⁶ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff Schwefel oder NR⁷ enthalten kann,
R⁷ für Wasserstoff oder C₁-C₄-Alkyl steht,
M für steht 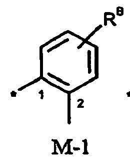
wobei die mit "*" markierte Bindung mit dem Amid verknüpft ist,
R⁸ für Wasserstoff: Fluor, Chlor, Methyl, iso-Propyl, Methylthio oder Trifluormethyl steht,
R⁸ außerdem für Methoxy steht,
R^{8-A} für Wasserstoff, Methyl, Methylthio oder Trifluormethyl steht,
L¹ für C₁-C₁₀-Alkylen (Alkandiyl) steht,
Q für O, S, SO, SO₂ oder NR⁹ steht,
L² für eine direkte Bindung, SiR¹⁰R¹¹ oder CO steht,
R für C₁-C₆Alkyl, C₁-C₆-Alkoxy, C₁-C₃-Alkoxy-C₁-C₃-alkyl, C₁-C₃-Alkylthio-C₁-C₃-alkyl, C₁-C₄-Halogenalkyl oder C₃-C₆-Cycloalkyl steht,
R⁹ für Wasserstoff C₁-C₆-Alkyl, C₁-C₃-Alkoxy-C₁-C₃-alkyl, C₁-C₃-Alkylthio-C₁-C₃-alkyl oder C₃-C₆-Cycloalkyl steht,
R¹⁰ und R¹¹ unabhängig voneinander für C₁-C₆-Alkyl C₁-C₆-Alkoxy, C₁-C₃-Alkoxy-C₁-C₃ alkyl oder C₁-C₃-Alkylthio-C₁-C₃-alkyl stehen,
A für den Rest der Formel (A1) 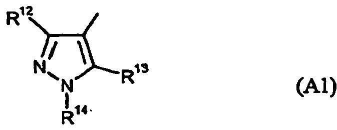 steht, in welcher
R¹² für Wasserstoff Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl steht,
R¹³ für Wasserstoff, Fluor, Chlor, Brom, Iod, Methyl, Ethyl, Methoxy, Ethoxy, Methylthio oder Ethylthio steht,
R¹⁴ für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxymethyl, Cyclopropyl, Cyclopentyl, Cyclohexyl oder Phenyl steht,
oder
A für den Rest der Formel (A2) 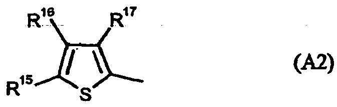 steht, in welcher
R¹⁵ und R¹⁶ unabhängig voneinander für Wasserstoff Fluor, Chlor, Brom, Methyl, ethyl oder C₁-C₂-Halogenalkyl mit 1 bis 5 Fluor, Chlor und/oder Bromatomen steht,
R¹⁷ für Fluor, Chlor, Brom, Cyano, Methyl, Ethyl, C₁-C₁-Halogenalkyl oder C₁-C₂-Halogenalkoxy mit jeweils 1 bis 5 Fluor, Chlor und/oder Bromatomen steht,
oder
A für den Rest der Formel (A3) 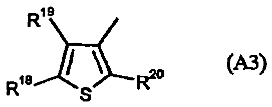 steht, in welcher
R¹⁸ und R¹⁹ unabhängig voneinander für Wasserstoff, Fluor, Chlor, Brom, Methyl, Ethyl oder C₁-C₂-Halogenalkyl mit 1 bis 5 Fluor, Chlor und/oder Bromatomen steht,
R²⁰ für Wasserstoff Fluor, Chlor, Brom, Methyl, Ethyl oder C₁-C₂-Halogenalky mit 1 bis 5 Fluor, Chlor und/oder Bromatomen steht,
oder
A für den Rest der Formel (A4) 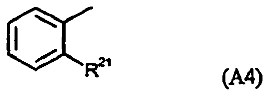 steht, in welcher
R²¹ für Fluor, Chlor, Brom, Iod, Hydroxy, Cyano, C₁-C₄-Alkyl, C₁-C₂-Halogenalkyl C₁-C₂-Halogenalkoxy oder C₁-C₂-Halogenalkylthio mit jeweils 1 bis 5 Fluor, Chlor und/oder Bromatomen steht,
oder
A für den Rest der Formel (A5) 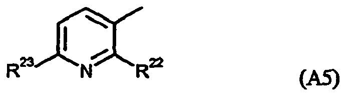 steht, in welcher
R²² für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomensteht,
R²³ für Wasserstoff, Fluor, Chlor, Brom, Iod, Cyano, C₁-C₄-Alkyl, Methoxy, Ethoxy, Methylthio, Ethylthio, C₁-C₂Halogenalkyl oder C₁-C₂-Halogenalkyloxy mit jeweils 1 bis 5 Fluor, Chlor und/oder Bromatomen, C₁-C₂-Alkylsulphinyl oder C₁-C₂-Alkylsulphonyl steht,
oder
A für den Rest der Formel (A6) 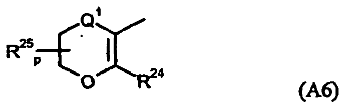 steht, in welche
R²⁴ für Methyl, Ethyl oder C₁-C₂-Halogenalkyl mit 1 bis 5 Fluor, Chlor und/oder Bromatomen steht,
R²⁵ für Methyl oder Ethyl steht,
Q¹ für S (Schwefel), SO₂ oder CH₂ steht,
p für 0 oder 1 steht,
oder
A für den Rest der Formel (A7) 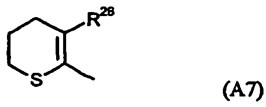 steht, in welcher
R²⁶ für Methyl, Ethyl oder C₁-C₂-Halogenalkyl mit 1 bis 5 Fluor, Chlor und/oder Bromatomen steht,
oder
A für den Rest der Formel (A8) 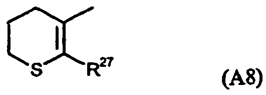 steht, in welcher
R²⁷ für Methyl, Ethyl, Trifluormethyl, Difluormethyl, Difluorchlormethyl ober Trichlormethyl steht,
oder
A für den Rest der Formel (A9) 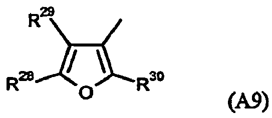 steht, in welcher
R²⁸ und R²⁹ unabhängig voneinander für Wasserstoff Fluor, Chlor, Brom, Amino, Methyl, Ethyl oder C₁-C₂-Halogenalkyl mit 1 bis 5 Fluor, Chlor und/oder Bromatomen stehen,
R³⁰ für Wasserstoff Fluor, Chlor, Brom, Iod, methyl, Ethyl oder C₁-C₂-Halogenalkyl mit 1 bis 5 Fluor, Chlor und/oder Bromatomen steht,
oder
A für den Rest der Formel (A10) 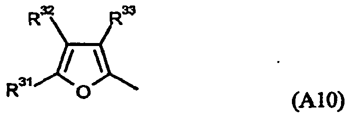 steht, in welcher
R³¹ und R³² unabhängig voneinander für Wasserstoff Fluor, Chlor, Brom, Amino, Nitro, Methyl, Ethyl oder C₁-C₂-Halogenalkyl mit 1 bis 5 Fluor, Chlor und/oder Bromatomen stehen,
R³³ für Wasserstoff Fluor, Chlor, Brom, Methyl, Ethyl oder C₁-C₂Halogenalkyl mit 1 bis 5 Fluor, Chlor und/oder Bromatomen steht,
oder
A für den Rest der Formel (A11) 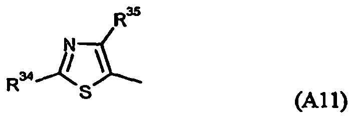 steht, in welcher
R³⁴ für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen steht,
R³⁵ für Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A12) 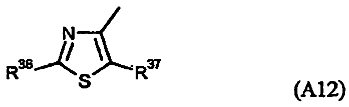 steht, in welcher
R³⁶ für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen steht,
R³⁷ für Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A13) 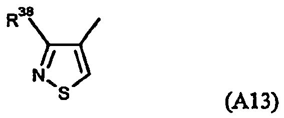 steht, in welcher
R³⁸ für Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A14) 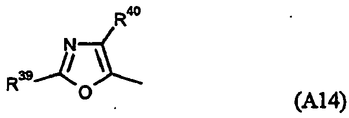 steht, in welcher
R³⁹ für Wasserstoff, Methyl oder Ethyl steht,
R⁴⁰ für Fluor, Chlor, Brom, Methyl oder Ethyl steht,
oder
A für den Rest der Formel (A15) 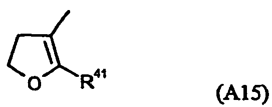 steht, in welcher
R⁴¹ für Methyl, Ethyl oder C₁-C₂-Halogenalkyl mit 1 bis 5 Fluor, Chlor und/oder Bromatomen steht,
oder
A für den Rest der Formel (A16) 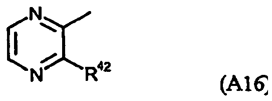 steht, in welcher
R⁴² für Wasserstoff, Fluor, Chlor, Brom, Methyl, Ethyl oder C₁-C₂-Halogenalkyl mit 1 bis 5 Fluor, Chlor und/odea Bromatomen steht,
oder
A für den Rest der Formel (A17) 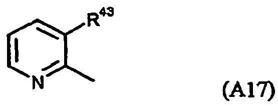 steht, in welcher
R⁴³ für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomensteht,
oder
A für den Rest der Formel (A18) 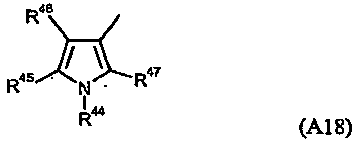 steht, in welcher
R⁴⁴ für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl Hydroxymethyl, Hydroxyethyl, Methylsulfonyl oder Dimethylaminosulfonyl steht,
R⁴⁵ für Wasserstoff Fluor, Chlor, Brom, Methyl, Ethyl oder C₁-C₄-Halogenalkyl mit 1 bis 5 Fluor-, Chlor- und/oder Bromatomen steht,
R⁴⁶ für Wasserstoff Fluor, Chlor, Brom, Iod, Cyano, Methyl, Ethyl, iso-Propyl oder C₁-C₂-Halogenalkyl mit 1 bis 5 Fluor-, Chlor- und/oder Bromatomen steht,
R⁴⁷ für Wasserstoff, Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A19) 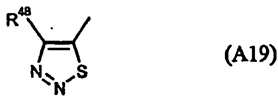 steht, in welcher
R⁴⁸ für Methyl, Ethyl, n-Propyl oder iso-Propyl steht.

4. Verfahren zum Herstellen der Carboxamide der Formel (I) gemäß Anspruch 1, **dadurch gekennzeichnet, dass** man
(a) Carbonsaure-Derivate der Formel (II) 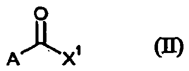 in welcher
A die oben angegebenen Bedeutungen hat und
X¹ für Halogen oder Hydroxy steht,
mit Anilin-Derivaten der Formel (III) 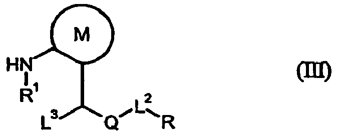 in welcher
R¹, M, Q L² und R die oben angegebenen Bedeutungen haben,
L³ für Wasserstoff oder C₁-C₉-Alkyl steht,
gegebenenfalls in Gegenwart eines Katalysators, gegebenenfalls in Gegenwart eines Kondensationsmittels, gegebenenfalls in Gegenwart eines Säurebindemittels und gegebenenfills in Gegenwart eines Verdünmmgsmittels umsetzt,
oder
(b) Carboxamide der Formel (IV) 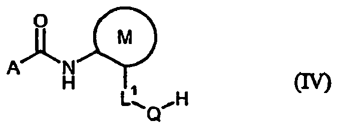 in welcher M, L¹, Q und A die oben angegebenen Bedeutungen haben, mit einer Verbindung der Formel (V) 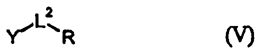 in welcher
L² und R die oben angegebenen Bedeutungen haben,
Y für Halogen, Triflat (Trifluormethylsulfonyl), Mesylat (Methylsulfonyl) oder Tosylat (4-Methylphenylsulfonyl) steht,
in Gegenwart einer Base und in Gegenwart eines Verdünnungsmittels umsetzt,
oder
(c) Carboxamide der Formel (I-a) 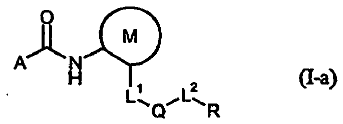 in welcher M, L¹, Q, L², R und A die oben angegebenen Bedeutungen haben,
mit Halogeniden der Formel (VI)
R^{1-A}-X² (VI)
in welcher
X² für Chlor, Brom oder Iod steht,
R^{1-A} für C₁-C₈-Alkyl, C₁-C₄-Alkylsulfinyl, C₁-C₆-Alkylsulfonyl, C₁-C₄-Alkoxy-C₁-C₄-alkyl, C₃-C₈-Cycloalkyl, C₁-C₆-Halogenalkyl, C₁-C₄-Halogenalkylthio, C₁-C₄-Halogenalkylsulfonyl, C₁-C₄-Halogenalkylsulfonyl Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)-carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₄-Halogencycloalkyl)carbonyl mit jeweils 1 bis 9 Fluor-, Chlor- und/oder Bromatomen; oder -C(=C)C(=O)R², -CONR³R⁴ oder -CH₂NR⁵R⁶ steht,
wobei R², R³, R⁴, R⁵ und R⁶ die oben angegebenen Bedeutungen haben,
in Gegenwart einer Base und in Gegenwart eines Verdünnungsmittels umsetzt.

5. Mittel zur Bekämpfung unerwünschter Mikroorganismen, **gekennzeichnet durch** einen Gehalt an mindestens einem Carboxamid der Formel (I) gemäß Anspruch 1 neben Streckmitteln und/oder oberflächenaktiven Stoffen.

6. Verwendung von Carboxamiden der Formel (I) gemäß Anspruch 1 zur Bekämpfimg unerwünschter Mikroorganismen.

7. Verfahren zur Bekämpfung unerwünschter Mikroorganismen, **dadurch gekennzeichnet, dass** man Carboxamide der Formel (I) gemäß Anspruch 1 auf die Mikroorganismen und/oder deren Lebensraum ausbringt

8. Verfahren zur Herstellung von Mitteln zur Bekämpfung unerwünschter Mikroorganismen, **dadurch gekennzeichnet, dass** man Carboxamide der Formel (I) gemäß Anspruch 1 mit Streckmitteln und/oder oberflächenaktiven Stoffen vermischt

## Claims

1. Carboxamides of the formula (I) 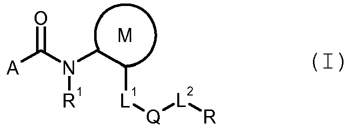 in which
R¹ stands for hydrogen, C₁-C₈ alkyl, C₁-C₆ alkylsulfinyl, C₁-C₆ alkylsulfonyl, C₁-C₄-alkoxy-C₁-C₄-alkyl, C₃-C₈ cycloalkyl; C₁-C₆ haloalkyl, C₁-C₄ haloalkylthio, C₁-C₄ haloalkylsulfinyl, C₁-C₄ haloalkylsulfonyl, halo-C₁-C₄-alkoxy-C₁-C₄-alkyl, C₃-C₈ halocycloalkyl with 1 to 9 fluorine, chlorine and/or bromine atoms in each case; formyl, formyl-C₁-C₃-alkyl, (C₁-C₃-alkyl)carbonyl-C₁-C₃-alkyl, (C₁-C₃-alkoxy)carbonyl-C₁-C₃-alkyl; halo-(C₁-C₃-alkyl)carbonyl-C₁-C₃-alkyl, halo-(C₁-C₃-alkoxy)carbonyl-C₁-C₃-alkyl with 1 to 13 fluorine, chlorine and/or bromine atoms in each case;
(C₁-C₈-alkyl)carbonyl, (C₁-C₈-alkoxy)carbonyl, (C₁-C₄-alkoxy-C₁-C₄-alkyl)carbonyl, (C₃-C₈-cycloalkyl)carbonyl; (C₁-C₆-haloalkyl)carbonyl, (C₁-C₆-haloalkoxy)carbonyl, (halo-C₁-C₄-alkoxy-C₁-C₄-alkyl)carbonyl, (C₃-C₈-halocycloalkyl)carbonyl with 1 to 9 fluorine, chlorine and/or bromine atoms in each case; or -C(=O)C(=O)R², -CONR³R⁴ or -CH₂NR⁵R⁶,
R² stands for hydrogen, C₁-C₈ alkyl, C₁-C₈ alkoxy, C₁-C₄-alkoxy-C₁-C₄-alkyl, C₃-C₈ cycloalkyl; C₁-C₆haloalkyl, C₁-C₆ haloalkoxy, halo-C₁-C₄-alkoxy-C₁-C₄-alkyl, C₃-C₈ halocycloalkyl with 1 to 9 fluorine, chlorine and/or bromine atoms in each case,
R³ and R⁴ stand independently of one another in each case for hydrogen, C₁-C₈ alkyl, C₁-C₄-alkoxy-C₁-C₄-alkyl, C₃-C₈ cycloalkyl; C₁-C₈ haloalkyl, halo-C₁-C₄-alkoxy-C₁-C₄-alkyl, C₃-C₈ halocycloalkyl with 1 to 9 fluorine, chlorine and/or bromine atoms in each case,
R³ and R⁴, moreover, form a substituted, saturated heterocycle with 5 to 8 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR⁷,
R⁵ and R⁶ stand independently of one another for hydrogen, C₁-C₈-alkyl, C₃-C₈ cycloalkyl; C₁-C₈ haloalkyl, C₃-C₈halocycloalkyl with 1 to 9 fluorine, chlorine and/or bromine atoms in each case,
R⁵ and R⁶, moreover, form a substituted, saturated heterocycle with 5 to 8 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR⁷,
R⁷ stands for hydrogen or C₁-C₆ alkyl,
M stands for a phenyl ring with a single substitution in each case by R⁸,
R⁸ stands for hydrogen, fluorine, chlorine, methyl, isopropyl, methylthio or trifluoromethyl,
R⁸ also stands for methoxy,
R^{8-A} stands for hydrogen, methyl, methylthio or trifluoromethyl,
L¹ stands for C₁-C₁₀ alkylene (alkanediyl),
Q stands for O, S, SO, SO₂ or NR⁹,
L² stands for a direct link, SiR¹⁰R¹¹ or CO,
R stands for C₁-C₈ alkyl, C₁-C₈ alkoxy, C₁-C₄-alkoxy-C₁-C₄-alkyl, C₁-C₄-alkylthio-C₁-C₄-alkyl, C₂-C₈ alkenyl, C₂-C₈ alkynyl, C₁-C₆ haloalkyl, C₂-C₆ haloalkenyl, C₂-C₆ haloalkynyl or C₃-C₆ cycloalkyl,
R⁹ stands for hydrogen, C₁-C₈ alkyl, C₁-C₄-alkoxy-C₁-C₄-alkyl, C₁-C₄-alkylthio-C₁-C₄-alkyl, C₂-C₈ alkenyl, C₂-C₈ alkynyl, C₁-C₆ haloalkyl, C₂-C₆ haloalkenyl, C₂-C₆ haloalkynyl or C₃-C₆ cycloalkyl,
R¹⁰ and R¹¹ stand independently of one another for hydrogen, C₁-C₈ alkyl, C₁-C₈ alkoxy, C₁-C₄-alkoxy-C₁-C₄-alkyl, C₁-C₄-alkylthio-C₁-C₄-alkyl or C₁-C₆ haloalkyl,
A stands for the group of the formula (A1) 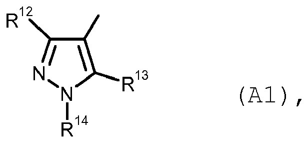 in which
R¹² stands for hydrogen, cyano, halogen, nitro, C₁-C₄ alkyl, C₁-C₄ alkoxy, C₁-C₄ alkylthio, C₃-C₆ cycloalkyl, C₁-C₄ haloalkyl, C₁-C₄ haloalkoxy or C₁-C₄ haloalkylthio, in each case with 1 to 5 halogen atoms, aminocarbonyl or aminocarbonyl-C₁-C₄-alkyl,
R¹³ stands for hydrogen, halogen, cyano, C₁-C₄-alkyl, C₁-C₄ alkoxy or C₁-C₄ alkylthio,
R¹⁴ stands for hydrogen, C₁-C₄ alkyl, hydroxy-C₁-C₄ alkyl, C₂-C₆ alkenyl, C₃-C₆ cycloalkyl, C₁-C₄-alkylthio-C₁-C₄-alkyl, C₁-C₄-alkoxy-C₁-C₄-alkyl, C₁-C₄ haloalkyl, C₁-C₄-haloalkylthio-C₁-C₄-alkyl, C₁-C₄-haloalkoxy-C₁-C₄-alkyl in each case with 1 to 5 halogen atoms, or phenyl,
or
A stands for the group of the formula (A2) 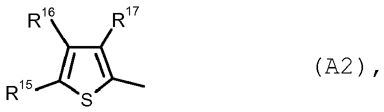 in which
R¹⁵ and R¹⁶ stand independently of one another for hydrogen, halogen, C₁-C₄ alkyl or C₁-C₄ haloalkyl with 1 to 5 halogen atoms,
R¹⁷ stands for halogen, cyano or C₁-C₄ alkyl, or C₁-C₄ haloalkyl or C₁-C₄ haloalkoxy with 1 to 5 halogen atoms in each case,
or
A stands for the group of the formula (A3) 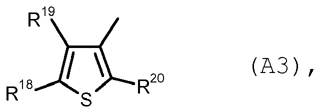 in which
R¹⁸ and R¹⁹ stand independently of one another for hydrogen, halogen, C₁-C₄ alkyl or C₁-C₄ haloalkyl with 1 to 5 halogen atoms,
R²⁰ stands for hydrogen, halogen, C₁-C₄ alkyl or C₁-C₄ haloalkyl with 1 to 5 halogen atoms,
or
A stands for the group of the formula (A4) 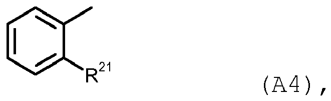 in which
R²¹ stands for halogen, hydroxy, cyano, C₁-C₆ alkyl, C₁-C₄ haloalkyl, C₁-C₄ haloalkoxy or C₁-C₄ haloalkylthio in each case with 1 to 5 halogen atoms,
or
A stands for the group of the formula (A5) 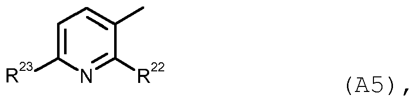 in which
R²² stands for halogen, hydroxy, cyano, C₁-C₄ alkyl, C₁-C₄ alkoxy, C₁-C₄ alkylthio, C₁-C₄ haloalkyl, C₁-C₄ haloalkylthio or C₁-C₄ haloalkoxy in each case with 1 to 5 halogen atoms,
R²³ stands for hydrogen, halogen, cyano, C₁-C₄ alkyl, C₁-C₄ alkoxy, C₁-C₄ alkylthio, C₁-C₉ haloalkyl, C₁-C₄ haloalkoxy in each case with 1 to 5 halogen atoms, C₁-C₄ alkylsulfinyl or C₁-C₄ alkylsulfonyl,
or
A stands for the group of the formula (A6) 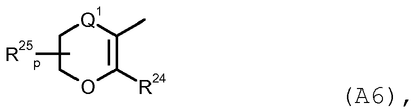 in which
R²⁴ stands for C₁-C₄ alkyl or C₁-C₄ haloalkyl with 1 to 5 halogen atoms,
R²⁵ stands for C₁-C₄ alkyl,
Q¹ stands for S (sulfur), SO, SO₂ or CH₂,
p stands for 0, 1 or 2, whereby R²⁵ stands for identical or various groups if p is 2,
or
A stands for the group of the formula (A7) 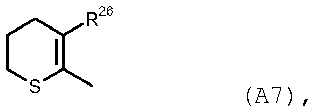 in which
R²⁶ stands for C₁-C₄ alkyl or C₁-C₄ haloalkyl with 1 to 5 halogen atoms,
or
A stands for the group of the formula (A8) 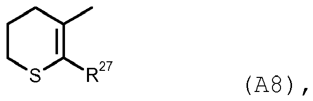 in which
R²⁷ stands for C₁-C₄ alkyl or C₁-C₄ haloalkyl with 1 to 5 halogen atoms,
or
A stands for the group of the formula (A9) 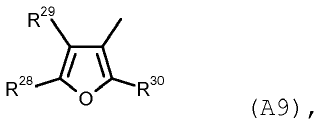 in which
R²⁸ and R²⁹ stand independently of one another for hydrogen, halogen, amino, C₁-C₄ alkyl or C₁-C₄ haloalkyl with 1 to 5 halogen atoms,
R³⁰ stands for hydrogen, halogen, C₁-C₄ alkyl or C₁-C₄ haloalkyl with 1 to 5 halogen atoms,
or
A stands for the group of the formula (A10) 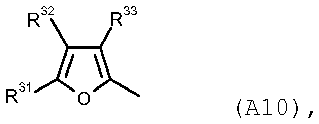 in which
R³¹ and R³² stand independently of one another for hydrogen, halogen, amino, nitro, C₁-C₄ alkyl or C₁-C₄ haloalkyl with 1 to 5 halogen atoms,
R³³ stands for hydrogen, halogen, C₁-C₄ alkyl or C₁-C₄ haloalkyl with 1 to 5 halogen atoms,
or
A stands for the group of the formula (A11) 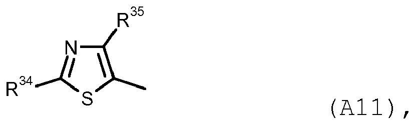 in which
R³⁴ stands for hydrogen, halogen, amino, C₁-C₄ alkylamino, di-(C₁-C₄ alkyl)amino, cyano, C₁-C₄ alkyl or C₁-C₄ haloalkyl with 1 to 5 halogen atoms,
R³⁵ stands for halogen, C₁-C₄ alkyl or C₁-C₄ haloalkyl with 1 to 5 halogen atoms,
or
A stands for the group of the formula (A12) 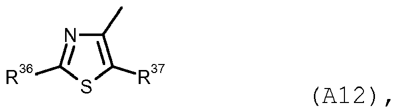 in which
R³⁶ stands for hydrogen, halogen, amino, C₁-C₄ alkylamino, di-(C₁-C₄ alkyl) amino, cyano, C₁-C₄ alkyl or C₁-C₄ haloalkyl with 1 to 5 halogen atoms,
R³⁷ stands for halogen, C₁-C₄ alkyl or C₁-C₄ haloalkyl with 1 to 5 halogen atoms,
or
A stands for the group of the formula (A13) 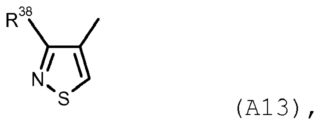 in which
R³⁸ stands for halogen, C₁-C₄ alkyl or C₁-C₄ haloalkyl with 1 to 5 halogen atoms,
or
A stands for the group of the formula (A14) 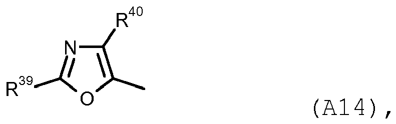 in which
R³⁹ stands for hydrogen or C₁-C₄ alkyl,
R⁴⁰ stands for halogen or C₁-C₄ alkyl,
or
A stands for the group of the formula (A15) 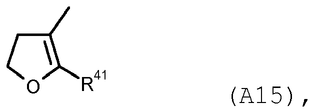 in which
R⁴¹ stands for C₁-C₄ alkyl or C₁-C₄ haloalkyl with 1 to 5 halogen atoms,
or
A stands for the group of the formula (A16) 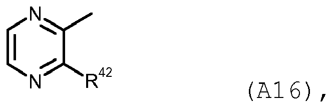 in which
R⁴² stands for hydrogen, halogen, C₁-C₄ alkyl or C₁-C₄ haloalkyl with 1 to 5 halogen atoms,
or
A stands for the group of the formula (A17) 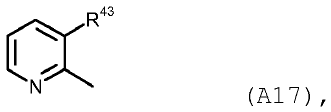 in which
R⁴³ stands for halogen, hydroxy, C₁-C₄ alkyl, C₁-C₄ alkoxy, C₁-C₄ alkylthio, C₁-C₄ haloalkyl, C₁-C₄ haloalkylthio or C₁-C₄ haloalkoxy with 1 to 5 halogen atoms in each case,
or
A stands for the group of the formula (A18) 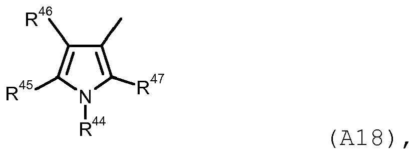 in which
R⁴⁴ stands for hydrogen, cyano, C₁-C₄ alkyl, C₁-C₄ haloalkyl with 1 to 5 halogen atoms, C₁-C₄-alkoxy-C₁-C₄-alkyl, hydroxy-C₁-C₄-alkyl, C₁-C₄ alkylsulfonyl, di(C₁-C₄ alkyl)aminosulfonyl, C₁-C₆ alkylcarbonyl or in each case possibly substituted phenylsulfonyl or benzoyl,
R⁴⁵ stands for hydrogen, halogen, C₁-C₄ alkyl or C₁-C₄ haloalkyl with 1 to 5 halogen atoms,
R⁴⁶ stands for hydrogen, halogen, cyano, C₁-C₄ alkyl or C₁-C₄ haloalkyl with 1 to 5 halogen atoms,
R⁴⁷ stands for hydrogen, halogen, C₁-C₄ alkyl or C₁-C₄ haloalkyl with 1 to 5 halogen atoms,
or
A stands for the group of the formula (A19) 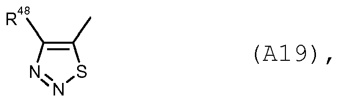 in which
R⁴⁸ stands for C₁-C₄ alkyl.

2. Carboxamides of the formula (I) according to Claim 1, in which R does not stand for alkoxy, if L² stands for a direct link.

3. Carboxamides of the formula (I) according to Claim 1 or 2, in which
R¹ stands for hydrogen, C₁-C₆ alkyl, C₁-C₄ alkylsulfinyl, C₁-C₄ alkylsulfonyl, C₁-C₃-alkoxy-C₁-C₃-alkyl, C₃-C₆ cycloalkyl; C₁-C₄ haloalkyl, C₁-C₄ haloalkylthio, C₁-C₄ haloalkylsulfinyl, C₁-C₄ haloalkylsulfonyl, halo-C₁-C₃-alkoxy-C₁-C₃-alkyl, C₃-C₈ halocycloalkyl with 1 to 9 fluorine, chlorine and/or bromine atoms in each case; formyl, formyl-C₁-C₃-alkyl, (C₁-C₃-alkyl)carbonyl-C₁-C₃-alkyl, (C₁-C₃-alkoxy)carbonyl-C₁-C₃-alkyl; halo-(C₁-C₃-alkyl)carbonyl-C₁-C₃-alkyl, halo-(C₁-C₃-alkoxy)carbonyl-C₁-C₃-alkyl with 1 to 13 fluorine, chlorine and/or bromine atoms in each case;
(C₁-C₆ alkyl)carbonyl, (C₁-C₄ alkoxy) carbonyl, (C₁-C₃-alkoxy-C₁-C₃-alkyl) carbonyl, (C₃-C₆ cycloalkyl)carbonyl; (C₁-C₄ haloalkyl)carbonyl, (C₁-C₄ haloalkoxy)carbonyl, (halo-C₁-C₃-alkoxy-C₁-C₃-alkyl)carbonyl, (C₃-C₆ halocycloalkyl)carbonyl with 1 to 9 fluorine, chlorine and/or bromine atoms in each case; or -C(=O)C(=O)R², -CONR³R⁴ or -CH₂NR⁵R⁶,
R² stands for hydrogen, C₁-C₆ alkyl, C₁-C₄ alkoxy, C₁-C₃-alkoxy-C₁-C₃-alkyl, C₃-C₆ cycloalkyl; C₁-C₄ haloalkyl, C₁-C₄ haloalkoxy, halo-C₁-C₃-alkoxy-C₁-C₃-alkyl, C₃-C₆ halocycloalkyl with 1 to 9 fluorine, chlorine and/or bromine atoms in each case,
R³ and R⁴ stand independently of one another in each case for hydrogen, C₁-C₆ alkyl, C₁-C₃-alkoxy-C₁-C₃-alkyl, C₃-C₆ cycloalkyl; C₁-C₄ haloalkyl, halo-C₁-C₃-alkoxy-C₁-C₃-alkyl, C₃-C₆ halocycloalkyl with 1 to 9 fluorine, chlorine and/or bromine atoms in each case,
R³ and R⁴, moreover, form a substituted, saturated heterocycle with 5 or 6 ring atoms together with the nitrogen atom to which they are bound, with single to quadruple, the same or various substitution by halogen or C₁-C₄ alkyl, whereby the heterocycle can contain 1 or 2 additional, non-adjacent hetero atoms constituted by oxygen, sulfur or NR⁷,
R⁵ and R⁶ stand independently of one another for hydrogen, C₁-C₆ alkyl, C₃-C₆ cycloalkyl; C₁-C₄ haloalkyl, C₃-C₆ halocycloalkyl with 1 to 9 fluorine, chlorine and/or bromine atoms in each case,
R⁵ and R⁶, moreover, form a substituted, saturated heterocycle with 5 or 6 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR⁷,
R⁷ stands for hydrogen or C₁-C₄ alkyl,
M stands for 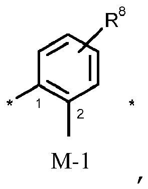 whereby the bond marked with an asterisk is linked to the amide,
R⁸ stands for hydrogen, fluorine, chlorine, methyl, isopropyl, methylthio or trifluoromethyl,
R⁸ also stands for methoxy,
R^{8-A} stands for hydrogen, methyl, methylthio or trifluoromethyl,
L¹ stands for C₁-C₁₀ alkylene (alkanediyl),
Q stands for O, S, SO, SO₂ or NR⁹,
L² stands for a direct link, SiR¹⁰R¹¹ or CO,
R stands for C₁-C₆ alkyl, C₁-C₆ alkoxy, C₁-C₃-alkoxy-C₁-C₃-alkyl, C₁-C₃-alkylthio-C₁-C₃-alkyl or C₁-C₄ haloalkyl or C₃-C₆ cycloalkyl,
R⁹ stands for hydrogen, C₁-C₆ alkyl, C₁-C₃-alkoxy-C₁-C₃-alkyl, C₁-C₃-alkylthio-C₁-C₃-alkyl or C₃-C₆ cycloalkyl,
R¹⁰ and R¹¹ stand independently of one another for C₁-C6 alkyl, C₁-C₆ alkoxy, C₁-C₃-alkoxy-C₁-C₃-alkyl or C₁-C₃-alkylthio-C₁-C₃-alkyl,
A stands for the group of the formula (A1) 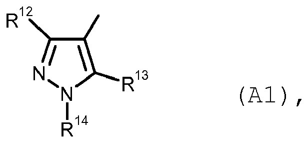 in which
R¹² stands for hydrogen, cyano, fluorine, chlorine, bromine, iodine, methyl, ethyl, isopropyl, methoxy, ethoxy, methylthio, ethylthio, cyclopropyl, C₁-C₂ haloalkyl, C₁-C₂ haloalkoxy in each case with 1 to 5 fluorine, chlorine and/or bromine atoms, trifluoromethylthio, difluoromethylthio, aminocarbonyl, aminocarbonylmethyl or aminocarbonylethyl,
R¹³ stands for hydrogen, fluorine, chlorine, bromine, iodine, methyl, ethyl, methoxy, ethoxy, methylthio or ethylthio,
R¹⁴ stands for hydrogen, methyl, ethyl, n-propyl, isopropyl, C₁-C₂ haloalkyl with 1 to 5 fluorine, chlorine and/or bromine atoms, hydroxymethyl, hydroxyethyl, cyclopropyl, cyclopentyl, cyclohexyl or phenyl,
or
A stands for the group of the formula (A2) 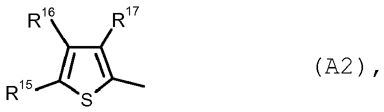 in which
R¹⁵ and R¹⁶ stand independently of one another for hydrogen, fluorine, chlorine, bromine, methyl, ethyl or C₁-C₂ haloalkyl with 1 to 5 fluorine, chlorine and/or bromine atoms,
R¹⁷ stands for fluorine, chlorine, bromine, cyano, methyl, ethyl, C₁-C₂ haloalkyl or C₁-C₂ haloalkoxy in each case with 1 to 5 fluorine, chlorine and/or bromine atoms,
or
A stands for the group of the formula (A3) 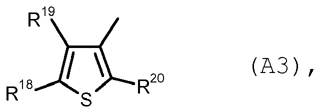 in which
R¹⁰ and R¹⁹ stand independently of one another for hydrogen, fluorine, chlorine, bromine, methyl, ethyl or C₁-C₂ haloalkyl with 1 to 5 fluorine, chlorine and/or bromine atoms,
R²⁰ stands for hydrogen, fluorine, chlorine, bromine, methyl, ethyl or C₁-C₂ haloalkyl with 1 to 5 fluorine, chlorine and/or bromine atoms,
or
A stands for the group of the formula (A4) 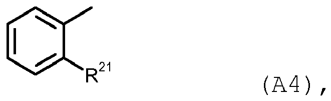 in which
R²¹ stands for fluorine, chlorine, bromine, iodine, hydroxy, cyano, C₁-C₄ alkyl, C₁-C₂ haloalkyl, C₁-C₂ haloalkoxy or C₁-C₂ haloalkylthio in each case with 1 to 5 fluorine, chlorine and/or bromine atoms,
or
A stands for the group of the formula (A5) 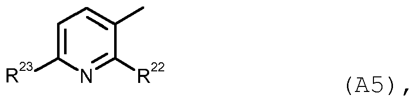 in which
R²² stands for fluorine, chlorine, bromine, iodine, hydroxy, cyano, C₁-C₄ alkyl, methoxy, ethoxy, methylthio, ethylthio, difluoromethylthio, trifluoromethylthio, C₁-C₂ haloalkyl or C₁-C₂ haloalkoxy in each case with 1 to 5 fluorine, chlorine and/or bromine atoms,
R²³ stands for hydrogen, fluorine, chlorine, bromine, iodine, cyano, C₁-C₄ alkyl, methoxy, ethoxy, methylthio, ethylthio, C₁-C₂ haloalkyl or C₁-C₂ haloalkoxy in each case with 1 to 5 fluorine, chlorine and/or bromine atoms, C₁-C₂ alkylsulfinyl or C₁-C₂ alkylsulfonyl,
or
A stands for the group of the formula (A6) 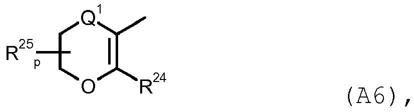 in which
R²⁴ stands for methyl, ethyl or C₁-C₂ haloalkyl with 1 to 5 fluorine, chlorine and/or bromine atoms,
R²⁵ stands for methyl or ethyl,
Q¹ stands for S (sulfur), SO₂ or CH₂,
p stands for 0 or 1,
or
A stands for the group of the formula (A7) 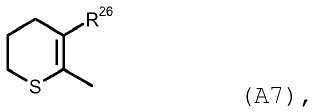 in which
R²⁶ stands for methyl, ethyl or C₁-C₂ haloalkyl with 1 to 5 fluorine, chlorine and/or bromine atoms,
or
A stands for the group of the formula (A8) 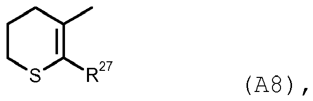 in which
R²⁷ stands for methyl, ethyl, trifluoromethyl, difluoromethyl, difluorochloromethyl or trichloromethyl,
or
A stands for the group of the formula (A9) 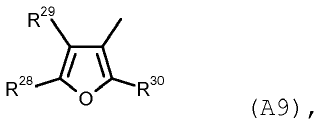 in which
R²⁸ and R²⁹ stand independently of one another for hydrogen, fluorine, chlorine, bromine, amino, methyl, ethyl or C₁-C₂ haloalkyl with 1 to 5 fluorine, chlorine and/or bromine atoms,
R³⁰ stands for hydrogen, fluorine, chlorine, bromine, iodine, methyl, ethyl or C₁-C₂ haloalkyl with 1 to 5 fluorine, chlorine and/or bromine atoms,
or
A stands for the group of the formula (A10) 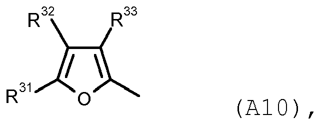 in which
R³¹ and R³² stand independently of one another for hydrogen, fluorine, chlorine, bromine, amino, nitro, methyl, ethyl or C₁-C₂ haloalkyl with 1 to 5 fluorine, chlorine and/or bromine atoms,
R³³ stands for hydrogen, fluorine, chlorine, bromine, methyl, ethyl or C₁-C₂ haloalkyl with 1 to 5 fluorine, chlorine and/or bromine atoms,
or
A stands for the group of the formula (A11) 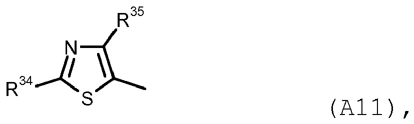 in which
R³⁴ stands for hydrogen, fluorine, chlorine, bromine, amino, C₁-C₄ alkylamino, di(C₁-C₄ alkyl)amino, cyano, methyl, ethyl or C₁-C₂ haloalkyl with 1 to 5 fluorine, chlorine and/or bromine atoms,
R³⁵ stands for fluorine, chlorine, bromine, methyl, ethyl or C₁-C₂ haloalkyl with 1 to 5 fluorine, chlorine and/or bromine atoms,
or
A stands for the group of the formula (A12) 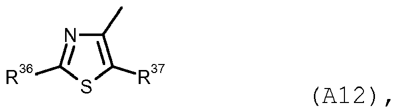 in which
R³⁶ stands for hydrogen, fluorine, chlorine, bromine, amino, C₁-C₄ alkylamino, di(C₁-C₄ alkyl)amino, cyano, methyl, ethyl or C₁-C₂ haloalkyl with 1 to 5 fluorine, chlorine and/or bromine atoms,
R³⁷ stands for fluorine, chlorine, bromine, methyl, ethyl or C₁-C₂ haloalkyl with 1 to 5 fluorine, chlorine and/or bromine atoms,
or
A stands for the group of the formula (A13) 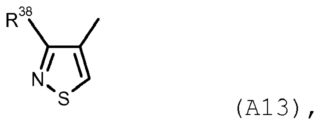 in which
R³⁸ stands for fluorine, chlorine, bromine, methyl, ethyl or C₁-C₂ haloalkyl with 1 to 5 fluorine, chlorine and/or bromine atoms,
or
A stands for the group of the formula (A14) 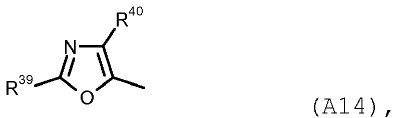 in which
R³⁹ stands for hydrogen, methyl or ethyl,
R⁴⁰ stands for fluorine, chlorine, bromine, methyl or ethyl,
or
A stands for the group of the formula (A15) 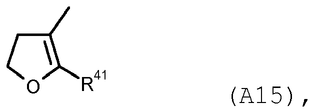 in which
R⁴¹ stands for methyl, ethyl or C₁-C₂ haloalkyl with 1 to 5 fluorine, chlorine and/or bromine atoms,
or
A stands for the group of the formula (A16) 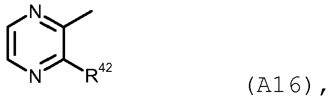 in which
R⁴² stands for hydrogen, fluorine, chlorine, bromine, methyl, ethyl or C₁-C₂ haloalkyl with 1 to 5 fluorine, chlorine and/or bromine atoms,
or
A stands for the group of the formula (A17) 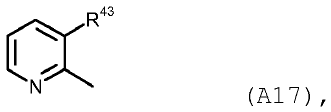 in which
R⁴³ stands for fluorine, chlorine, bromine, iodine, hydroxy, C₁-C₄ alkyl, methoxy, ethoxy, methylthio, ethylthio, difluoromethylthio, trifluoromethylthio, C₁-C₂ haloalkyl or C₁-C₂ haloalkoxy in each case with 1 to 5 fluorine, chlorine and/or bromine atoms,
or
A stands for the group of the formula (A18) 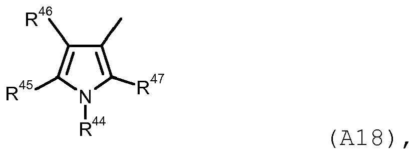 in which
R⁴⁴ stands for hydrogen, methyl, ethyl, C₁-C₂ haloalkyl with 1 to 5 fluorine, chlorine and/or bromine atoms, C₁-C₂-alkoxy-C₁-C₂-alkyl, hydroxymethyl, hydroxyethyl, methylsulfonyl or dimethylaminosulfonyl,
R⁴⁵ stands for hydrogen, fluorine, chlorine, bromine, methyl, ethyl or C₁-C₂ haloalkyl with 1 to 5 fluorine, chlorine and/or bromine atoms,
R⁴⁶ stands for hydrogen, fluorine, chlorine, bromine, iodine, cyano, methyl, ethyl, isopropyl or C₁-C₂ haloalkyl with 1 to 5 fluorine, chlorine and/or bromine atoms,
R⁴⁷ stands for hydrogen, fluorine, chlorine, bromine, methyl, ethyl or C₁-C₂ haloalkyl with 1 to 5 fluorine, chlorine and/or bromine atoms,
or
A stands for the group of the formula (A19) 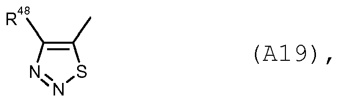 in which
R⁴⁸ stands for methyl, ethyl, n-propyl or isopropyl.

4. Process for synthesizing the carboxamides of the formula (I) according to Claim 1, **characterized in that**
(a) carboxylic acid derivatives the formula (II) 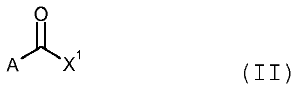 in which
A has the meanings specified above and
X¹ stands for halogen or hydroxy,
are reacted with aniline derivatives of the formula (III) 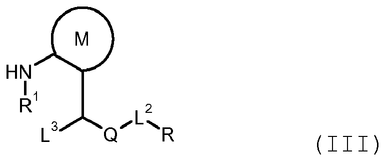 in which
R¹, M, Q, L² and R have the meanings specified above,
L³ stands for hydrogen or C₁-C₉ alkyl,
possibly in the presence of a catalyst, possibly in the presence of a condensation agent, possibly in the presence of an acid binder and possibly in the presence of a diluent,
or
(b) carboxamides of the formula (IV) 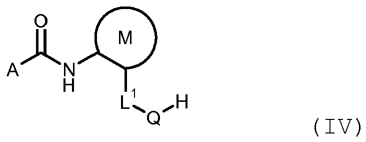 in which M, L¹, Q and A have the meanings specified above
are reacted with a compound of the formula (V), 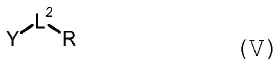 in which
L² and R have the meanings specified above and
Y stands for halogen, triflate (trifluoromethylsulfonyl), mesylate (methylsulfonyl) or tosylate (4-methylphenylsulfonyl),
in the presence of a base and in the presence of a diluent,
or
(c) carboxamides of the formula (I-a) 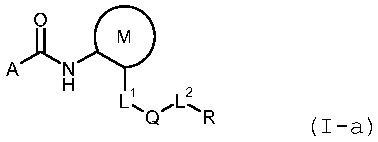 in which M, L¹, Q, L², R and A have the meanings specified above,
are reacted with halides of the formula (VI)
R^{1-A}—X² (VI)
in which
X² stands for chlorine, bromine or iodine,
R^{1-A} stands for C₁-C₈ alkyl, C₁-C₆ alkylsulfinyl, C₁-C₆ alkylsulfonyl, C₁-C₄-alkoxy-C₁-C₄-alkyl, C₃-C₈ cycloalkyl; C₁-C₆ haloalkyl, C₁-C₄ haloalkylthio, C₁-C₄ haloalkylsulfinyl, C₁-C₄ haloalkylsulfonyl, halo-C₁-C₄-alkoxy-C₁-C₄-alkyl, C₃-C₈ halocycloalkyl with 1 to 9 fluorine, chlorine and/or bromine atoms in each case; formyl, formyl-C₁-C₃-alkyl, (C₁-C₃-alkyl) carbonyl-C₁-C₃-alkyl, (C₁-C₃-alkoxy)carbonyl-C₁-C₃-alkyl; halo-(C₁-C₃-alkyl)carbonyl-C₁-C₃-alkyl, halo-(C₁-C₃-alkoxy)carbonyl-C₁-C₃-alkyl with 1 to 13 fluorine, chlorine and/or bromine atoms in each case; (C₁-C₈alkyl)carbonyl, (C₁-C₈ alkoxy)carbonyl, (C₁-C₄-alkoxy-C₁-C₄-alkyl)carbonyl, (C₃-C₈ cycloalkyl)carbonyl; (C₁-C₆ haloalkyl)carbonyl, (C₁-C₆ haloalkoxy)carbonyl, (halo-C₁-C₄-alkoxy-C₁-C₄-alkyl)carbonyl, (C₃-C₈ halocycloalkyl)carbonyl with 1 to 9 fluorine, chlorine and/or bromine atoms in each case; or -C(=O)C(_{=O})R², -CONR³R⁴ or -CH₂NR⁵R⁶,
whereby R², R³, R⁴, R⁵ and R⁶ have the meanings specified above,
in the presence of a base and in the presence of a diluent.

5. Media for combating undesirable microorganisms, **characterized by** containing at least one carboxamide of the formula (I) according to Claim 1 together with extenders and/or surface-active materials.

6. Use of carboxamides of the formula (I) according to Claim 1 to combat undesired microorganisms.

7. Processes for combating undesired microorganisms, **characterized in that** carboxamides of the formula (I) according to Claim 1 are applied to the microorganisms and/or their environment.

8. Processes for synthesizing materials to combat undesired microorganisms, **characterized in that** carboxamides of the formula (I) according to Claim 1 are mixed with extenders and/or surface-active materials.

## Revendications

1. Carboxamides de formule (I) 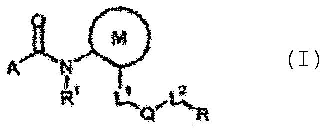 dans laquelle
R¹ représente un atome d'hydrogène, un groupe alkyle en C₁-C₈, alkyl (C₁-C₆) sulfinyle, alkyl (C₁-C₆)-sulfonyle, alcoxy(C₁-C₄)-alkyle(C₁-C₄), cycloalkyle en C₃-C₈ ; halogénoalkyle(C₁-C₆), halogénoalkyl(C₁-C₄)thio, halogénoalkyl(C₁-C₄)sulfinyle, halogénoalkyl(C₁-C₄)sulfonyle, halogénoalcoxy(C₁-C₄)-alkyle(C₁-C₄), halogénocycloalkyle(C₃-C₈) comportant chacun de 1 à 9 atomes de fluor, de chlore et/ou de brome ; formyle, formyl-alkyle(C₁-C₃), [alkyl(C₁-C₃)]-carbonyl-alkyle(C₁-C₃), [alcoxy(C₁-C₃)]carbonyl-alkyle(C₁-C₃) ; halogéno-[alkyl(C₁-C₃)]carbonyl-alkyle(C₁-C₃), halogéno-[alcoxy(C₁-C₃)]carbonyl-alkyle(C₁-C₃) comportant chacun de 1 à 13 atomes de fluor, de chlore et/ou de brome ; [alkyl(C₁-C₈)]carbonyle, [alcoxy(C₁-C₈)]carbonyle, [alcoxy(C₁-C₄)-alkyl(C₁-C₄)] carbonyle, [cycloalkyl(C₃-C₈)]carbonyle ; [halogénoalkyl(C₁-C₆)]carbonyle, [halogénoalcoxy(C₁-C₆)]carbonyle, [halogénoalcoxy(C₁-C₄)-alkyl(C₁-C₄)]carbonyle, [halogénocycloalkyl(C₃-C₈)]carbonyle comportant chacun de 1 à 9 atomes de fluor, de chlore et/ou de brome ; ou -C(=O)C(=O)R², -CONR³R⁴ ou -CH₂NR⁵R⁶,
R² représente un atome d'hydrogène, un groupe alkyle en C₁-C₈; alcoxy en C₁-C₈, alcoxy (C₁-C₄)-alkyle(C₁-C₄), cycloalkyle en C₃-C₈ ; halogénoalkyle(C₁-C₆), halogénoalcoxy (C₁-C₆), halogénoalcoxy(C₁-C₄₎-alkyle (C₁-C₄), halogéno-cycloalkyle(C₃-C₈) comportant chacun de 1 à 9 atomes de fluor, de chlore et/ou de brome,
R³ et R⁴ représentent chacun, indépendamment l'un de l'autre, un atome d'hydrogène, un groupe alkyle en C₁-C₈, alcoxy (C₁-C₄)-alkyle (C₁-C₄), cycloalkyle en C₃-C₈ ; halogénoalkyle(C₁-C₆), halogénoalcoxy(C₁-C₄)-alkyle(C₁-C₄), halogénocycloalkyle(C₃-C₆) comportant chacun de 1 à 9 atomes de fluor, de chlore et/ou de brome,
R³ et R⁴ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans l'ensemble constitué par les atomes d'oxygène, de soufre ou NR⁷,
R⁵ et R⁶ représentent, indépendamment l'un de l'autre, un atome d'hydrogène, un groupe alkyle en C₁-C₈, cycloalkyle en C₃-C₈, halogénoalkyle(C₁-C₆), halogénocycloalkyle(C₃-C₈) comportant chacun de 1 à 9 atomes de fluor, de chlore et/ou de brome,
R⁵ et R⁶ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans l'ensemble constitué par les atomes d'oxygène, de soufre ou NR⁷,
R⁷ représente un atome d'hydrogène ou un groupe alkyle en C₁-C₆,
M représente un cycle phényle chaque fois monosubstitué par R⁸,
R⁸ représente un atome d'hydrogène, de fluor, de chlore, un groupe méthyle, isopropyle, méthylthio ou trifluorométhyle,
R⁸ représente en outre un groupe méthoxy,
R^{8-A} représente un atome d'hydrogène, un groupe méthyle, méthylthio ou trifluorométhyle,
L¹ représente un groupe alkylène (alcanediyle) en C₁-C₁₀,
Q représente O, S, SO, SO₂ ou NR⁹,
L² représente une liaison directe, SiR¹⁰R¹¹ ou CO,
R représente un groupe alkyle en C₁-C₈, alcoxy en C₁-C₈, alcoxy (C₁-C₄) -alkyle (C₁-C₄), alkyl(C₁-C₄)-thio-alkyle(C₁-C₄), alcényle en C₂-C₈, alcynyle en C₂-C₈, halogénoalkyle(C₁-C₆), halogéno-alcényle(C₂-C₆), halogénoalcynyle(C₂-C₆) ou cycloalkyle en C₃-C₆,
R⁹ représente un atome d'hydrogène, un groupe alkyle en C₁-C₈, alcoxy (C₁-C₄) -alkyle (C₁-C₄), alkyl (C₁-C₄)-thio-alkyle(C₁-C₄), alcényle en C₂-C₈, alcynyle en C₂-C₈, halogénoalkyle(C₁-C₆), halogéno-alcényle(C₂-C₆), halogénoalcynyle(C₂-C₆) ou cycloalkyle en C₃-C₆,
R¹⁰ et R¹¹ représentent, indépendamment l'un de l'autre, un atome d'hydrogène, un groupe alkyle en C₁-C₈, alcoxy en C₁-C₈, alcoxy (C₁-C₄) -alkyle (C₁-C₄), alkyl(C₁-C₄)-thio-alkyle(C₁-C₄) ou halogéno-alkyle(C₁-C₆),
A représente le radical de formule (A1) 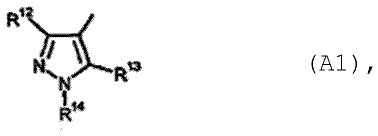 dans laquelle
R¹² représente un atome d'hydrogène, d'halogène, un groupe cyano, nitro, alkyle en C₁-C₄, alcoxy en C₁-C₄, alkyl(C₁-C₄)thio, cycloalkyle en C₃-C₆, halogénoalkyle(C₁-C₄), halogénoalcoxy(C₁-C₄) ou halogéno-alkyl(C₁-C₄)thio comportant chacun de 1 à 5 atomes d'halogène, un groupe aminocarbonyle ou aminocarbonyl-alkyle(C₁-C₄),
R¹³ représente un atome d'hydrogène, d'halogène, un groupe cyano, alkyle en C₁-C₄, alcoxy en C₁-C₄ ou alkyl(C₁-C₄)thio,
R¹⁴ représente un atome d'hydrogène, un groupe alkyle en C₁-C₄, hydroxyalkyle(C₁-C₄), alcényle en C₂-C₆, cycloalkyle en C₃-C₆, alkyl (C₁-C₄)-thio-alkyle(C₁-C₄), alcoxy(C₁-C₄)-alkyle(C₁-C₄), halogénoalkyle(C₁-C₄), halogénoalkyl(C₁-C₄)-thio-alkyle(C₁-C₄), halogénoalcoxy(C₁-C₄)-alkyle(C₁-C₄) comportant chacun de 1 à 5 atomes d'halogène, ou phényle,
ou
A représente le radical de formule (A2) 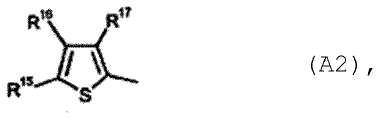 dans laquelle
R¹⁵ et R¹⁶ représentent, indépendamment l'un de l'autre, un atome d'hydrogène, d'halogène, un groupe alkyle en C₁-C₄ ou halogénoalkyle(C₁-C₄) comportant de 1 à 5 atomes d'halogène,
R¹⁷ représente un atome d'halogène, un groupe cyano ou alkyle en C₁-C₄, ou halogéno-alkyle(C₁-C₄) ou halogénoalcoxy(C₁-C₄) comportant chacun de 1 à 5 atomes d'halogène,
ou
A représente le radical de formule (A3) 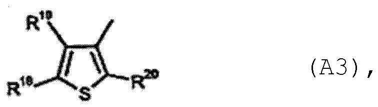 dans laquelle
R¹⁸ et R¹⁹ représentent, indépendamment l'un de l'autre, un atome d'hydrogène, d'halogène, un groupe alkyle en C₁-C₄ ou halogénoalkyle(C₁-C₄) comportant de 1 à 5 atomes d'halogène,
R²⁰ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A4) 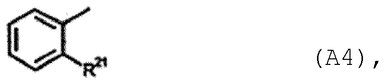 dans laquelle
R²¹ représente un atome d'halogène, un groupe hydroxy, cyano, alkyle en C₁-C₆, halogénoalkyle(C₁-C₄), halogénoalcoxy(C₁-C₄) ou halogénoalkyl(C₁-C₄)thio comportant chacun de 1 à 5 atomes d'halogène,
ou
A représente le radical de formule (A5) 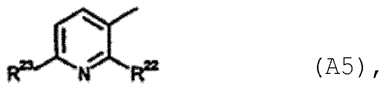 dans laquelle
R²² représente un atome d'halogène, un groupe hydroxy, cyano, alkyle en C₁-C₄, alcoxy en C₁-C₄, alkyl(C₁-C₄)thio, halogénoalkyle(C₁-C₄), halogénoalkyl(C₁-C₄)thio ou halogéno-alcoxy(C₁-C₄) comportant chacun de 1 à 5 atomes d'halogène,
R²³ représente un atome d'hydrogène, d'halogène, un groupe cyano, alkyle en C₁-C₄, alcoxy en C₁-C₄, alkyl(C₁-C₄)thio, halogénoalkyle(C₁-C₄), halogénoalcoxy(C₁-C₄) comportant chacun de 1 à 5 atomes d'halogène, alkyl(C₁-C₄)sulfinyle ou alkyl(C₁-C4)sulfonyle,
ou
A représente le radical de formule (A6) 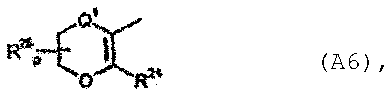 dans laquelle
R²⁴ représente un groupe alkyle en C₁-C₄ ou halogénoalkyle(C₁-C₄) comportant de 1 à 5 atomes d'halogène,
R²⁵ représente un groupe alkyle en C₁-C₄,
Q¹ représente S (soufre), SO, SO₂ ou CH₂,
p représente 0, 1 ou 2, R²⁵ représentant des radicaux identiques ou différents, lorsque p représente 2,
ou
A représente le radical de formule (A7) 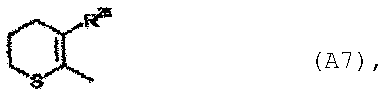 dans laquelle
R²⁶ représente un groupe alkyle en C₁-C₄ ou halogénoalkyle(C₁-C₄) comportant de 1 à 5 atomes d'halogène,
ou
A représente le radical de formule (A8) 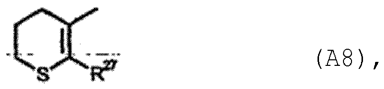 dans laquelle
R²⁷ représente un groupe alkyle en C₁-C₄ ou halogénoalkyle(C₁-C₄) comportant de 1 à 5 atomes d'halogène,
ou
A représente le radical de formule (A9) 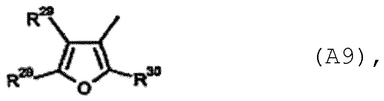 dans laquelle
R²⁸ et R²⁹ représentent, indépendamment l'un de l'autre, un atome d'hydrogène, d'halogène, un groupe amino, alkyle en C₁-C₄ ou halogénoalkyle(C₁-C₄) comportant de 1 à 5 atomes d'halogène,
R³⁰ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A10) 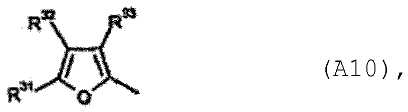 dans laquelle
R³¹ et R³² représentent, indépendamment l'un de l'autre, un atome d'hydrogène, d'halogène, un groupe amino, nitro, alkyle en C₁-C₄ ou halogénoalkyle(C₁-C₄) comportant de 1 à 5 atomes d'halogène,
R³³ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A11) 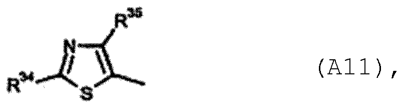 dans laquelle
R³⁴ représente un atome d'hydrogène, d'halogène, un groupe amino, alkyl(C₁-C₄)amino, di[alkyl(C₁-C₄)]amino, cyano, alkyle en C₁-C₄ ou halogénoalkyle(C₁-C₄) comportant de 1 à 5 atomes d'halogène,
R³⁵ représente un atome d'halogène, un groupe alkyle en C₁-C₄ ou halogénoalkyle(C₁-C₄) comportant de 1 à 5 atomes d'halogène,
ou
A représente le radical de formule (A12) 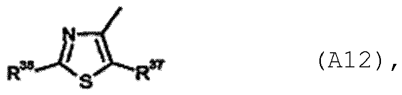 dans laquelle
R³⁶ représente un atome d'hydrogène, d'halogène, un groupe amino, alkyl(C₁-C₄)amino, di[alkyl(C₁-C₄)]amino, cyano, alkyle en C₁-C₄ ou halogénoalkyle(C₁-C₄) comportant de 1 à 5 atomes d'halogène,
R³⁷ représente un atome d'halogène, un groupe alkyle en C₁-C₄ ou halogénoalkyle(C₁-C₄) comportant de 1 à 5 atomes d'halogène,
ou
A représente le radical de formule (A13) 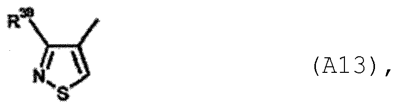 dans laquelle
R³⁸ représente un atome d'halogène, un groupe alkyle en C₁-C₄ ou halogénoalkyle(C₁-C₄) comportant de 1 à 5 atomes d'halogène,
ou
A représente le radical de formule (A14) 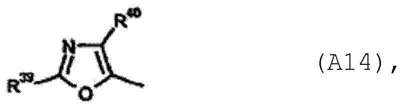 dans laquelle
R³⁹ représente un atome d'hydrogène ou un groupe alkyle en C₁-C₄,
R⁴⁰ représente un atome d'halogène ou un groupe alkyle en C₁-C₄,
ou
A représente le radical de formule (A15) 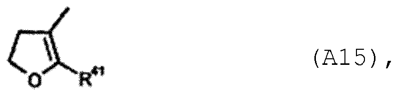 dans laquelle
R⁴¹ représente un groupe alkyle en C₁-C₄ ou halogénoalkyle(C₁-C₄) comportant de 1 à 5 atomes d'halogène,
ou
A représente le radical de formule (A16) 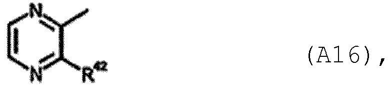 dans laquelle
R⁴² représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A17) 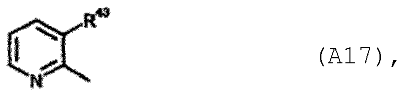 dans laquelle
R⁴³ représente un atome d'halogène, un groupe hydroxy, alkyle en C₁-C₄, alcoxy en C₁-C₄, alkyl(C₁-C₄)thio, halogénoalkyle(C₁-C₄), halogénoalkyl(C₁-C₄)thio ou halogéno-alcoxy(C₁-C₄) comportant chacun de 1 à 5 atomes d'halogène,
ou
A représente le radical de formule (A18) 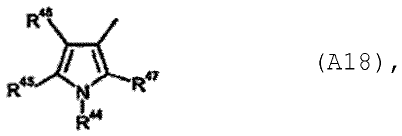 dans laquelle
R⁴⁴ représente un atome d'hydrogène, un groupe cyano, alkyle en C₁-C₄, halogénoalkyle(C₁-C₄) comportant de 1 à 5 atomes d'halogène, alcoxy(C₁-C₄)-alkyle(C₁-C₄), hydroxy-alkyle(C₁-C₄), alkyl(C₁-C₄)sulfonyle, di[alkyl(C₁-C₄)]aminosulfonyle, alkyl(C₁-C₆)-carbonyle ou à chaque fois un groupe phénylesulfonyle ou benzoyle éventuellement substitué,
R⁴⁵ représente un atome d'hydrogène, d'halogène, un groupe alkyle en C₁-C₄ ou halogénoalkyle(C₁-C₄) comportant de 1 à 5 atomes d'halogène,
R⁴⁶ représente un atome d'hydrogène, d'halogène, un groupe cyano, alkyle en C₁-C₄ ou halogénoalkyle(C₁-C₄) comportant de 1 à 5 atomes d'halogène,
R⁴⁷ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A19) 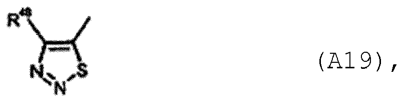 dans laquelle
R⁴⁸ représente un groupe alkyle en C₁-C₄.

2. Carboxamides de formule (I) selon la revendication 1, formule dans laquelle R ne représente pas un groupe alcoxy lorsque L² représente une liaison directe.

3. Carboxamides de formule (I) selon la revendication 1 ou 2, formule dans laquelle
R¹ représente un atome d'hydrogène, un groupe alkyle en C₁-C₆, alkyl (C₁-C₄) sulfinyle, alkyl(C₁-C₄)-sulfonyle, alcoxy(C₁-C₃)-alkyle(C₁-C₃), cycloalkyle en C₃-C₆ ; halogénoalkyle(C₁-C₄), halogéno-alkyl(C₁-C₄)thio, halogénoalkyl(C₁-C₄)sulfinyle, halogénoalkyl(C₁-C₄)sulfonyle, halogéno-alcoxy(C₁-C₃)-alkyle(C₁-C₃), halogéno-cycloalkyle(C₃-C₈) comportant chacun de 1 à 9 atomes de fluor, de chlore et/ou de brome ; formyle, formyl-alkyle(C₁-C₃), [alkyl(C₁-C₃)]-carbonyl-alkyle(C₁-C₃), [alcoxy(C₁-C₃)]carbonyl-alkyle(C₁-C₃) ; halogéno-[alkyl(C₁-C₃)]carbonyl-alkyle(C₁-C₃), halogéno-[alcoxy(C₁-C₃)]carbonyl-alkyle(C₁-C₃) comportant chacun de 1 à 13 atomes de fluor, de chlore et/ou de brome ; [alkyl(C₁-C₆)]carbonyle, [alcoxy(C₁-C₄)]carbonyle, [alcoxy(C₁-C₃)-alkyl(C₁-C₃) carbonyle, [cycloalkyl(C₃-C₆)]carbonyle ; [halogéno-alkyl(C₁-C₄)]carbonyle, [halogénoalcoxy(C₁-C₄)]-carbonyle, [halogénoalcoxy(C₁-C₃)-alkyl(C₁-C₃)]-carbonyle, [halogénocycloalkyl(C₃-C₆)]carbonyle comportant chacun de 1 à 9 atomes de fluor, de chlore et/ou de brome ; ou -C(=O)C(=O)R², -CONR³R⁴ ou -CH₂NR⁵R⁶,
R² représente un atome d'hydrogène, un groupe alkyle en C₁-C₆, alcoxy en C₁-C₄, alcoxy(C₁-C₃)-alkyle (C₁-C₃), cycloalkyle en C₃-C₆ ; halogénoalkyle(C₁-C₄), halogénoalcoxy(C₁-C₄), halogénoalcoxy(C₁-C₃)-alkyle (C₁-C₃), halogéno-cycloalkyle(C₃-C₆) comportant chacun de 1 à 9 atomes de fluor, de chlore et/ou de brome,
R³ et R⁴ représentent chacun, indépendamment l'un de l'autre, un atome d'hydrogène, un groupe alkyle en C₁-C₆, alcoxy (C₁-C₃) -alkyle (C₁-C₃), cycloalkyle en C₃-C₆ ; halogénoalkyle(C₁-C₄), halogénoalcoxy(C₁-C₃)-alkyle(C₁-C₃), halogénocycloalkyle(C₃-C₆) comportant chacun de 1 à 9 atomes de fluor, de chlore et/ou de brome,
R³ et R⁴ en outre forment ensemble avec l'atome d'azote, auquel ils sont liés, un hétérocycle saturé ayant 5 ou 6 atomes formant le cycle, portant éventuellement un à quatre substituants, identiques ou différents, halogéno ou alkyle en C₁-C₄, l'hétérocycle pouvant contenir 1 ou 2 autres hétéroatomes non contigus, choisis dans l'ensemble constitué par les atomes d'oxygène, de soufre ou NR⁷,
R⁵ et R⁶ représentent, indépendamment l'un de l'autre, un atome d'hydrogène, un groupe alkyle en C₁-C₆, cycloalkyle en C₃-C₆ ; halogénoalkyle (C₁-C₄), halogénocycloalkyle(C₃-C₆) comportant chacun de 1 à 9 atomes de fluor, de chlore et/ou de brome,
R⁵ et R⁶ en outre forment ensemble avec l'atome d'azote, auquel ils sont liés, un hétérocycle saturé ayant 5 ou 6 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans l'ensemble constitué par les atomes d'oxygène, de soufre ou NR⁷,
R⁷ représente un atome d'hydrogène ou un groupe alkyle en C₁-C₄,
M représente 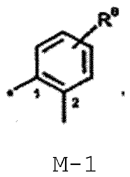 la liaison marquée par « * » étant liée à l'amide,
R⁸ représente un atome d'hydrogène, de fluor, de chlore, un groupe méthyle, isopropyle, méthylthio ou trifluorométhyle,
R⁸ représente en outre un groupe méthoxy,
R^{8-A} représente un atome d'hydrogène, un groupe méthyle, méthylthio ou trifluorométhyle,
L¹ représente un groupe alkylène (alcanediyle) en C₁-C₁₀,
Q représente O, S, SO, SO₂ ou NR⁹,
L² représente une liaison directe, SiR¹⁰R¹¹ ou CO,
R représente un groupe alkyle en C₁-C₆, alcoxy en C₁-C₆, alcoxy (C₁-C₃) -alkyle (C₁-C₃), alkyl (C₁-C₃)-thio-alkyle(C₁-C₃), halogénoalkyle(C₁-C₄) ou cycloalkyle en C₃-C₆,
R⁹ représente un atome d'hydrogène, un groupe alkyle en C₁-C₆, alcoxy(C₁-C₃)-alkyle(C₁-C₃), alkyl(C₁-C₃)-thio-alkyle(C₁-C₃) ou cycloalkyle en C₃-C₆,
R¹⁰ et R¹¹ représentent, indépendamment l'un de l'autre, un groupe alkyle en C₁-C₆, alcoxy en C₁-C₆, alcoxy(C₁-C₃)-alkyle(C₁-C₃) ou alkyl(C₁-C₃)-thio-alkyle(C₁-C₃),
A représente le radical de formule (A1) 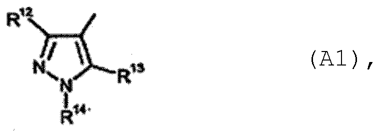 dans laquelle
R¹² représente un atome d'hydrogène, de fluor, de chlore, de brome, d'iode, un groupe cyano, méthyle, éthyle, isopropyle, méthoxy, éthoxy, méthylthio, éthylthio, cyclopropyle, halogénoalkyle(C₁-C₂), halogénoalcoxy(C₁-C₂) comportant chacun de 1 à 5 atomes de fluor, de chlore et/ou de brome, un groupe trifluorométhylthio, difluorométhylthio, aminocarbonyle, aminocarbonylméthyle ou aminocarbonyléthyle,
R¹³ représente un atome d'hydrogène, de fluor, de chlore, de brome, d'iode, un groupe méthyle, éthyle, méthoxy, éthoxy, méthylthio ou éthylthio,
R¹⁴ représente un atome d'hydrogène, un groupe méthyle, éthyle, n-propyle, isopropyle, halogénoalkyle(C₁-C₂) comportant de 1 à 5 atomes de fluor, de chlore et/ou de brome, un groupe hydroxyméthyle, hydroxyéthyle, cyclopropyle, cyclopentyle, cyclohexyle ou phényle,
ou
A représente le radical de formule (A2) 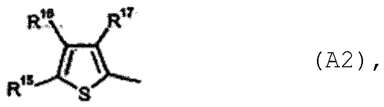 dans laquelle
R¹⁵ et R¹⁶ représentent, indépendamment l'un de l'autre, un atome d'hydrogène, de fluor, de chlore, de brome, un groupe méthyle, éthyle ou halogénoalkyle(C₁-C₂) comportant de 1 à 5 atomes de fluor, de chlore et/ou de brome,
R¹⁷ représente un atome de fluor, de chlore, de brome, un groupe cyano, méthyle, éthyle, halogénoalkyle(C₁-C₂) ou halogénoalcoxy(C₁-C₂) comportant chacun de 1 à 5 atomes de fluor, de chlore et/ou de brome,
ou
A représente le radical de formule (A3) 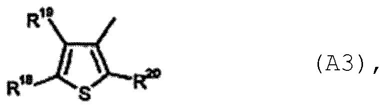 dans laquelle
R¹⁸ et R¹⁹ représentent, indépendamment l'un de l'autre, un atome d'hydrogène, de fluor, de chlore, de brome, un groupe méthyle, éthyle ou halogénoalkyle(C₁-C₂) comportant de 1 à 5 atomes de fluor, de chlore et/ou de brome,
R²⁰ représente un atome d'hydrogène, de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A4) 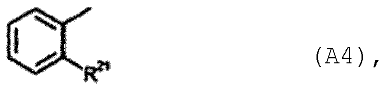 dans laquelle
R²¹ représente un atome de fluor, de chlore, de brome, d'iode, un groupe hydroxy, cyano, alkyle en C₁-C₄, halogénoalkyle(C₁-C₂), halogénoalcoxy(C₁-C₂) ou halogéno-alkyl(C₁-C₂)thio comportant chacun de 1 à 5 atomes de fluor, de chlore et/ou de brome,
ou
A représente le radical de formule (A5) 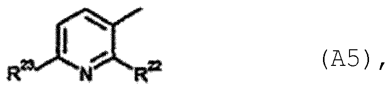 dans laquelle
R²² représente un atome de fluor, de chlore, de brome, d'iode, un groupe hydroxy, cyano, alkyle en C₁-C₄, méthoxy, éthoxy, méthylthio, éthylthio, difluorométhylthio, trifluorométhylthio, halogénoalkyle(C₁-C₂) ou halogénoalcoxy(C₁-C₂) comportant chacun de 1 à 5 atomes de fluor, de chlore et/ou de brome,
R²³ représente un atome d'hydrogène, de fluor, de chlore, de brome, d'iode, un groupe cyano, alkyle en C₁-C₄, méthoxy, éthoxy, méthylthio, éthylthio, halogénoalkyle(C₁-C₂) ou halogénoalcoxy(C₁-C₂) comportant chacun de 1 à 5 atomes de fluor, de chlore et/ou de brome, alkyl (C₁-C₂)sulfinyle ou alkyl(C₁-C₂)-sulfonyle,
ou
A représente le radical de formule (A6) 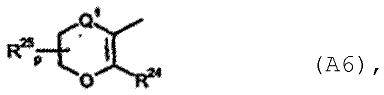 dans laquelle
R²⁴ représente un groupe méthyle, éthyle ou halogénoalkyle(C₁-C₂) comportant de 1 à 5 atomes de fluor, de chlore et/ou de brome,
R²⁵ représente un groupe méthyle ou éthyle,
Q¹ représente S (soufre), SO₂ ou CH₂,
p représente 0 ou 1,
ou
A représente le radical de formule (A7) 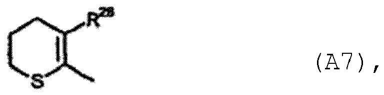 dans laquelle
R²⁶ représente un groupe méthyle, éthyle ou halogénoalkyle(C₁-C₂) comportant de 1 à 5 atomes de fluor, de chlore et/ou de brome,
ou
A représente le radical de formule (A8) 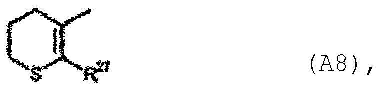 dans laquelle
R²⁷ représente un groupe méthyle, éthyle, trifluorométhyle, difluorométhyle, difluorochlorométhyle ou trichlorométhyle,
ou
A représente le radical de formule (A9) 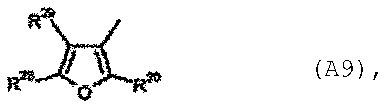 dans laquelle
R²⁸ et R²⁹ représentent, indépendamment l'un de l'autre, un atome d'hydrogène, de fluor, de chlore, de brome, un groupe amino, méthyle, éthyle ou halogénoalkyle(C₁-C₂) comportant de 1 à 5 atomes de fluor, de chlore et/ou de brome,
R³⁰ représente un atome d'hydrogène, de fluor, de chlore, de brome, d'iode, un groupe méthyle, éthyle ou halogénoalkyle(C₁-C₂) comportant de 1 à 5 atomes de fluor, de chlore et/ou de brome,
ou
A représente le radical de formule (A10) 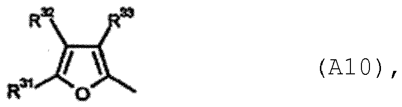 dans laquelle
R³¹ et R³² représentent, indépendamment l'un de l'autre, un atome d'hydrogène, de fluor, de chlore, de brome, un groupe amino, nitro, méthyle, éthyle ou halogénoalkyle(C₁-C₂) comportant de 1 à 5 atomes de fluor, de chlore et/ou de brome,
R³³ représente un atome d'hydrogène, de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A11) 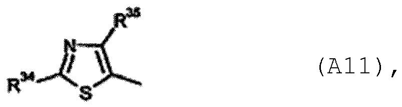 dans laquelle
R³⁴ représente un atome d'hydrogène, de fluor, de chlore, de brome, un groupe amino, alkyl(C₁-C₄)amino, di[alkyl(C₁-C₄)]amino, cyano, méthyle, éthyle ou halogénoalkyle(C₁-C₂) comportant de 1 à 5 atomes de fluor, de chlore et/ou de brome,
R³⁵ représente un atome de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A12) 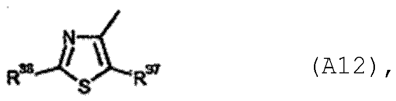 dans laquelle
R³⁶ représente un atome d'hydrogène, de fluor, de chlore, de brome, un groupe amino, alkyl(C₁-C₄)amino, di[alkyl(C₁-C₄)]amino, cyano, méthyle, éthyle ou halogéno-alkyle(C₁-C₂) comportant de 1 à 5 atomes de fluor, de chlore et/ou de brome,
R³⁷ représente un atome de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A13) 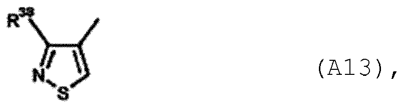 dans laquelle
R³⁸ représente un atome de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A14) 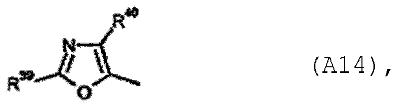 dans laquelle
R³⁹ représente un atome d'hydrogène, un groupe méthyle ou éthyle,
R⁴⁰ représente un atome de fluor, de chlore, de brome, un groupe méthyle ou éthyle,
ou
A représente le radical de formule (A15) 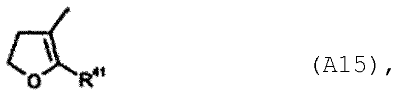 dans laquelle
R⁴¹ représente un groupe méthyle, éthyle ou halogénoalkyle(C₁-C₂) comportant de 1 à 5 atomes de fluor, de chlore et/ou de brome,
ou
A représente le radical de formule (A16) 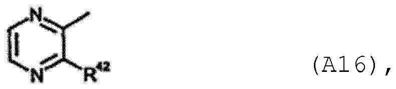 dans laquelle
R⁴² représente un atome d'hydrogène, de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A17) 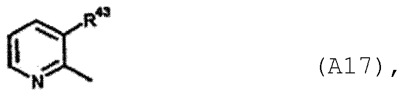 dans laquelle
R⁴³ représente un atome de fluor, de chlore, de brome, d'iode, un groupe hydroxy, alkyle en C₁-C₄, méthoxy, éthoxy, méthylthio, éthylthio, difluorométhylthio, trifluorométhylthio, halogénoalkyle(C₁-C₂) ou halogénoalcoxy(C₁-C₂) comportant chacun de 1 à 5 atomes de fluor, de chlore et/ou de brome,
ou
A représente le radical de formule (A18) 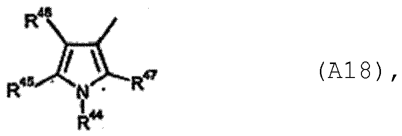 dans laquelle
R⁴⁴ représente un atome d'hydrogène, un groupe méthyle, éthyle, halogénoalkyle(C₁-C₂) comportant de 1 à 5 atomes de fluor, de chlore et/ou de brome, alcoxy(C₁-C₂)-alkyle(C₁-C₂), hydroxyméthyle, hydroxyéthyle, méthylsulfonyle ou diméthylaminosulfonyle,
R⁴⁵ représente un atome d'hydrogène, de fluor, de chlore, de brome, un groupe méthyle, éthyle ou halogénoalkyle(C₁-C₂) comportant de 1 à 5 atomes de fluor, de chlore et/ou de brome,
R⁴⁶ représente un atome d'hydrogène, de fluor, de chlore, de brome, d'iode, un groupe cyano, méthyle, éthyle, isopropyle ou halogénoalkyle(C₁-C₂) comportant de 1 à 5 atomes de fluor, de chlore et/ou de brome,
R⁴⁷ représente un atome d'hydrogène, de fluor, de chlore, de brome, un groupe méthyle, éthyle ou halogénoalkyle(C₁-C₂) comportant de 1 à 5 atomes de fluor, de chlore et/ou de brome,
ou
A représente le radical de formule (A19) 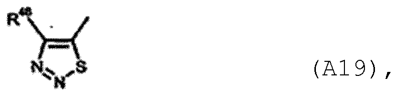 dans laquelle
R⁴⁸ représente un groupe méthyle, éthyle, n-propyle ou isopropyle.

4. Procédé pour la préparation des carboxamides de formule (I) selon la revendication 1, **caractérisé en ce que**
(a) on fait réagir des dérivés d'acides carboxyliques de formule (II) 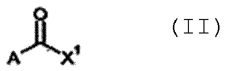 dans laquelle
A a les significations indiquées plus haut et
X¹ représente un atome d'halogène ou un groupe hydroxy,
avec des dérivés d'aniline de formule (III) 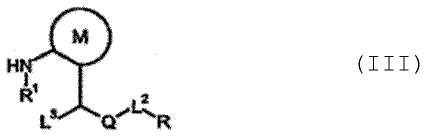 dans laquelle
R¹, M, Q, L² et R ont les significations indiquées plus haut,
L³ représente un atome d'hydrogène ou un groupe alkyle en C₁-C₉,
éventuellement en présence d'un catalyseur, éventuellement en présence d'un agent de condensation, éventuellement en présence d'un capteur d'acide et éventuellement en présence d'un diluant,
ou
(b) on fait réagir des carboxamides de formule (IV) 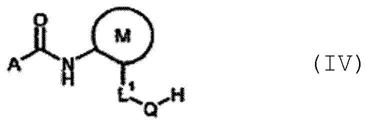 dans laquelle M, L¹, Q et A ont les significations indiquées plus haut,
avec un composé de formule (V) 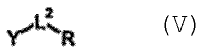 dans laquelle
L² et R ont les significations indiquées plus haut,
Y représente un atome d'halogène, un groupe triflate (trifluorométhylsulfonyle), mésylate (méthylsulfonyle) ou tosylate (4-méthyl-phénylsulfonyle),
en présence d'une base et en présence d'un diluant,
ou
(c) on fait réagir des carboxamides de formule (I-a) 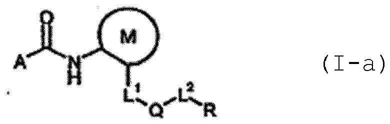 dans laquelle M, L¹, Q, L², R et A ont les significations indiquées plus haut,
avec des halogénures de formule (VI)
R^{1-A}-X² (VI)
dans laquelle
X² représente un atome de chlore, de brome ou d'iode,
R^{1-A} représente un groupe alkyle en C₁-C₈, alkyl (C₁-C₆) sulfinyle, alkyl(C₁-C₆)sulfonyle, alcoxy(C₁-C₄)-alkyle(C₁-C₄), cycloalkyle en C₃-C₈ ; halogénoalkyle(C₁-C₆), halogéno-alkyl(C₁-C₄)thio, halogénoalkyl(C₁-C₄)-sulfinyle, halogénoalkyl(C₁-C₄)sulfonyle, halogéno-alcoxy(C₁-C₄)-alkyle(C₁-C₄), halogénocycloalkyle(C₃-C₈₎ comportant chacun de 1 à 9 atomes de fluor, de chlore et/ou de brome ; formyle, formyl-alkyle(C₁-C₃), [alkyl(C₁-C₃)]carbonyl-alkyle(C₁-C₃), [alcoxy(C₁-C₃)]carbonyl-alkyle(C₁-C₃) ; halogéno-[alkyl(C₁-C₃)]carbonyl-alkyle(C₁-C₃), halogéno-[alcoxy(C₁-C₃)]carbonyl-alkyle(C₁-C₃) comportant chacun de 1 à 13 atomes de fluor, de chlore et/ou de brome ;
[alkyl(C₁-C₈)]carbonyle, [alcoxy(C₁-C₈)]-carbonyle, [alcoxy(C₁-C₄)-alkyl(C₁-C₄)]-carbonyle, [cycloalkyl(C₃-C₈)]carbonyle ; [halogénoalkyl(C₁-C₆)]carbonyle, [halogénoalcoxy(C₁-C₆)]carbonyle, [halogénoalcoxy(C₁-C₄)-alkyl(C₁-C₄)]carbonyle, [halogénocycloalkyl(C₃-C₈)]carbonyle comportant chacun de 1 à 9 atomes de fluor, de chlore et/ou de brome ; ou - C (=O) C (=O) R², -CONR³R⁴ ou -CH₂NR⁵_{R}⁶, R², R³, R⁴, R⁵ et R⁶ ayant les significations indiquées plus haut,
en présence d'une base et en présence d'un diluant.

5. Composition destinée à la lutte contre des micro-organismes indésirables, **caractérisée par** une teneur en au moins un carboxamide de formule (I) selon la revendication 1, en plus d'excipients et/ou de substances tensioactives.

6. Utilisation de carboxamides de formule (I) selon la revendication 1, pour la lutte contre des micro-organismes indésirables.

7. Procédé pour la lutte contre des micro-organismes indésirables, **caractérisé en ce qu'**on applique sur les micro-organismes et/ou leur habitat des carboxamides de formule (I) selon la revendication 1.

8. Procédé pour la préparation de compositions destinées à la lutte contre des micro-organismes indésirables, **caractérisé en ce qu'**on mélange des carboxamides de formule (I) selon la revendication 1 avec des excipients et/ou des substances tensioactives.
